# EUROPEAN PATENT SPECIFICATION

(11) **EP 1 962 890 B1**
(45) Date of publication and mention of the grant of the patent: **11.11.2015**
(21) Application number: 06830724.8
(22) Date of filing: 19.12.2006
(51) Int. Cl.: A61K 39/00, A61K 39/085, C07K 14/31

(54) **IMMUNOGENIC COMPOSITION**
IMMUNOGENE ZUSAMMENSETZUNG
COMPOSITION IMMUNOGENE

(30) Priority: 21.12.2005 GB 0526038
(43) Date of publication of application: 03.09.2008
(73) Proprietor: GlaxoSmithKline Biologicals S.A., 1330 Rixensart (BE)
(72) Inventor: CASTADO, Cindy, 1330 Rixensart (BE); NEYT, Cecile, Anne, 1330 Rixensart (BE); POOLMAN, Jan, 1330 Rixensart (BE)
(74) Representative: Johnston, Caroline Louise
(86) International application number: PCT/EP2006/069944
(87) International publication number: WO 2007/071692

(56) References cited:
- WO-A-99/24467
- WO-A-2006/032472
- WO-A1-99/24467
- WO-A2-2006/032472
- US-A- 5 571 514
- US-A- 5 571 514
- EKSTEDT R D ET AL: "Immunity to staphylococcal infection in mice: effect of living versus killed vaccine, role of circulating antibody, and induction of protection-inducing antigen(s) in vitro." JOURNAL OF BACTERIOLOGY NOV 1969, vol. 100, no. 2, November 1969 (1969-11), pages 745-750, XP002442499 ISSN: 0021-9193
- LÉONETTI M ET AL: "Presentation of antigen in immune complexes is boosted by soluble bacterial immunoglobulin binding proteins." THE JOURNAL OF EXPERIMENTAL MEDICINE 19 APR 1999, vol. 189, no. 8, 19 April 1999 (1999-04-19), pages 1217-1228, XP002442500 ISSN: 0022-1007
- ZHANG L ET AL: "Staphylococcus aureus expresses a cell surface protein that binds both IgG and beta2-glycoprotein I." MICROBIOLOGY (READING, ENGLAND) JAN 1999, vol. 145 ( Pt 1), January 1999 (1999-01), pages 177-183, XP002442501 ISSN: 1350-0872
- EKSTEDT R D ET AL: "Immunity to staphylococcal infection in mice: effect of living versus killed vaccine, role of circulating antibody, and induction of protection-inducing antigen(s) in vitro" JOURNAL OF BACTERIOLOGY, AMERICAN SOCIETY FOR MICROBIOLOGY, WASHINGTON, DC; US, vol. 100, no. 2, 1 November 1969 (1969-11-01), pages 745-750, XP002442499 ISSN: 0021-9193
- ZHANG L ET AL: "Staphylococcus aureus expresses a cell surface protein that binds both IgG and beta2-glycoprotein I" MICROBIOLOGY, SOCIETY FOR GENERAL MICROBIOLOGY, READING, GB, vol. 145, no. PART 1, 1 January 1999 (1999-01-01), pages 177-183, XP002442501 ISSN: 1350-0872
- LEONETTI M ET AL: "Presentation of antigen in immune complexes is boosted by soluble bacterial immunoglobulin binding proteins" THE JOURNAL OF EXPERIMENTAL MEDICINE, ROCKEFELLER UNIVERSITY PRESS, UNITED STATES, vol. 189, no. 8, 19 April 1999 (1999-04-19), pages 1217-1228, XP002442500 ISSN: 0022-1007

## Description

The present invention relates to the field of Staphylococcal immunogenic compositions and vaccines, their manufacture and the use of such compositions in medicine. More particularly, it relates to compositions comprising an immunoglobulin binding domain derived from *S*. *aureus* protein A and immunoglobulin domain derived from the Sbi protein. Fusion proteins comprising an IgG binding domain from protein A and an IgG binding domain from Sbi are described as well as a polynucleotide encoding such a protein.

Protein A is a well studied cell wall associated protein in *S*. *aureus* which binds to the Fc and Fab regions of IgG from several species. Protein A consists of five consecutive domains, all with IgG binding activity, followed by a region anchoring the protein in the cell wall (Lofdahl et al 1983 Eur J Biochem 156; 637-643). Protein A has been investigated as a vaccine component for use in mastitis a vaccine (Carter and Kerr J. Diary Sci 2003 86; 1177-1186).

More recently Sbi was identified as a second IgG binding protein expressed by *S*. *aureus* (Zhang et al 1998, Microbiology 144; 985-991 and Zhang et al 1999, 145; 177-183). Sbi protein consists of about 436 amino acids and has an immunoglobulin binding specificity similar to protein A. Sbi contains two IgG binding domains towards the N-terminus of the protein and a further apolipoprotein H binding domain towards the C-terminus of Sbi (WO 99/24467).

*S*. *aureus* infections are treated with antibiotics, with penicillin being the drug of choice whereas vancomycin is used for methicillin resistant isolates. The percentage of staphylococcal strains exhibiting wide-spectrum resistance to antibiotics has become increasingly prevalent since the 1980's (Panlilo et al 1992, Infect.Control. Hosp. Epidemiol. 13; 582), posing a threat for effective antimicrobial therapy. In addition, the recent emergence of vancomycin resistant *S. aureus* strain has aroused fear that methicillin resistant *S.aureus* strains will emerge and spread for which no effective therapy is available.

An alternative approach of using antibodies against staphylococcal antigens in passive immunotherapy has been investigated. Therapy involving administration of polyclonal antisera are under development (WO 00/15238, WO 00/12132).

Attempts to protect against *S. aureus* bacteremia in an infant rat model using various doses of staphylococcal protein A antiserum were unsuccessful. Even the highest levels of antibody did not significantly reduce mortality, bacteremia or metastatic infection to lungs or liver (Greenberg et al Infection and Immunity 57; 1113-1118 1989).

There remains a need to develop a vaccine which protects against staphylococcal disease. The provision of a method of blocking one of the staphylococcal defence mechanisms would improve the likelihood of producing an effective active or passive immunogenic composition.

Accordingly, there is provided an immunogenic composition comprising two different isolated staphylococcal polypeptides, each comprising an IgG binding domain, wherein the isolated staphylococcal polypeptides comprise an isolated protein A polypeptide from *S. aureus* having a sequence which is at least 90% idenitical to SEQ ID NO:12-40, and an isolated Sbi polypeptide from *S. aureus* having a sequence which is at least 90% identical to SEQ ID N0:1-11.

The disclosure also provides a polypeptide comprising: a protein A part having an amino acid sequence having at least 90% identity to an amino acid sequence selected from the group consisting of SEQ ID NOs 12-40 and an Sbi part which has an amino acid sequence having at least 90% identity to an amino acid sequence selected from the group consisting of SEQ ID NOs 1-11.

Such a polypeptide comprises IgG binding domains from each of the known S. *aureus* IgG binding proteins and is a convenient way of introducing the polypeptide sequences allowing an immune response to be raised against both of the IgG binding proteins.

In a further aspect of the invention there is provided, a polynucleotide comprising a Protein A encoding region having at least 90% identity to a polynucleotide sequence selected from the group consisting of SEQ ID NOs 54-72 and an Sbi-encoding region which has at least 90% identity to a polynucleotide sequence selelcted from the group consisting of SEQ ID NOs 43-53.

In a further aspect of the invention, there is provided a process for making the vaccine of the invention comprising the step of adding a pharmaceutically acceptable excipient to a composition comprising an IgG domain from protein A and an IgG domain from Sbi.

In a further aspect of the invention, there is provided an immunogenic composition of the invention for use in the treatment of prevention of staphylococcal disease.

In a further aspect of the invention, there is provided a use of the immunogenic composition of the invention in the preparation of a medicament for the treatment or prevention of staphylococcal disease.

In a further aspect of the disclosure, there is provided a method of treating or preventing staphylococcal disease comprising administering the immunogeninc composition of the invention to a patient in need thereof.

### Description of Figures

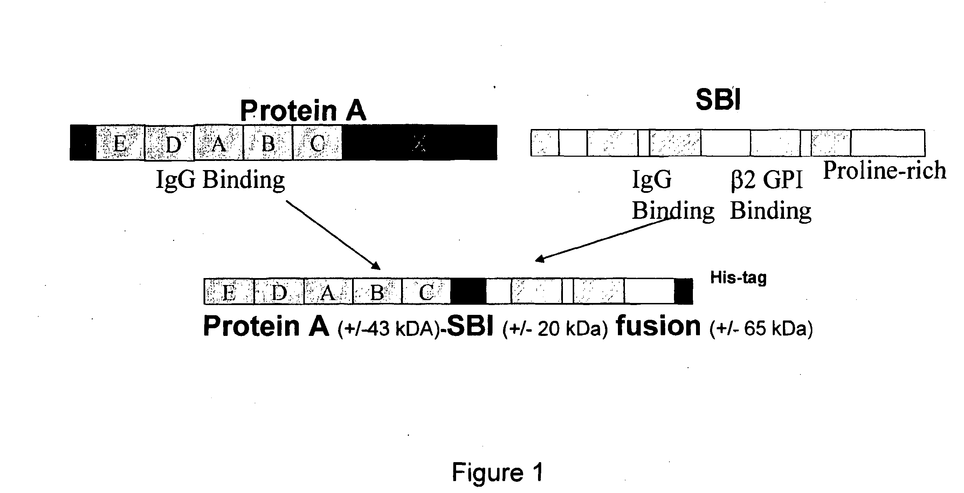

Figure 1 - Schematic drawing of the ProteinA-Sbi fusion protein

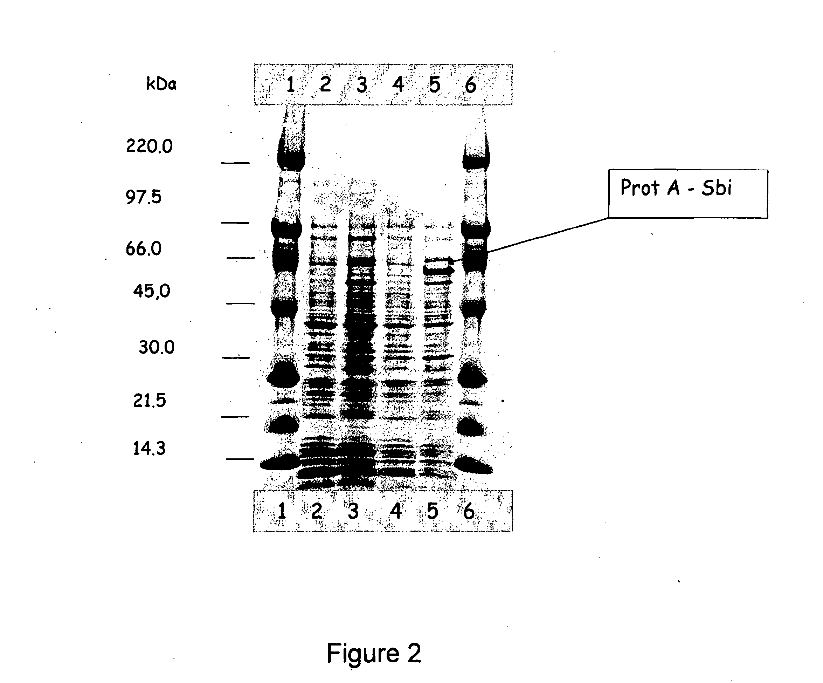

Figure 2 - Coomassie blue stained 4-20% PAGE showing expressin of the ProteinA-Sbi fusion protein. Lanes 1 and 6 contain molecular weight markers, lane 2 contains the AR58 *E. coli* prior to induction, lane 3 contains AR58 *E.coli* after 4 hours induction, lane 4 contains AR58 *E. coli* transformed with ProteinA-Sbi plasmid prior to induction, lane 5 contains AR58 *E. coli* transformed with ProteinA-Sbi after 4 hours induction.

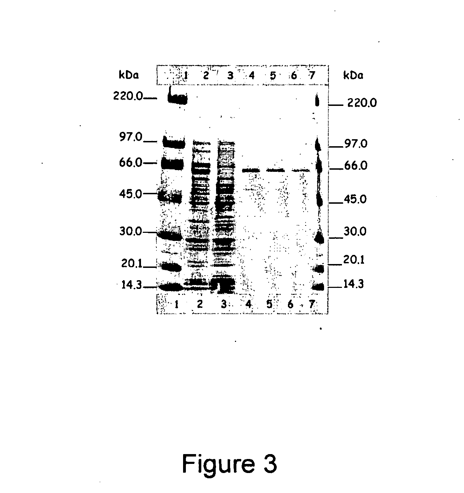

Figure 3 - Coomassie blue stained 4-20% PAGE showing purification of the Protein A-Sbi fusion protein. Lanes 1 and 7 contain molecular weight markers, lane 2 contains the soluble fraction prior to loading onto the Hi-Trap column, lane 3 contains the soluble fraction in an uninduced culture, lane 4 contains 1µg of the column purified protein, lane 5 contains 0.5µg of the column purified protein, lane 6 contains 0.25µg of the column purified protein.

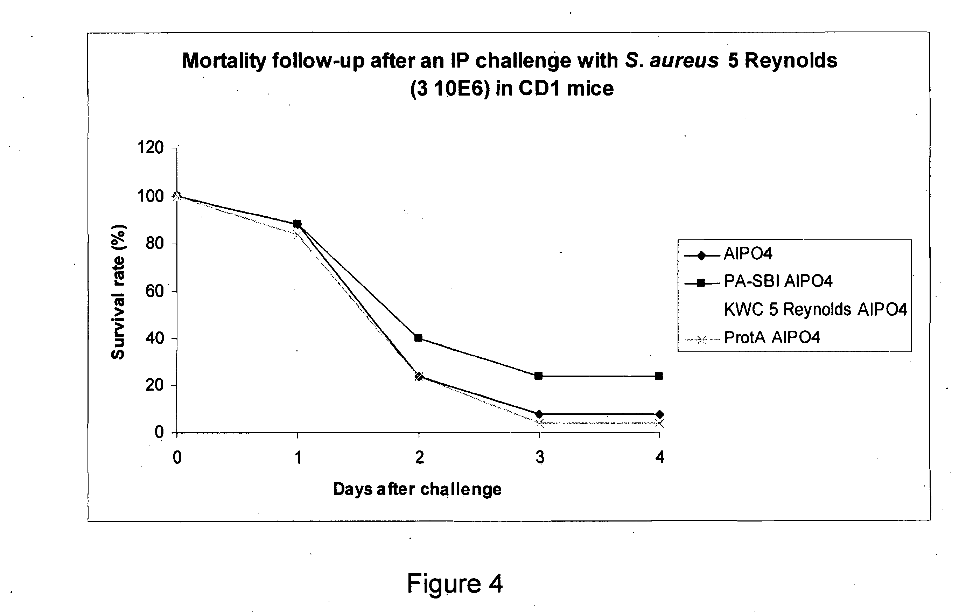

Figure 4 - Graph showing the mortality follow-up after a challenge with *S*. *aureus* 5 Reynolds (3 10E6) in CD1 mice. The line marked with triangle shows survival after immunisation with killed whole cells of *S. aureus* 5 Reynolds adjuvanted with AlPO4. The line marked with diamonds shows survival after immunisation with adjuvant alone. The line marked with crosses shows survival after inoculation with protein A adjuvanted with AlPO4. The line marked with squares shows survival after inoculation with ProteinA-Sbi fusion protein adjuvanted with AlPO4.

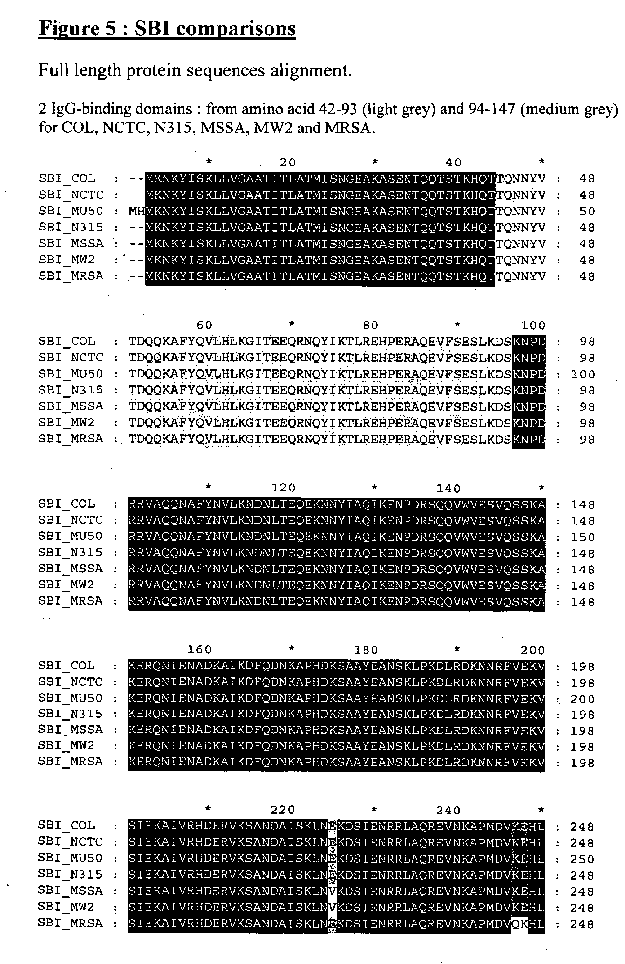

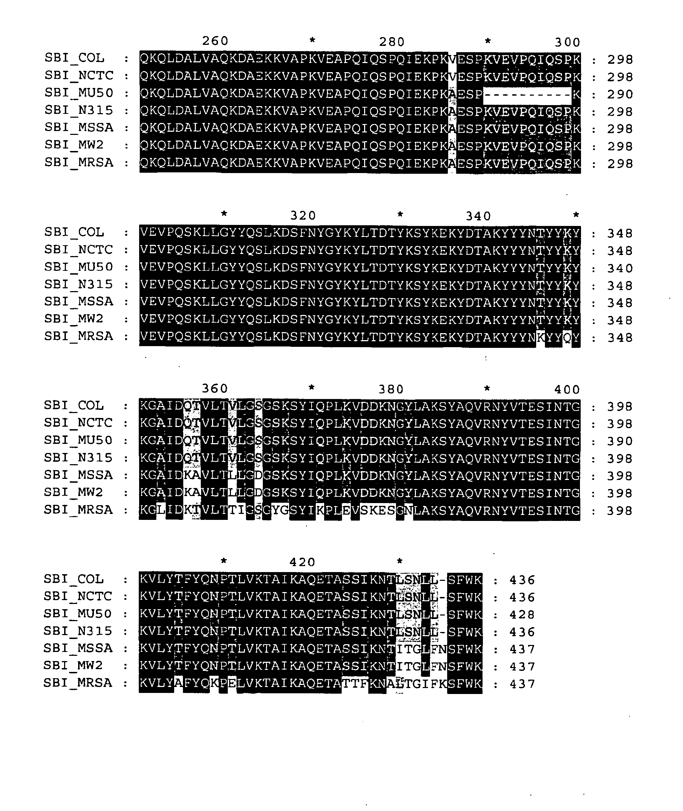

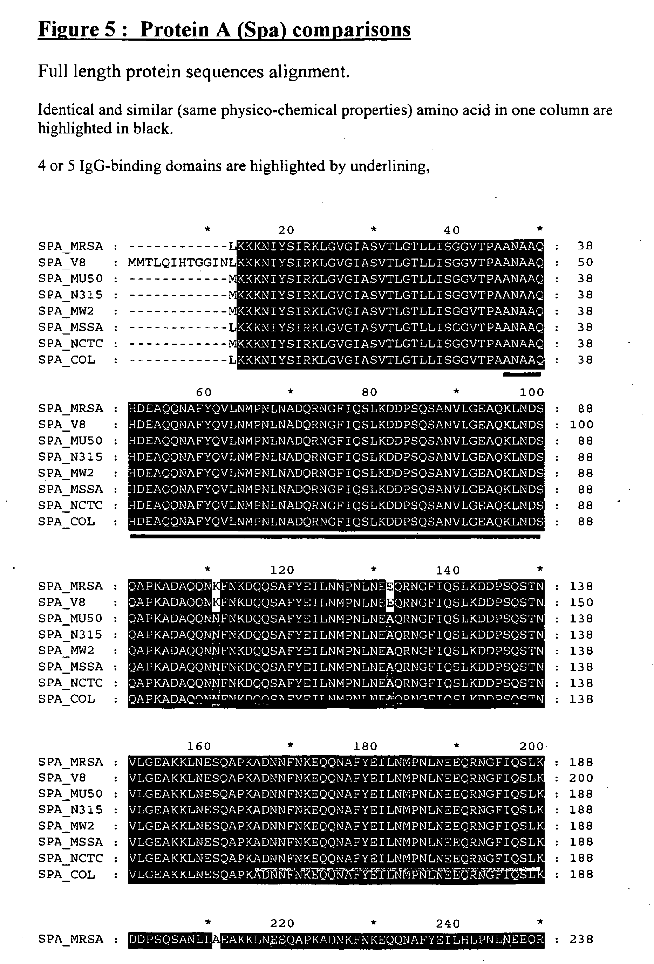

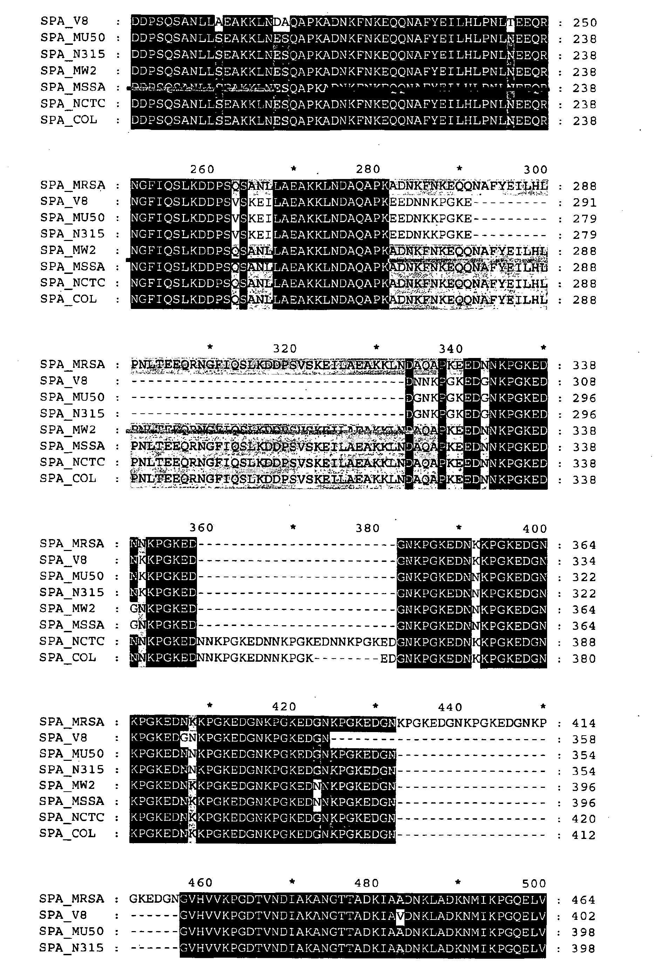

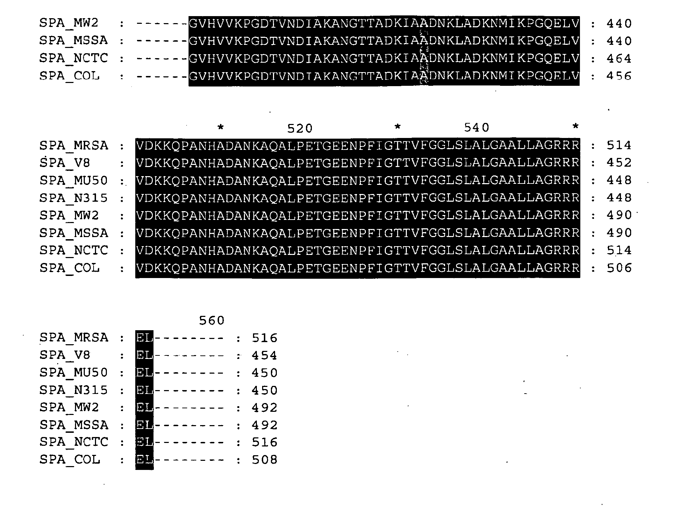

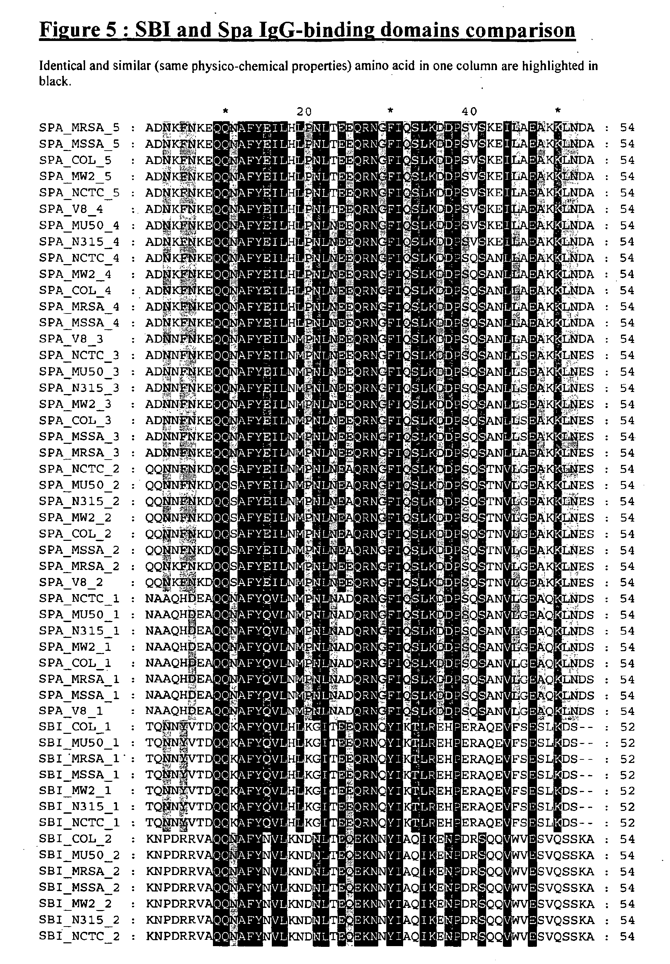

Figure 5 - Amino acid alignments of proteinA and Sbi proteins from different strains of *S. aureus.*

### Detailed description

The present application discloses an immunogenic composition comprising at least or exactly two, three, four or five different staphylococcal polypeptides, each comprising an IgG binding domain. Such an immunogenic composition may further comprise further antigens which do not comprise an IgG binding domain.

A staphylococcal polypeptide is defined as a polypeptide which is expressed by a staphylococcal bacterium for instance *S. aureus* or *S. epidermidis.* The staphylococcal polypeptide is either derived from a staphylococcal strain or is expressed recombinantly in a different expression system, for example in *E. coli.* The staphylococcal polypeptide may be a full length protein or a fragment of a full length protein that contains an IgG binding domain.

By different, it is meant that the polypeptides are encoded by separate genes. In an embodiment, the different polypeptides are present as noncovalently linked polypeptides (i.e. separate or free proteins). In an embodiment, the different polypeptides are present as a covalently cross-linked conjugate. In an embodiment, the different polypeptides are present as at least one fusion protein.

An IgG binding domain is a domain that is capable of binding to IgG. The interaction between an antigen and the CDR regions of the Fv domains which typically interact with the antigen to which the antibody is raised, is excluded from this definition. An IgG binding domain typically interacts with the Fc domain or with conserved regions of the Fab domains. The binding of individual IgG binding domains of protein A to Fc and F(ab')₂ doamins has been analysed (Jansson et al FEMS Immunology and Medical Microbiology 20; 69-78 1998). Optionally, an IgG binding domain is capable of binding to an antibody or fragment thereof (such as Fc or Fab) with an affinity of 0.1-1000, 1-500, 1-100 or 5-50 x 10-6 M-1 or approximately 10 x 10-6 M-1.

An IgG binding domain typically has an amino acid sequence which is at least 90%, 95% or 98% identical to that of SEQ ID NOs: 1, 2, or 12-21. Fragments of SEQ ID NOs 1, 2, or 12-21 which bind to IgG and/or are capable of generating an immune response against Protein A or Sbi, are also considered to be IgG binding proteins of the disclosure.

The immunogenic composition of the invention comprises a protein A polypeptide from *S*. *aureus*.

The protein A polypeptide has a sequence which is at least 90%, 95%, 98% or 100% identical to that of SEQ ID NO 12-40.

In an embodiment, the protein A polypeptide is encoded by a polynucleotide having a sequence which is at least 90%, 95%, 98% or 100% identical to SEQ ID NO: 54-72.

Protein A is a 58kDa protein containing about or exactly 524 amino acid (or about or exactly 509 amino acids in the mature protein). It comprises 5 IgG binding domains (E, D, A, B and C) which are located towards the N-terminus of the protein as shown in figure 1. All IgG domains of Protein A share 91-100% identity (computed with the ClustalW program).

In an embodiment, the protein A polypeptide or fragment thereof comprises 1, 2, 3, 4 or 5 IgG binding domains. For example, the immunogenic composition comprises IgG binding domain(s), E; D; A; B; C; E and D; D and A; A and B; B and C; E and A; E and B; E and C; D and B; D and C; A and C; E, D and A; D, A and B; A, B and C; E, D, A and B; D, A, B and C; or E, D, A, B and C. Combinations comprising E and 1, 2, 3 or 4 further IgG binding domains selected from A, B, C and D are present in an embodiment. These combinations of IgG binding domains are present in a single polypeptide or may be present in 2, 3, 4 or 5 separate polypeptides. In an embodiment, the IgG domains have an amino acid sequence comprising the sequence of SEQ ID NO: 12-21. Where multiple protein A IgG binding domains are present, the sequence may be that of SEQ ID NO: 22-32.

Alternatively, the sequence of the protein A polypeptide is the whole or a fragment of SEQ ID NO 33-40, or variants sharing at least 90%, 95%, 98% or 99% identity to the sequence of SEQ ID NO: 33-40 or the polypeptide encoded by SEQ ID NO 65-72 or variants sharing at least 90%, 95%, 98% or 99% identity to SEQ ID NO: 65-72. Such a fragment comprises at least 1, 2, 3, 4 or 5 IgG binding domains. Such a fragment optionally contains at least 50, 60, 70, 80, 90, 100, 150, 200, 250 or 300 amino acids.

The sequence of the Protein A IgG binding domains are represented by SEQ ID NOs 12-21 and the protein A IgG binding domains have sequences which are at least 90%, 95%, 98% or 100% identical to SEQ ID NOs 12-21.

In an embodiment, the fragments of Protein A consist essentially of the amino acid sequence of SEQ ID NOs 12-32 but additionally comprise a further 1, 2, 3, 4, 5, 10, 20, 30, 40, 50 or 100 amino acids on either or both the N and C terminal side of the SEQ ID 12-32. In an embodiment, the additional sequence is that found in Protein A as set out in SEQ ID NOs 33-40 or Figure 4.

Specific variants of the polypeptide of the invention are envisaged. In particular, the third amino acid of protein A IgG binding domain D may be K or N, the 24^{th} amino acid of protein A IgG binding domain D may be E or A, the 46^{th} amino acid of protein A IgG binding domain A may be A or S, the 53^{rd} amino acid of protein A IgG binding domain A may be D or E, the 23^{rd} amino acid of protein A IgG binding domain B may be N or T, the 40^{th} amino acid of protein A IgG binding domain B may be Q or V , the 42^{nd} amino acid of protein A IgG binding domain B may be A or K, the 43^{rd} amino acid of protein A IgG binding domain B may be N or E and/or the 44^{th} amino acid of protein A IgG binding domain B may be I or L.

In an embodiment, an N-terminal M residue is added to the sequence of SEQ ID NO 1-32.

The immunogenic composition of the invention comprises an Sbi polypeptide from S. *aureus* or a fragment thereof containing an IgG binding domain.

Sbi is a protein of about 48kDa, containing about 436 amino acids. Two IgG binding domains are present towards the N-terminus of the protein and Sbi further comprises a apolipoprotein H (β2-GPI) binding domain followed by a proline rich domain (see Figure C).

In an embodiment, the Sbi polypeptide has a sequence which is at least 90%, 95%, 98% or 100% identical to SEQ ID NO: 1-11 or fragment thereof containing an IgG binding domain (optionally containing 2 IgG binding domains). Such a fragment optionally contains at least 50, 60, 70, 80, 90, 100, 150, 200, 250 or 300 amino acids.

In an embodiment, the Sbi polypeptide is encoded by a polynucleotide having a sequence which is at least 90%, 95%, 98% or 100% identical to SEQ ID NO: 43-53 or fragment thereof encoding an IgG binding domain (optionally encoding 2 IgG binding domains).

For example, fragments of Sbi consist of or comprise the N-terminal IgG binding domain, the C-terminal IgG binding domain or both IgG binding domains. In an embodiment, the fragments have amino acid sequences which are at least 90%, 95%, 98% or 100% identical to SEQ ID NOs 1-4.

In an embodiment, the fragments of Sbi consist essentially of the amino acid sequence of SEQ ID NOs 1-4 but additionally comprise a further 1, 2, 3, 4, 5, 10, 20, 30, 40, 50 or 100 amino acids on either or both the N and C terminal side of the SEQ ID 1-4. In an embodiment, the sequence is that of the Sbi protein as set out in SEQ ID NOs 5-11 and Figure 4.

Where both IgG binding domains of Sbi are present in an immunogenic composition, they may be present on separate polypeptide chains or in the same polypeptide chain.

In an embodiment, both protein A (or a fragment thereof comprising an IgG binding domain) and Sbi (or a fragment thereof comprising an IgG binding domain) are present in the immunogenic composition. These antigens may be present as separate proteins within the immunogenic composition of they may be covalently linked together , for example as a fusion protein or using a crosslinking reagent to link the two polypeptides.

In an embodiment, the polypeptide comprises a protein A part and an Sbi part. For example, the Protein A part has an amino acid sequence having at least 90%, 95%, 98% or 100% identity to an amino acid sequence from the group consisting of SEQ ID NOs 12-40 or an immunogenic fragment thereof comprising at least one IgG binding domain and an Sbi part which has an amino acid sequence having at least 90%, 95%, 98% or 100% identity to an amino acid sequence selected from the group consisting of SEQ ID NOs 1-11 or an immunogenic fragment thereof comprising at least one IgG binding domain.

In the context of a fusion protein or a crosslinked conjugate, any of the fragments or variants of protein A or Sbi as described above may be incorporated.

In an embodiment, the polypeptide is a fusion protein containing the protein A part N-terminal to the Sbi part. Alternatively the fusion protein may contain the Sbi part N-terminal to the protein A part.

The polypeptide of the invention optionally further comprises sequence from further staphylococcal protein(s) optionally selected from the group consisting of Ebh (WO 02/59148), Elastin binding protein (EbpS WO 98/38312), EFB (FIB) (WO 94/06830), ClfA (US6008341), ClfB (WO 99/27109), SdrC (WO 99/27109), SdrG (WO 97/48727), FnbA (including variants desribed in WO 05/116064), FbpA, IsaA/PisA (DE 199 17 098), IsdA (WO 02/59148, WO 06/59247), IsdB (WO 02/059148, including variants described in WO 05/09379 and WO 05/09378), IsdC (WO 06/59247), HarA (WO 05/09378), alpha toxin (Hla US4615884), alpha toxin H35R mutant, penicillin binding protein 4 (WO 06/33918), SsaA (WO 05/115113), Aap (WO 05/86663), RAP (WO 99/32133), AhpC and variants (WO 06/78680), SasA (WO 06/121664).

In an embodiment, the polypeptide has a polypeptide sequence which is at least 90%, 95%, 98% or 100% identical to the sequence of SEQ ID NO:41 or 42 or 76 or fragment thereof comprising an IgG binding domain, preferably from both protein A and Sbi.

In an embodiment, the polypeptide is a fusion protein encoded by a polynucleotide having a sequence which is at least 90%, 95%, 98% or 100% identical to SEQ ID NO: 73-75 or fragment thereof comprising an IgG binding domain , preferably from both protein A and Sbi.

SEQ group 1 contains SEQ ID NOs 43-75.

SEQ group 2 contains SEQ ID NOs 1-42 and 76.

The present invention further provides for:
(a) an isolated polypeptide which comprises an amino acid sequence which has at least 90% identity, more preferably at least 95% identity, most preferably at least 97, 98 or 99% or exact identity, to that of any sequence of SEQ Group 2;
(b) a polypeptide encoded by an isolated polynucleotide comprising a polynucleotide sequence which has at least 90% identity, more preferably at least 95% identity, even more preferably at least 97, 98 or 99% or exact identity to any sequence of SEQ Group 1 over the entire length of the selected sequence of SEQ Group 1; or
(c) a polypeptide encoded by an isolated polynucleotide comprising a polynucleotide sequence encoding a polypeptide which has preferably at least 90% identity, more preferably at least 95% identity, even more preferably at least 97-99% or exact identity, to the amino acid sequence of any sequence of SEQ Group 2.

The invention also provides an immunogenic fragment of a polypeptide of the invention, that is, a contiguous portion of the Protein A-Sbi polypeptide which has the same or substantially the same immunogenic activity as the polypeptide comprising the corresponding amino acid sequence selected from SEQ Group 2 ; That is to say, the fragment (if necessary when coupled to a carrier) is capable of raising an immune response which recognises the Protein A-Sbi polypeptide. Such an immunogenic fragment may include, for example, the Protein A-Sbi polypeptide lacking an N-terminal leader sequence, and/or a transmembrane domain and/or a C-terminal anchor domain. In an embodiment, the immunogenic fragment of Protein A-Sbi according to the invention comprises substantially all of the extracellular domain of a polypeptide which has at least 85% identity, preferably at least 90% identity, more preferably at least 95% identity, most preferably at least 97, 98 or 99% identity, to that a sequence selected from SEQ Group 2 over the entire length of said sequence.

A fragment is a polypeptide having an amino acid sequence that is entirely the same as part but not all of any amino acid sequence of any polypeptide of the invention. As with Protein A-Sbi polypeptides, fragments may be "free-standing," or comprised within a larger polypeptide of which they form a part or region, most preferably as a single continuous region in a single larger polypeptide.

In an embodiment, fragments include, for example, truncation polypeptides having a portion of an amino acid sequence selected from SEQ Group 2 or of variants thereof, such as a continuous series of residues that includes an amino- and/or carboxyl-terminal amino acid sequence. Degradation forms of the polypeptides of the invention produced by or in a host cell, are also preferred. Further preferred are fragments characterized by structural or functional attributes such as fragments that comprise alpha-helix and alpha-helix forming regions, beta-sheet and beta-sheet-forming regions, turn and turn-forming regions, coil and coil-forming regions, hydrophilic regions, hydrophobic regions, alpha amphipathic regions, beta amphipathic regions, flexible regions, surface-forming regions, substrate binding region, and high antigenic index regions.

In an embodiment, fragments include an isolated polypeptide comprising an amino acid sequence having at least 15, 20, 30, 40, 50 or 100 contiguous amino acids from the amino acid sequence selected from SEQ Group 2 or an isolated polypeptide comprising an amino acid sequence having at least 15, 20, 30, 40, 50 or 100 contiguous amino acids truncated or deleted from the amino acid sequence selected from SEQ Group 2 .

The present invention also includes variants of the aforementioned polypeptides, that is polypeptides that vary from the referents by conservative amino acid substitutions, whereby a residue is substituted by another with like characteristics. Typical such substitutions are among Ala, Val, Leu and Ile; among Ser and Thr; among the acidic residues Asp and Glu; among Asn and Gln; and among the basic residues Lys and Arg; or aromatic residues Phe and Tyr.

Polypeptides of the present invention can be prepared in any suitable manner. Such polypeptides include isolated naturally occurring polypeptides, recombinantly produced polypeptides, synthetically produced polypeptides, or polypeptides produced by a combination of these methods. Means for preparing such polypeptides are well understood in the art.

### Polynucleotides

The invention discloses any polynucleotide encoding any one of the polypeptides of the invention as described above.

A further embodiment of the invention is a polynucleotide comprising a Protein A encoding region having at least 90%, 95%, 98%, 99% or 100% identity to a polynucleotide sequence selected from the group consisting of SEQ ID NOs 54-73 and an Sbi-encoding region which has at least 90%, 95%, 98%, 99% or 100% identity to a polynucleotide sequence selected from the group consisting of SEQ ID NOs 43-53.

In the case of SEQ ID NO: 47-53 and 65-72, the sequences are of the complete Sbi or protein A, respectively. Fragments of these sequences encoding at least 1, 2, 3, 4 or 5 IgG domains may be counted as the protein A or Sbi encoding part of the polynicelotides of the invention.

Specific variants of the polynucleotide of the invention are envisaged. For example, the fragment contains 1, 2, 3, 4 or 5 IgG binding domains as depicted in Figure X. In particular, the third codon of protein A IgG binding domain D may encode K or N, the 24^{th} codon of protein A IgG binding domain D may encode E or A, the 46^{th} codon of protein A IgG binding domain A may encode A or S, the 53^{rd} codon of protein A IgG binding domain A may encode D or E, the 23^{rd} codon of protein A IgG binding domain B may encode N or T, the 40^{th} codon of protein A IgG binding domain B may encode Q or V , the 42^{nd} codon of protein A IgG binding domain B may encode A or K, the 43^{rd} codon of protein A IgG binding domain B may encode N or E and/or the 44^{th} codon of protein A IgG binding domain B may encode I or L.

In an embodiment of the disclosure the polynucleotide encodes a fusion protein comprising a protein A like part having at least 90%, 95%, 98% or 100% identity to an amino acid sequence selected from the group consisting of SEQ ID NOs 12-21 and 32-40 or an immunogenic fragment thereof comprising at least 1, 2, 3, 4 or 5 IgG binding domains and an Sbi-like part which has at least 90%, 95%, 98% or 100% identity to an amino acid sequence selected from the group consisting of SEQ ID NOs 1-11, or an immunogenic fragment thereof comprising at least one IgG binding domain.

In an embodiment the polynuclotide of the invention has a polynucleotide sequence having at least 90%, 95%, 98% or 100% identity to SEQ ID NO 73 or 74.

In a preferred embodiment of the invention the polynucleotide comprises a region encoding Protein A-Sbi polypeptides comprising sequences set out in SEQ Group1 which include full length gene, or a variant or fragment thereof.

Polynucleotides of the invention do not encompass a complete genomic DNA from a staphylococcal species, e.g. *S. aureus* or *S. epidermidis*.

As a further aspect of the disclosure there are provided isolated nucleic acid molecules encoding and/or expressing Protein A-Sbi polypeptides and polynucleotides, particularly *S*. *aureus* or *S*. *epidermidis* Protein A-Sbi polypeptides and polynucleotides, including, for example, unprocessed RNAs, ribozyme RNAs, mRNAs, cDNAs, B- and Z-DNAs. Further embodiments of the invention include biologically, diagnostically, prophylactically, clinically or therapeutically useful polynucleotides and polypeptides, and variants thereof, and compositions, preferably immunogenic compositions, comprising the same.

Another aspect of the disclosure relates to isolated polynucleotides, that encode Protein A-Sbi polypeptides having a deduced amino acid sequence of SEQ Group 2 and polynucleotides closely related thereto and variants thereof.

An embodiment of the disclosure relates to Protein A-Sbi polypeptides from *S. aureus* or *S. epidermidis* comprising or consisting of an amino acid sequence selected from SEQ Group 2 or a variant thereof.

Using the information provided herein, such as a polynucleotide sequence set out in SEQ Group 1, a polynucleotide of the invention encoding Protein A-Sbi polypeptide may be obtained using standard cloning and screening methods, such as those for cloning and sequencing chromosomal DNA fragments from bacteria using *S. aureus* as starting material, followed by obtaining a full length clone. For example, to obtain a polynucleotide sequence of the invention, such as a polynucleotide sequence given in SEQ Group 1, typically a library of clones of chromosomal DNA of a different staphylococcal strain in *E.coli* or some other suitable host is probed with a radiolabeled oligonucleotide, preferably a 17-mer or longer, derived from a partial sequence. Clones carrying DNA identical to that of the probe can then be distinguished using stringent hybridization conditions. By sequencing the individual clones thus identified by hybridization with sequencing primers designed from the original polypeptide or polynucleotide sequence it is then possible to extend the polynucleotide sequence in both directions to determine a full length gene sequence. Conveniently, such sequencing is performed, for example, using denatured double stranded DNA prepared from a plasmid clone. Suitable techniques are described by Maniatis, T., Fritsch, E.F. and Sambrook et al., MOLECULAR CLONING, A LABORATORY MANUAL, 2nd Ed.; Cold Spring Harbor Laboratory Press, Cold Spring Harbor, New York (1989). (see in particular Screening By Hybridization 1.90 and Sequencing Denatured Double-Stranded DNA Templates 13.70). Direct genomic DNA sequencing may also be performed to obtain a full length gene sequence.

Moreover, each DNA sequence set out in SEQ Group 1 contains an open reading frame encoding a protein having about the number of amino acid residues set forth in SEQ Group 2 with a deduced molecular weight that can be calculated using amino acid residue molecular weight values well known to those skilled in the art.

The polynucleotides of SEQ Group 1, between the start codon and the stop codon, encode respectively the polypeptides of SEQ Group 2. The nucleotide number of start codon and first nucleotide of the stop codon are listed in table 3 for each polynucleotide of SEQ Group 1.

In a further aspect, the present invention provides for an isolated polynucleotide comprising or consisting of:
(a) a polynucleotide sequence which has at least 90% identity, more preferably at least 95% identity, even more preferably at least 97, 98 or 99% or exact identity to any sequence from SEQ Group 1 over the entire length of the polunucleotide sequence from SEQ Group 1; or
(b) a polynucleotide sequence encoding a polypeptide which has at least 90% identity, more preferably at least 95% identity, even more preferably at least 97, 98 or 99% or 100% exact, to any amino acid sequence selected from SEQ Group 2 , over the entire length of the amino acid sequence from SEQ Group 2.

A polynucleotide encoding a polypeptide of the present invention, including homologs and orthologs from species other than *S. aureus,* may be obtained by a process which comprises the steps of screening an appropriate library under stringent hybridization conditions (for example, using a temperature in the range of 45 - 65°C and an SDS concentration from 0.1 - 1%) with a labeled or detectable probe consisting of or comprising any sequence selected from SEQ Group 1 or a fragment thereof; and isolating a full-length gene and/or genomic clones containing said polynucleotide sequence.

The disclosure provides a polynucleotide sequence identical over its entire length to a coding sequence (open reading frame) set out in SEQ Group 1. Also provided by the disclosure is a coding sequence for a mature polypeptide or a fragment thereof, by itself as well as a coding sequence for a mature polypeptide or a fragment in reading frame with another coding sequence, such as a sequence encoding a leader or secretory sequence, a pre-, or pro- or prepro-protein sequence. The polynucleotide of the invention may also contain at least one non-coding sequence, including for example, but not limited to at least one non-coding 5' and 3' sequence, such as the transcribed but non-translated sequences, termination signals (such as rho-dependent and rho-independent termination signals), ribosome binding sites, Kozak sequences, sequences that stabilize mRNA, introns, and polyadenylation signals. The polynucleotide sequence may also comprise additional coding sequence encoding additional amino acids. For example, a marker sequence that facilitates purification of the fused polypeptide can be encoded. In certain embodiments of the disclosure, the marker sequence is a hexa-histidine peptide, as provided in the pQE vector (Qiagen, Inc.) and described in Gentz et al., Proc. Natl. Acad. Sci., USA 86:821-824 (1989), or an HA peptide tag (Wilson et al., Cell 37: 767 (1984), both of which may be useful in purifying polypeptide sequence fused to them. Polynucleotides of the disclosure also include, but are not limited to, polynucleotides comprising a structural gene and its naturally associated sequences that control gene expression.

The term "polynucleotide encoding a polypeptide" as used herein encompasses polynucleotides that include a sequence encoding a polypeptide of the invention, particularly a bacterial polypeptide and more particularly a polypeptide of the S. *aureus* having an amino acid sequence set out in any of the sequences of SEQ Group 2. The term also encompasses polynucleotides that include a single continuous region or discontinuous regions encoding the polypeptide (for example, polynucleotides interrupted by integrated phage, an integrated insertion sequence, an integrated vector sequence, an integrated transposon sequence, or due to RNA editing or genomic DNA reorganization) together with additional regions, that also may contain coding and/or non-coding sequences.

The disclosure further relates to variants of the polynucleotides described herein that encode variants of a polypeptides having a deduced amino acid sequence of any of the sequences of SEQ Group 2. Fragments of polynucleotides may be used, for example, to synthesize full-length polynucleotides of the invention.

Further particularly preferred embodiments are polynucleotides encoding Protein A-Sbi variants, that have the amino acid sequence of Protein A-Sbi polypeptides of any sequence from SEQ Group 2 in which several, a few, 5 to 10, 1 to 5, 1 to 3, 2, 1 or no amino acid residues are substituted, modified, deleted and/or added, in any combination. Especially preferred among these are silent substitutions, additions and deletions, that do not alter the properties and activities of Protein A-Sbi polypeptides.

Further preferred embodiments of the disclosure are polynucleotides that are at least 85% identical over their entire length to polynucleotides encoding Protein A-Sbi polypeptides having an amino acid sequence set out in any of the sequences of SEQ Group 2 , and polynucleotides that are complementary to such polynucleotides. Alternatively, most highly preferred are polynucleotides that comprise a region that is at least 90% identical over its entire length to polynucleotides encoding Protein A-Sbi polypeptides and polynucleotides complementary thereto. In this regard, polynucleotides at least 95% identical over their entire length to the same are particularly preferred. Furthermore, those with at least 97% are highly preferred among those with at least 95%, and among these those with at least 98% and at least 99% are particularly highly preferred, with at least 99% being the more preferred.

Preferred embodiments are polynucleotides encoding polypeptides that retain substantially the same biological function or activity as mature polypeptides encoded by a DNA sequences selected from SEQ Group 1.

In accordance with certain preferred embodiments of this disclosure there are provided polynucleotides that hybridize, particularly under stringent conditions, to Protein A-Sbi polynucleotide sequences, such as those polynucleotides in SEQ Group 1.

The disclosure further relates to polynucleotides that hybridize to the polynucleotide sequences provided herein. In this regard, the disclosure especially relates to polynucleotides that hybridize under stringent conditions to the polynucleotides described herein. As herein used, the terms "stringent conditions" and "stringent hybridization conditions" mean hybridization occurring only if there is at least 95% and preferably at least 97% identity between the sequences. A specific example of stringent hybridization conditions is overnight incubation at 42°C in a solution comprising: 50% formamide, 5x SSC (150mM NaCl, 15mM trisodium citrate), 50 mM sodium phosphate (pH7.6), 5x Denhardt's solution, 10% dextran sulfate, and 20 micrograms/ml of denatured, sheared salmon sperm DNA, followed by washing the hybridization support in 0.1x SSC at about 65°C. Hybridization and wash conditions are well known and exemplified in Sambrook, et al., Molecular Cloning: A Laboratory Manual, Second Edition, Cold Spring Harbor, N.Y., (1989), particularly Chapter 11 therein. Solution hybridization may also be used with the polynucleotide sequences provided by the disclosure.

The disclosure also provides a polynucleotide consisting of or comprising a polynucleotide sequence obtained by screening an appropriate library containing the complete gene for a polynucleotide sequence set forth in any of the sequences of SEQ Group 1 under stringent hybridization conditions with a probe having the sequence of said polynucleotide sequence set forth in the corresponding sequences of SEQ Group 1 or a fragment thereof; and isolating said polynucleotide sequence. Fragments useful for obtaining such a polynucleotide include, for example, probes and primers fully described elsewhere herein.

As discussed elsewhere herein regarding polynucleotide assays of the disclosure, for instance, the polynucleotides of the disclosure, may be used as a hybridization probe for RNA, cDNA and genomic DNA to isolate full-length cDNAs and genomic clones encoding Protein A-Sbi and to isolate cDNA and genomic clones of other genes that have a high identity, particularly high sequence identity, to the Protein A-Sbi sequences. Such probes generally will comprise at least 15 nucleotide residues or base pairs. Preferably, such probes will have at least 30 nucleotide residues or base pairs and may have at least 50 nucleotide residues or base pairs. Particularly preferred probes will have at least 20 nucleotide residues or base pairs and will have less than 30 nucleotide residues or base pairs.

A coding region of Protein A-Sbi genes may be isolated by screening using a DNA sequences provided in SEQ Group 1 to synthesize an oligonucleotide probe. A labeled oligonucleotide having a sequence complementary to that of a gene of the disclosure is then used to screen a library of cDNA, genomic DNA or mRNA to determine which members of the library the probe hybridizes to.

There are several methods available and well known to those skilled in the art to obtain full-length DNAs, or extend short DNAs, for example those based on the method of Rapid Amplification of cDNA ends (RACE) (see, for example, Frohman, et al., PNAS USA 85: 8998-9002, 1988). Recent modifications of the technique, exemplified by the Marathon™ technology (Clontech Laboratories Inc.) for example, have significantly simplified the search for longer cDNAs. In the Marathon™ technology, cDNAs have been prepared from mRNA extracted from a chosen tissue and an 'adaptor' sequence ligated onto each end. Nucleic acid amplification (PCR) is then carried out to amplify the "missing" 5' end of the DNA using a combination of gene specific and adaptor specific oligonucleotide primers. The PCR reaction is then repeated using "nested" primers, that is, primers designed to anneal within the amplified product (typically an adaptor specific primer that anneals further 3' in the adaptor sequence and a gene specific primer that anneals further 5' in the selected gene sequence). The products of this reaction can then be analyzed by DNA sequencing and a full-length DNA constructed either by joining the product directly to the existing DNA to give a complete sequence, or carrying out a separate full-length PCR using the new sequence information for the design of the 5' primer.

The polynucleotides and polypeptides of the disclosure may be employed, for example, as research reagents and materials for discovery of treatments of and diagnostics for diseases, particularly human diseases, as further discussed herein relating to polynucleotide assays.

The polynucleotides of the invention that are oligonucleotides derived from a sequence of SEQ Group 1 may be used in the processes herein as described, but preferably for PCR, to determine whether or not the polynucleotides identified herein in whole or in part are transcribed in bacteria in infected tissue. It is recognized that such sequences will also have utility in diagnosis of the stage of infection and type of infection the pathogen has attained.

The disclosure also provides polynucleotides that encode a polypeptide that is the mature protein plus additional amino or carboxyl-terminal amino acids, or amino acids interior to the mature polypeptide (when the mature form has more than one polypeptide chain, for instance). Such sequences may play a role in processing of a protein from precursor to a mature form, may allow protein transport, may lengthen or shorten protein half-life or may facilitate manipulation of a protein for assay or production, among other things. As generally is the case *in vivo*, the additional amino acids may be processed away from the mature protein by cellular enzymes.

For each and every polynucleotide of the disclosure there is provided a polynucleotide complementary to it. It is preferred that these complementary polynucleotides are fully complementary to each polynucleotide with which they are complementary.

A precursor protein, having a mature form of the polypeptide fused to one or more prosequences may be an inactive form of the polypeptide. When prosequences are removed such inactive precursors generally are activated. Some or all of the prosequences may be removed before activation. Generally, such precursors are called proproteins.

In addition to the standard A, G, C, T/U representations for nucleotides, the term "N" may also be used in describing certain polynucleotides of the disclosure. "N" means that any of the four DNA or RNA nucleotides may appear at such a designated position in the DNA or RNA sequence, except it is preferred that N is not a nucleic acid that when taken in combination with adjacent nucleotide positions, when read in the correct reading frame, would have the effect of generating a premature termination codon in such reading frame.

In sum, a polynucleotide of the disclosure may encode a mature protein, a mature protein plus a leader sequence (which may be referred to as a preprotein), a precursor of a mature protein having one or more prosequences that are not the leader sequences of a preprotein, or a preproprotein, which is a precursor to a proprotein, having a leader sequence and one or more prosequences, which generally are removed during processing steps that produce active and mature forms of the polypeptide.

In accordance with an aspect of the invention, there is provided the use of a polynucleotide of the invention for therapeutic or prophylactic purposes, in particular genetic immunization.

The use of a polynucleotide of the invention in genetic immunization will preferably employ a suitable delivery method such as direct injection of plasmid DNA into muscles (Wolff et al., Hum Mol Genet (1992) 1: 363, Manthorpe et al., Hum. Gene Ther. (1983) 4: 419), delivery of DNA complexed with specific protein carriers (Wu et al., J Biol Chem. (1989) 264: 16985), coprecipitation of DNA with calcium phosphate (Benvenisty & Reshef, PNAS USA, (1986) 83: 9551), encapsulation of DNA in various forms of liposomes (Kaneda et al., Science (1989) 243: 375), particle bombardment (Tang et al., Nature (1992) 356:152, Eisenbraun et al., DNA Cell Biol (1993) 12: 791) and *in vivo* infection using cloned retroviral vectors (Seeger et al., PNAS USA (1984) 81: 5849).

### Vectors, Host Cells, Expression Systems

The disclosure also relates to vectors that comprise a polynucleotide or polynucleotides of the disclosure, host cells that are genetically engineered with vectors of the disclosure and the production of polypeptides of the disclosure by recombinant techniques. Cell-free translation systems can also be employed to produce such proteins using RNAs derived from the DNA constructs of the invention.

Recombinant polypeptides of the present invention may be prepared by processes well known in those skilled in the art from genetically engineered host cells comprising expression systems. Accordingly, in a further aspect, the present disclosure relates to expression systems that comprise a polynucleotide or polynucleotides of the present invention, to host cells which are genetically engineered with such expression systems, and to the production of polypeptides of the invention by recombinant techniques.

For recombinant production of the polypeptides of the invention, host cells can be genetically engineered to incorporate expression systems or portions thereof or polynucleotides of the invention. Introduction of a polynucleotide into the host cell can be effected by methods described in many standard laboratory manuals, such as Davis, et al., BASIC METHODS IN MOLECULAR BIOLOGY, (1986) and Sambrook, et al., MOLECULAR CLONING: A LABORATORY MANUAL, 2nd Ed., Cold Spring Harbor Laboratory Press, Cold Spring Harbor, N.Y. (1989), such as, calcium phosphate transfection, DEAE-dextran mediated transfection, transvection, microinjection, cationic lipid-mediated transfection, electroporation, conjugation, transduction, scrape loading, ballistic introduction and infection.

Representative examples of appropriate hosts include bacterial cells, such as cells of streptococci, staphylococci, enterococci, *E. coli,* streptomyces, cyanobacteria, *Bacillus subtilis, Neisseria meningitidis, Haemophilus influenzae* and *Moraxella catarrhalis;* fungal cells, such as cells of a yeast, *Kluveromyces, Saccharomyces, Pichia,* a basidiomycete, *Candida albicans* and *Aspergillus*; insect cells such as cells of *Drosophila* S2 and *Spodoptera* Sf9; animal cells such as CHO, COS, HeLa, C127, 3T3, BHK, 293, CV-1 and Bowes melanoma cells; and plant cells, such as cells of a gymnosperm or angiosperm.

A great variety of expression systems can be used to produce the polypeptides of the invention. Such vectors include, among others, chromosomal-, episomal- and virus-derived vectors, for example, vectors derived from bacterial plasmids, from bacteriophage, from transposons, from yeast episomes, from insertion elements, from yeast chromosomal elements, from viruses such as baculoviruses, papova viruses, such as SV40, vaccinia viruses, adenoviruses, fowl pox viruses, pseudorabies viruses, picornaviruses, retroviruses, and alphaviruses and vectors derived from combinations thereof, such as those derived from plasmid and bacteriophage genetic elements, such as cosmids and phagemids. The expression system constructs may contain control regions that regulate as well as engender expression. Generally, any system or vector suitable to maintain, propagate or express polynucleotides and/or to express a polypeptide in a host may be used for expression in this regard. The appropriate DNA sequence may be inserted into the expression system by any of a variety of well-known and routine techniques, such as, for example, those set forth in Sambrook et al., MOLECULAR CLONING, A LABORATORYMANUAL*, (supra).*

In recombinant expression systems in eukaryotes, for secretion of a translated protein into the lumen of the endoplasmic reticulum, into the periplasmic space or into the extracellular environment, appropriate secretion signals may be incorporated into the expressed polypeptide. These signals may be endogenous to the polypeptide or they may be heterologous signals.

Polypeptides of the present invention can be recovered and purified from recombinant cell cultures by well-known methods including ammonium sulfate or ethanol precipitation, acid extraction, anion or cation exchange chromatography, phosphocellulose chromatography, hydrophobic interaction chromatography, affinity chromatography, hydroxylapatite chromatography and lectin chromatography. Most preferably, ion metal affinity chromatography (IMAC) is employed for purification. Well known techniques for refolding proteins may be employed to regenerate active conformation when the polypeptide is denatured during intracellular synthesis, isolation and or purification.

The expression system may also be a recombinant live microorganism, such as a virus or bacterium. The gene of interest can be inserted into the genome of a live recombinant virus or bacterium. Inoculation and *in vivo* infection with this live vector will lead to *in vivo* expression of the antigen and induction of immune responses. Viruses and bacteria used for this purpose are for instance: poxviruses (e.g; vaccinia, fowlpox, canarypox), alphaviruses (Sindbis virus, Semliki Forest Virus, Venezuelian Equine Encephalitis Virus), adenoviruses, adeno-associated virus, picornaviruses (poliovirus, rhinovirus), herpesviruses (varicella zoster virus, etc), *Listeria, Salmonella*, *Shigella,* BCG, streptococci. These viruses and bacteria can be virulent, or attenuated in various ways in order to obtain live vaccines. Such live vaccines also form part of the invention.

### Immunogenic compositions comprising further antigens

The immunogenic compositions of the invention are optionally combined with further antigens. The neutralisation of staphylococcal IgG binding proteins may allow an improved immune response to be generated against further antigens.

In an embodiment, the immunogenic composition of the invention comprises a further staphylococcal antigen. In an embodiment, the further staphylococcal antigen is derived from *S. aureus* or *S. epidermidis.* Examples of further antigens include staphylococcal polysaccharide such as *S. aureus* type 5 capsular polysaccharide, *S. aureus* type 8 capsular polysaccharide, PNAG which is optionally less than 50%, 40%, 30%, 20% or 10% acetylated.

Most strains of *S.aureus* that cause infection in man contain either Type 5 or Type 8 polysaccharides. Approximately 60% of human strains are Type 8 and approximately 30% are Type 5. The structures of Type 5 and Type 8 capsular polysaccharide antigens are described in Moreau et al Carbohydrate Res. 201; 285 (1990) and Fournier et al Infect. Immun. 45; 87 (1984). Both have FucNAcp in their repeat unit as well as ManNAcA which can be used to introduce a sulfhydryl group. The structures were reported as :
Type 5
   →4)-β-D-ManNAcA(3OAc)-(1 →4)-α-L-FucNAc(1 →3)-β-D-FucNAc-(1 →
Type 8
   →3)-β-D-ManNAcA(40Ac)-(1 →3)-α-L-FucNAc(1 →3)-β-D-FucNAc-(1 →

Recently (Jones Carbohydrate Research 340, 1097-1106 (2005)) NMR spectroscopy revised to structures to :
Type 5
   →4)-β-D-ManNAcA-(1 →4)-α-L-FucNAc(3OAc)-(1 →3)-β-D-FucNAc-(1 →
Type 8
   →3)-β-D-ManNAcA(4OAc)-(1 →3)-α-L-FucNAc(1 →3)-α-D-FucNAc(1 →

Polysaccharides may be extracted from the appropriate strain of *S. aureus* using method well known to the skilled man, for instance as described in US6294177. For example, ATCC 12902 is a Type 5 *S. aureus* strain and ATCC 12605 is a Type 8 *S. aureus* strain.

In an embodiment, both Type 5 and Type 8 capsular polysaccharides are present in the immunogenic composition of the invention. The immunogenic composition of the invention alternatively contains either type 5 or type 8 polysaccharide.

Polysaccharides are of native size or alternatively may be sized, for instance by microfluidisation, ultrasonic irradiation or by chemical treatment. The invention also covers oligosaccharides derived from the type 5 and 8 polysaccharides from *S. aureus.*

The type 5 and 8 polysaccharides included in the immunogenic composition of the invention are preferably conjugated to a carrier protein as described below or are alternatively unconjugated.

The immunogenic compositions of the invention alternatively contains either type 5 or type 8 polysaccharide.

PNAG is a polysaccharide adhesin and is composed of a polymer of β-(1 →6)-linked glucosamine substituted with N-acetyl and O-succinyl constituents. This polysaccharide is present in both *S.aureus* and *S. epidermidis* (Joyce et al 2003, Carbohydrate Research 338; 903; Maira-Litran et al 2002, Infect. Imun. 70; 4433).

PIA (or PNAG) may be of different sizes varying from over 400kDa to between 75 and 400kDa to between 10 and 75kDa to oligosaccharides composed of up to 30 repeat units (of β-(1 →6)-linked glucosamine substituted with N-acetyl and O-succinyl constituents). Any size of PIA polysaccharide or oligosaccharide may be use in an immunogenic composition of the invention, however a size of over 40kDa is preferred. Sizing may be achieved by any method known in the art, for instance by microfluidisation, ultrasonic irradiation or by chemical cleavage (WO 03/53462, EP497524, EP497525).

In an embodiment, the size range of PIA (PNAG) is 40-400kDa, 50-350kDa, 40-300kDa, 60-300kDa, 50-250kDa or 60-200kDa.

PIA (PNAG) can have different degree of acetylation due to substitution on the amino groups by acetate. PIA produced in vitro is almost fully substituted on amino groups (95-100%). Alternatively, a deacetylated PIA (PNAG) can be used having less than 60%, preferably less than 50%, 40%, 30%, 20%, 10% N-acetylation. Use of a deacetylated PIA (PNAG) is preferred since non-acetylated epitopes of PNAG are efficient at mediating opsonic killing of Gram positive bacteria, preferably *S. aureus* and/or *S. epidermidis.* In an embodiment, the PIA (PNAG) has a size between 40kDa and 300kDa and is deacetylated so that less than 60%, 50%, 40%, 30% or 20% of amino groups are acetylated.

The term deacetylated PNAG (dPNAG) refers to a PNAG polysaccharide or oligosaccharide in which less than 60%, 50%, 40%, 30%, 20% or 10% of the amino agroups are acetylated.

In an embodiment, PNAG is a deaceylated to form dPNAG by chemically treating the native polysaccharide. For example, the native PNAG is treated with a basic solution such that the pH rises to above 10. For instance the PNAG is treated with 0.1-5M, 0.2-4M, 0.3-3M, 0.5-2M, 0.75-1.5M or 1 M NaOH, KOH or NH₄OH. Treatment is for at least 10 or 30 minutes, or 1, 2, 3, 4, 5, 10, 15 or 20 hours at a temperature of 20-100, 25-80, 30-60 or 30-50 or 35-45 °C. dPNAG may be prepared as described in WO 04/43405.

The polysaccharide(s) included in the immunogenic composition of the invention are preferably conjugated to a carrier protein as described below or alternatively unconjugated.

In an embodiment, the immunogenic composition fo the invention comprises Type 5 and 8 capsular polysaccharides and PNAG.

In an embodiment, the immunogenic composition of the invention comprises the *S*. *aureus* 336 antigen described in US6294177.

The 336 antigen comprises β-linked hexosamine, contains no O-acetyl groups and specifically binds to antibodies to *S*. *aureus* Type 336 deposited under ATCC 55804. It is further characterised in US 2006/0228368.

In an embodiment, the 336 antigen is a polysaccharide which is of native size or alternatively may be sized, for instance by microfluidisation, ultrasonic irradiation or by chemical treatment. The invention also covers oligosaccharides derived from the 336 antigen.

The 336 antigen, where included in the immunogenic composition of the invention is preferably conjugated to a carrier protein as described below or are alternatively unconjugated.

In an embodiment, the carrier protein is independently selected from the group consisting of tetanus toxoid, diphtheria toxoid, CRM197, protein D, alpha toxin, SdrG, ClfA, IsdA, IsdB, IsdH, protein A, Sbi and a proteinA-Sbi fusion protein or fragments thereof.

In an embodiment, the polysaccharides and PNAG utilised in the invention are linked to a protein carrier which provides bystander T -cell help. Examples of these carriers which are currently commonly used for the production of polysaccharide immunogens include the Diphtheria and Tetanus toxoids (DT, DT crm197 and TT respectively), Keyhole Limpet Haemocyanin (KLH), and the purified protein derivative of Tuberculin (PPD), protein D from *Haemophilus influenzae,* pneumolysin or fragments of any of the above. Fragments suitable for use include fragments encompassing T-helper epitopes. In particular protein D fragment will preferably contain the N-terminal 1/3 of the protein. Protein D is an IgD-binding protein from *Haemophilus influenzae* (EP 0 594 610 B1) and is a potential immunogen.

Despite the common use of these carriers and their success in the induction of anti polysaccharide antibody responses they are associated with several drawbacks. For example, it is known that antigen specific immune responses may be suppressed by the presence of pre-existing antibodies directed against the carrier, in this case Tetanus toxin (Di John et al; Lancet, December 16, 1989). In the population at large, a very high percentage of people will have pre-existing immunity to both DT and TT as people are routinely vaccinated with these antigens. In the UK for example 95% of children receive the DTP vaccine comprising both DT and TT. Other authors have described the problem of epitope suppression to peptide vaccines in animal models (Sad et al, Immunology, 1991; 74:223-227; Schutze et al, J. Immunol. 135: 4,1985; 2319-2322).

An alternative staphylococcal carrier protein is alpha toxoid. The native form may be conjugated to a polysaccharide since the process of conjugation reduces toxicity. Alternatively a genetically detoxified alpha toxin such as the His35Leu or His 35 Arg variants are used as carriers since residual toxicity is lower. Alternatively the alpha toxin is chemically detoxified by treatment with a cross-linking reagent, formaldehyde or glutaraldehyde. A genetically detoxified alpha toxin is optionally chemically detoxified, preferably by treatment with a cross-linking reagent, formaldehyde or glutaraldehyde to further reduce toxicity.

The polysaccharides may be linked to the carrier protein(s) by any known method (for example, by Likhite, U.S. Patent 4,372,945 by Armor et al., U.S. Patent 4,474,757, and Jennings et al., U.S. Patent 4,356,170). Preferably, CDAP conjugation chemistry is carried out (see WO95/08348).

In CDAP, the cyanylating reagent 1-cyano-dimethylaminopyridinium tetrafluoroborate (CDAP) is preferably used for the synthesis of polysaccharide-protein conjugates. The cyanilation reaction can be performed under relatively mild conditions, which avoids hydrolysis of the alkaline sensitive polysaccharides. This synthesis allows direct coupling to a carrier protein.

The polysaccharide is solubilized in water or a saline solution. CDAP is dissolved in acetonitrile and added immediately to the polysaccharide solution. The CDAP reacts with the hydroxyl groups of the polysaccharide to form a cyanate ester. After the activation step, the carrier protein is added. Amino groups of lysine react with the activated polysaccharide to form an isourea covalent link. After the coupling reaction, a large excess of glycine is then added to quench residual activated functional groups. The product is then passed through a gel permeation column to remove unreacted carrier protein and residual reagents.

In an embodiment, the immunogenic composition of the invention comprises a protein selected from the group consisting of Ebh (WO 02/59148), Elastin binding protein (EbpS WO 98/38312), EFB (FIB) (WO 94/06830), ClfA (US6008341), ClfB (WO 99/27109), SdrC (WO 99/27109), SdrG (WO 97/48727), FnbA (including variants desribed in WO 05/116064), FbpA, IsaA/PisA (DE 199 17 098), IsdA (WO 02/59148, WO 06/59247), IsdB (WO 02/059148, including variants described in WO 05/09379 and WO 05/09378), IsdC (WO 06/59247), HarA (WO 05/09378), alpha toxin (Hla US4615884), alpha toxin H35R mutant, penicillin binding protein 4 (WO 06/33918), SsaA (WO 05/115113), Aap (WO 05/86663), RAP (WO 99/32133), AhpC and variants (WO 06/78680), SasA (WO 06/121664).

In a preferred embodiment, immunogenic composition of the invention further comprises a number of proteins equal to or greater than 2, 3, 4, 5 or 6 selected from 2, 3 or 4 of the following groups:
- group a) - at least one staphylococcal extracellular component binding protein or fragment thereof selected from the group consisting of Ebh. Elastin binding protein (EbpS), EFB (FIB), ClfA, ClfB, SdrC, SdrG, FnbA,SsaA, SasA, Aap, AhpC, penicillin binding protein 4, IsaA/PisA;
- group b) - at least one staphylococcal transporter protein or fragment thereof selected from the group consisting of IsdA, IsdB, IsdC, HarA;
- group c) - at least one staphylococcal regulator of virulence, toxin or fragment thereof selected from the group consisting of alpha toxin (Hla), alpha toxin H35R mutant, RAP.

### Vaccines

Another aspect of the invention is a vaccine comprising the immunogenic composition of the invention and a pharmaceutically acceptable carrier.

Another aspect of the disclosure relates to a method for inducing an immunological response in an individual, particularly a mammal, preferably humans, which comprises inoculating the individual with the polynucleotide and/or polypeptide or the invention, or a fragment or variant thereof, or a combination thereof as described above, adequate to produce antibody and/ or T cell immune response to protect said individual from infection, particularly bacterial infection and most particularly staphylococcal infection including *S*. *aureus* and/or *S*. *epidermidis* infection. Also provided are methods whereby such immunological response slows bacterial replication. Yet another aspect of the disclosure relates to a method of inducing immunological response in an individual which comprises delivering to such individual a nucleic acid vector, sequence or ribozyme to direct expression of polynucleotide and/or polypeptide or the invention, or a fragment or a variant thereof, for expressing polynucleotide and/or polypeptide of the invention, or a fragment or a variant thereof, or a combination thereof as described above, *in vivo* in order to induce an immunological response, such as, to produce antibody and/or T cell immune response, including, for example, cytokine-producing T cells or cytotoxic T cells, to protect said individual, preferably a human, from disease, whether that disease is already established within the individual or not. One example of administering the gene is by accelerating it into the desired cells as a coating on particles or otherwise. Such nucleic acid vector may comprise DNA, RNA, a ribozyme, a modified nucleic acid, a DNA/RNA hybrid, a DNA-protein complex or an RNA-protein complex.

A further aspect of the invention relates to an immunological composition that when introduced into an individual, preferably a human, capable of having induced within it an immunological response, induces an immunological response in such individual to a polynucleotide and/or polypeptide of the invention encoded therefrom, or a combination thereof as described above, wherein the composition comprises a recombinant polynucleotide and/or polypeptide encoded therefrom and/or comprises DNA and/or RNA which encodes and expresses an antigen of said polynucleotide, polypeptide encoded therefrom, or other polypeptide of the invention. The immunological response may be used therapeutically or prophylactically and may take the form of antibody immunity and/or cellular immunity, such as cellular immunity arising from CTL or CD4+ T cells.

A polypeptide of the invention or a fragment thereof may be fused with co-protein or chemical moiety which may or may not by itself produce antibodies, but which is capable of stabilizing the first protein and producing a fused or modified protein which will have antigenic and/or immunogenic properties, and preferably protective properties. Thus fused recombinant protein, preferably further comprises an antigenic co-protein, such as lipoprotein D from *Haemophilus influenzae,* Glutathione-S-transferase (GST) or beta-galactosidase, or any other relatively large co-protein which solubilizes the protein and facilitates production and purification thereof. Moreover, the co-protein may act as an adjuvant in the sense of providing a generalized stimulation of the immune system of the organism receiving the protein. The co-protein may be attached to either the amino- or carboxy-terminus of the first protein.

In a vaccine composition according to the invention, a polypeptide and/or polynucleotide, or a fragment, or a mimotope, or a variant thereof, or a combination thereof as described above, may be present in a vector, such as the live recombinant vectors described above for example live bacterial vectors.

Also provided by this disclosure are compositions, particularly vaccine compositions, and methods comprising the polypeptides and/or polynucleotides of the invention and immunostimulatory DNA sequences, such as those described in Sato, Y. et al. Science 273: 352 (1996).

In an embodiment, the immunogenic composition of the invention is mixed with a pharmaceutically acceptable excipient, and/or optionally with an adjuvant.

The vaccines of the present invention are optionally adjuvanted. Suitable adjuvants include an aluminum salt such as aluminum hydroxide gel (alum) or aluminium phosphate, but may also be a salt of calcium, magnesium, iron or zinc, or may be an insoluble suspension of acylated tyrosine, or acylated sugars, cationically or anionically derivatized polysaccharides, or polyphosphazenes.

Optionally the adjuvant is a preferential inducer of either a TH1 or a TH2 type of response. High levels of Th1-type cytokines tend to favor the induction of cell mediated immune responses to a given antigen, whilst high levels of Th2-type cytokines tend to favour the induction of humoral immune responses to the antigen.

It is important to remember that the distinction of Th1 and Th2-type immune response is not absolute. In reality an individual will support an immune response which is described as being predominantly Th1 or predominantly Th2. However, it is often convenient to consider the families of cytokines in terms of that described in murine CD4 +ve T cell clones by Mosmann and Coffman (Mosmann, T.R. and Coffman, R.L. (1989) TH1 and TH2 cells: different patterns of lymphokine secretion lead to different functional properties. Annual Review of Immunology, 7, p145-173). Traditionally, Th1-type responses are associated with the production of the INF-γ and IL-2 cytokines by T-lymphocytes. Other cytokines often directly associated with the induction of Th1-type immune responses are not produced by T-cells, such as IL-12. In contrast, Th2-type responses are associated with the secretion of II-4, IL-5, IL-6, IL-10. Suitable adjuvant systems which promote a predominantly Th1 response include: Monophosphoryl lipid A or a derivative thereof, particularly 3-de-O-acylated monophosphoryl lipid A (3D-MPL) (for its preparation see GB 2220211 A); and a combination of monophosphoryl lipid A, preferably 3-de-O-acylated monophosphoryl lipid A, together with either an aluminium salt (for instance aluminium phosphate or aluminium hydroxide) or an oil-in-water emulsion. In such combinations, antigen and 3D-MPL are contained in the same particulate structures, allowing for more efficient delivery of antigenic and immunostimulatory signals. Studies have shown that 3D-MPL is able to further enhance the immunogenicity of an alum-adsorbed antigen [Thoelen et al. Vaccine (1998) 16:708-14; EP 689454-B1].

An enhanced system involves the combination of a monophosphoryl lipid A and a saponin derivative, particularly the combination of QS21 and 3D-MPL as disclosed in WO 94/00153, or a less reactogenic composition where the QS21 is quenched with cholesterol as disclosed in WO 96/33739. A particularly potent adjuvant formulation involving QS21, 3D-MPL and tocopherol in an oil in water emulsion is described in WO 95/17210, and is a preferred formulation. Preferably the vaccine additionally comprises a saponin, more preferably QS21. The formulation may also comprise an oil in water emulsion and tocopherol (WO 95/17210). The present invention also provides a method for producing a vaccine formulation comprising mixing a protein of the present invention together with a pharmaceutically acceptable excipient, such as 3D-MPL. Unmethylated CpG containing oligonucleotides (WO 96/02555) are also preferential inducers of a TH1 response and are suitable for use in the present invention.

The vaccine preparations of the present disclosure may be used to protect or treat a mammal susceptible to infection, by means of administering said vaccine via systemic or mucosal route. These administrations may include injection *via* the intramuscular, intraperitoneal, intradermal or subcutaneous routes; or *via* mucosal administration to the oral/alimentary, respiratory, genitourinary tracts. Although the vaccine of the disclosure may be administered as a single dose, components thereof may also be co-administered together at the same time or at different times. For co-administration, the optional Th1 adjuvant may be present in any or all of the different administrations. In addition to a single route of administration, 2 different routes of administration may be used. In addition, the vaccines of the disclosure may be administered IM for priming doses and IN for booster doses.

The amount of antigen in each vaccine dose is selected as an amount which induces an immunoprotective response without significant, adverse side effects in typical vaccines. Such amount will vary depending upon which specific immunogen is employed and how it is presented. Generally, it is expected that for polysaccharide or oligosaccharide antigens each dose will comprise 0.1-100 µg of saccharide, 0.1-50 µg for saccharide conjugates, 0.1-10 µg, 1-10 µg, or 1 to 5 µg is.

The content of protein antigens in the vaccine will typically be in the range 1-100µg, preferably 5-50µg, most typically in the range 5 - 25µg. Following an initial vaccination, subjects may receive one or several booster immunizations adequately spaced.

Vaccine preparation is generally described in Vaccine Design ("The subunit and adjuvant approach" (eds Powell M.F. & Newman M.J.) (1995) Plenum Press New York). Encapsulation within liposomes is described by Fullerton, US Patent 4,235,877.

The vaccines of the present invention may be stored in solution or lyophilized. Optionally the solution is lyophilized in the presence of a sugar such as sucrose, trehalose or lactose. Optionally they are lyophilized and extemporaneously reconstituted prior to use. Lyophilizing may result in a more stable composition (vaccine).

A further aspect of the invention relates to a process for making the vaccine of the invention comprising the step of adding a pharmaceutically acceptable excipient to the immunogenic composition of the invention.

A further aspect of the invention relates to the immunogenic composition, polypeptide or polynucleotide of the invention for use in the treatment of prevention of staphylococcal disease.

A further aspect of the invention relates to a use of the immunogenic composition, polypeptide or polynucleotide of the invention in the preparation of a medicament for the treatment or prevention of staphylococcal disease.

A further aspect of the disclosure relates to a method of treating or preventing staphylococcal disease comprising administering the immunogenic composition, vaccine, polypeptide or polynucleotide of the invention to a patient in need thereof.

The disclosure also encompasses method of treatment or staphylococcal infection, particularly hospital acquired nosocomial infections.

The immunogenic composition or vaccine of the invention is particularly advantageous to use in cases of elective surgery. Such patients will know the date of surgery in advance and could be inoculated in advance. Since it is not know whether the patient will be exposed to *S.aureus* or *S*. *epidermidis* infection, it is preferred to inoculate with a vaccine of the invention that protects against both, as described above. Preferably adults over 16 awaiting elective surgery are treated with the immunogenic compositions and vaccines of the invention.

It is also advantageous to inoculate health care workers with the vaccine of the invention.

The vaccine preparations of the present invention may be used to protect or treat a mammal susceptible to infection, by means of administering said vaccine via systemic or mucosal route. These administrations may include injection *via* the intramuscular, intraperitoneal, intradermal or subcutaneous routes; or *via* mucosal administration to the oral/alimentary, respiratory, genitourinary tracts.

The amount of antigen in each vaccine dose is selected as an amount which induces an immunoprotective response without significant, adverse side effects in typical vaccines. Such amount will vary depending upon which specific immunogen is employed and how it is presented. The protein content of the vaccine will typically be in the range1 -100µg, preferably 5-50µg, most typically in the range 10 - 25µg. Generally, it is expected that each dose will comprise 0.1-100 µg of polysaccharide where present, preferably 0.1-50 µg, preferably 0.1-10 µg, of which 1 to 5 µg is the most preferable range. An optimal amount for a particular vaccine can be ascertained by standard studies involving observation of appropriate immune responses in subjects. Following an initial vaccination, subjects may receive one or several booster immunisations adequately spaced.

Although the vaccines of the present invention may be administered by any route, administration of the described vaccines into the skin (ID) forms one embodiment of the present invention. Human skin comprises an outer "horny" cuticle, called the stratum corneum, which overlays the epidermis. Underneath this epidermis is a layer called the dermis, which in turn overlays the subcutaneous tissue. Researchers have shown that injection of a vaccine into the skin, and in particular the dermis, stimulates an immune response, which may also be associated with a number of additional advantages. Intradermal vaccination with the vaccines described herein forms a preferred feature of the present invention.

The conventional technique of intradermal injection, the "mantoux procedure", comprises steps of cleaning the skin, and then stretching with one hand, and with the bevel of a narrow gauge needle (26-31 gauge) facing upwards the needle is inserted at an angle of between 10-15°. Once the bevel of the needle is inserted, the barrel of the needle is lowered and further advanced whilst providing a slight pressure to elevate it under the skin. The liquid is then injected very slowly thereby forming a bleb or bump on the skin surface, followed by slow withdrawal of the needle.

More recently, devices that are specifically designed to administer liquid agents into or across the skin have been described, for example the devices described in WO 99/34850 and EP 1092444, also the jet injection devices described for example in WO 01/13977; US 5,480,381, US 5,599,302, US 5,334,144, US 5,993,412, US 5,649,912, US 5,569,189, US 5,704,911, US 5,383,851, US 5,893,397, US 5,466,220, US 5,339,163, US 5,312,335, US 5,503,627, US 5,064,413, US 5,520, 639, US 4,596,556, US 4,790,824, US 4,941,880, US 4,940,460, WO 97/37705 and WO 97/13537. Alternative methods of intradermal administration of the vaccine preparations may include conventional syringes and needles, or devices designed for ballistic delivery of solid vaccines (WO 99/27961), or transdermal patches (WO 97/48440; WO 98/28037); or applied to the surface of the skin (transdermal or transcutaneous delivery WO 98/20734 ; WO 98/28037).

When the vaccines of the present invention are to be administered to the skin, or more specifically into the dermis, the vaccine is in a low liquid volume, particularly a volume of between about 0.05 ml and 0.2 ml.

The content of antigens in the skin or intradermal vaccines of the present invention may be similar to conventional doses as found in intramuscular vaccines (see above). However, it is a feature of skin or intradermal vaccines that the formulations may be "low dose". Accordingly the protein antigens in "low dose" vaccines are preferably present in as little as 0.1 to 10µg, preferably 0.1 to 5 µg per dose; and the polysaccharide (preferably conjugated) antigens may be present in the range of 0.01-1µg, and preferably between 0.01 to 0.5 µg of polysaccharide per dose.

As used herein, the term "intradermal delivery" means delivery of the vaccine to the region of the dermis in the skin. However, the vaccine will not necessarily be located exclusively in the dermis. The dermis is the layer in the skin located between about 1.0 and about 2.0 mm from the surface in human skin, but there is a certain amount of variation between individuals and in different parts of the body. In general, it can be expected to reach the dermis by going 1.5 mm below the surface of the skin. The dermis is located between the stratum corneum and the epidermis at the surface and the subcutaneous layer below. Depending on the mode of delivery, the vaccine may ultimately be located solely or primarily within the dermis, or it may ultimately be distributed within the epidermis and the dermis.

A further embodiment of the disclosure is a method of preventing or treating staphylococcal infection comprising the step of administering the vaccine of the invention to a patient in need thereof, for example a patient awaiting elective surgery.

The term 'staphylococcal infection' encompasses infection caused by *S.aureus* and/or *S*. *epidermidis* and other staphylococcal strains capable of causing infection in a mammalian, preferably human host.

In order that this invention may be better understood, the following examples are set forth. These examples are for purposes of illustration only, and are not to be construed as limiting the scope of the invention in any manner.

### Examples

### Example 1 Recombinant expression in E.coli and purification of Protein A-Sbi Fusion Protein

A 65kDa Protein A-Sbi fusion protein was made by fusing a fragment of protein A gene to a fragment of an gene Sbi, cloning in the expression vector TCMP14 and expressing the fusion protein in *E. coli.*

The *S*. *aureus* Protein A fragment, encoding a 43kDa fragment of Protein A included the five IgG-binding domains but not including the signal peptide or the C terminal wall anchor region, was amplified from NCTC8325 DNA strain (ATCC35556D) as a 1164 base pair fragment. The primers used were:-
ggaattc c**atatg** GCGCAACACGATGAAGCTC (including an Ndel site in bold)
   and
cgc **ggatcc** GCCGACATGTACTCCGTTACCATC (including a BamHI site in bold)

The Sbi fragment, encoding a 20kDa fragment of Sbi including the two IgG binding domains but excluding the β-2-glycoprotein 1 IgG binding domain, was amplified from NCTC8325 DNA strain (ATCC35556D) as a 522 base pair fragment. The primers used were:
cgc **ggatcc**-AGTGAAAACACGCAACAAACTTC (including a BamH1 site in bold)
   and
gc **tctaga tta actagt** TGCTTTTTCAATTGAAACTTTTTCTAC (including a Xba1 site and a stop codon, underlined in bold and a Spe1 site in bold)

The two fragments were cloned into a TCMP14 vector using Ndel, BamHI and SpeI cloning sites and were transformed in *E.coli* AR58.

Bacteria cultures (4x250 ml) were performed at 30°C in LBT + kanamycin 50 µg/ml. Protein expression was induced at the temperature of 42°C for four hours. The bacterial culture was centrifuged to form bacterial cell pellets and pellets were conserved at - 20°C until extraction. Figure 2 shows a coomassie strained 4-20% PAGE showing that Protein A-Sbi expression is seen in transformed cells after 4 hours at the induction temperature. A new band of 65kDa appears in lane 5 containing bacteria incubated at the induction temperature for 4 hours.

The bacterial pellet (corresponding to 4 x 250 ml of bacterial culture) was resuspended in binding buffer containing 20 mM phosphate (Na2HPO4/NaH2PO4), 500 mM NaCl, pH 7.4. As proteins are sensitive to proteases, protease inhibitor (Pefablock 1mM) was added to the suspension. Then bacteria were lysed by 4 passages at the French Press. The lysate was centrifuged for 20 minutes at 13,000rpm and the supernatent was used for the purification. A Hi-Trap chelating HP (5 ml) column (Amersham Biosciences) was used for the purification. The supernatant was loaded onto the column which was subsequently washed with 20 mM phosphate (Na2HPO4/NaH2PO4), 500 mM NaCl, pH 7.4. The bound fusion protein was then eluted with elution buffer containing: 20mM phosphate (Na2HPO4/NaH2PO4), 500 mM NaCl, 100 mM imidazole, pH 7.4. After purification, eluted fractions were pooled, dialysed against 50mM phosphate (Na2HPO4/NaH2PO4), 150mM NaCl, pH 7.4, filtrated (0.22µm) and quantified using kit BCA^{™} Protein Assay (Pierce). The yield of the purification was 4.73 mg.

SDS-PAGE and Western blot analysis were performed on the purified protein A-SBI. Figure 3 shows a coomassie stained 4-20% PAGE. A pure band of 65kDa was present in the purified protein samples.

### Example 2 Evaluation of the proteinA-SBI fusion protein in a mice mortality model induced by S. aureus intraperitoneal infection

### Method

Groups of 25 4 week old female CD1 mice were immunized intramuscularly three times (days 0, 14 and 28) with 8 µg of proteinA (ProtA) or ProtA-SBI fusion protein, both adjuvanted in AlPO₄. Control mice were immunised with the equivalent amount of AlPO₄ adjuvant or killed whole cell *S*. *aureus* serotype 5 Reynolds (5 10⁸ CFU) adjuvanted with AlPO₄. On day 42, mice were challenged intraperitoneally with 500 µl of *S*. *aureus* strain 5 Reynolds (3 10⁶ CFU) supplemented with 5% of mucin. Mortality of mice was followed until 4 days after challenge.

### Results

The results are shown in Figure 4. As expected, no protection was observed in mice immunized with AlPO4 alone. A very good protection was observed in positive control group immunized with the homologous killed whole cell. No protection was observed after immunization with the ProtA. Although not statistically significant, a increased survival rate of 24% was obtained in mice immunized with the ProtA-SBI construct.

### Conclusion

Immunisation with the combination of protein A and Sbi IgG domains in the fusion protein was able to provide greater protection than immunisation with protein A alone. The protection against *S*. *aureus* infection induced by immunization with the proteinA-SBI fusion protein in this model suggests that this antigen may be used in combination with other candidates in order to obtain an effective *S*. *aureus* vaccine.

### Example 3 Bioinformatic analysis of protein A and Sbi sequences

The sequences of Sbi from *S*. *aureus* strains Col, Mu50, NCTC8325, N315, Mw2, MRSA252 and MSSA476 were compared and the percentage identity was calculated using the ClustalW program. The alignment is shown in Figure 5 and the sequences were found to share 92-100% identity.

The IgG binding domains were identified as shown in Table 1.

| **Strain** | **Domain** | **Begin (aa)** | **End (aa)** | **Begin (nT)** | **End (nT)** |
|---|---|---|---|---|---|
| **NCTC8325** | Dom1 | 43 | 94 | 127 | 282 |
| | Dom2 | 95 | 148 | 283 | 444 |
| **Mu50** | Dom1 | 45 | 96 | 133 | 288 |
| | Dom2 | 97 | 150 | 289 | 450 |
| **N315** | Dom1 | 43 | 94 | 127 | 282 |
| | Dom2 | 95 | 148 | 283 | 444 |
| **Mw2** | Dom1 | 43 | 94 | 127 | 282 |
| | Dom2 | 95 | 148 | 283 | 444 |
| **Col** | Dom1 | 43 | 94 | 127 | 282 |
| | Dom2 | 95 | 148 | 283 | 444 |
| **MRSA252** | Dom1 | 43 | 94 | 127 | 282 |
| | Dom2 | 95 | 148 | 283 | 444 |
| **MSSA476** | Dom1 | 43 | 94 | 127 | 282 |
| | Dom2 | 95 | 148 | 283 | 444 |

The sequences of Protein A from *S*. *aureus* strains Col, Mu50, NCTC8325, N315, Mw2, MRSA252 and MSSA476 were compared and the percentage identity was calculated using the ClustalW program. The alignment is shown in Figure 4 and the sequences were found to share 91-100% identity. The IgG binding domains are identified in Table 2.

| **Strain** | **Domain** | **Begin (aa)** | **End (aa)** | **Begin (nT)** | **End (nT)** |
|---|---|---|---|---|---|
| **NCTC8325** | Dom1 | 35 | 88 | 103 | 264 |
| | Dom2 | 96 | 149 | 286 | 447 |
| | Dom3 | 154 | 207 | 460 | 621 |
| | Dom4 | 212 | 265 | 634 | 795 |
| | Dom5 | 270 | 323 | 808 | 969 |
| **Mu50** | Dom1 | 35 | 88 | 103 | 264 |
| | Dom2 | 96 | 149 | 286 | 447 |
| | Dom3 | 154 | 207 | 460 | 621 |
| | Dom4 | 212 | 265 | 634 | 795 |
| **N315** | Dom1 | 35 | 88 | 103 | 264 |
| | Dom2 | 96 | 149 | 286 | 447 |
| | Dom3 | 154 | 207 | 460 | 621 |
| | Dom4 | 212 | 265 | 634 | 795 |
| **Mw2** | Dom1 | 35 | 88 | 103 | 264 |
| | Dom2 | 96 | 149 | 286 | 447 |
| | Dom3 | 154 | 207 | 460 | 621 |
| | Dom4 | 212 | 265 | 634 | 795 |
| | Dom5 | 270 | 323 | 808 | 969 |
| **COL** | Dom1 | 35 | 88 | 103 | 264 |
| | Dom2 | 96 | 149 | 286 | 447 |
| | Dom3 | 154 | 207 | 460 | 621 |
| | Dom4 | 212 | 265 | 634 | 795 |
| | Dom5 | 270 | 323 | 808 | 969 |
| **MRSA252** | Dom1 | 35 | 88 | 103 | 264 |
| | Dom2 | 96 | 149 | 286 | 447 |
| | Dom3 | 154 | 207 | 460 | 621 |
| | Dom4 | 212 | 265 | 634 | 795 |
| | Dom5 | 270 | 323 | 808 | 969 |
| **MSSA476** | Dom1 | 35 | 88 | 103 | 264 |
| | Dom2 | 96 | 149 | 286 | 447 |
| | Dom3 | 154 | 207 | 460 | 621 |
| | Dom4 | 212 | 265 | 634 | 795 |
| | Dom5 | 270 | 323 | 808 | 969 |
| **V8** | Dom1 | 47 | 100 | 139 | 300 |
| | Dom2 | 108 | 161 | 336 | 483 |
| | Dom3 | 166 | 219 | 496 | 657 |
| | Dom4 | 224 | 277 | 670 | 831 |

### Example 4 Construction of a fusion protein containing ProtA-SdrG-Sbi (+/- 150 kDa)

The sdrG gene was cloned in the Protein A - Sbi construct described in Example 1, by inserting sdrG into the *BamHI* site between gene ProtA and gene Sbi to form a ProtA-SdrG-Sbi construct.

SdrG was amplified by PCR from genomic DNA of the strain *S*. *epidermidis* 12228 using the following primers:
Primer 1 : fus SdrG28 - 01 **(BamHI).**
   *CGC**GGATCC**GAGAAGAATTCAGTACAAGAC*
Primer 2 : fus SdrG28 - 02 **(BamHI).**
   *CGC****GGA*T*CC***TT*CG*T*CA*T*CA*T*AG*T*A*T*CCG*TT*ATC*

The resultant construct had the sequence of SEQ ID NO: 75. This construct was used to express ProteinA-SdrG-Sbi fusion protein using the protocol described in example 1. The expression resulted in a 150kDa protein protein A-SdrG-Sbi protein which was visualized by running on a 4-20% polyacrylamide gel and staining with commassie blue or by western blotting using an anti-His tag antibody.

### Sequences

SEQ ID NO 1 Sbi domain 1
TQNNYVTDQQKAFYQVLHLKGITEEQRNQYIKTLREHPERAQEVFSESLKDS
SEQ ID NO 2 Sbi domain 2
KNPDRRVAQQNAFYNVLKNDNLTEQEKNNYIAQIKENPDRSQQVWVESVQSSKA
SEQ ID NO 3 SBI domains 1 and 2 
TQNNYVTDQQKAFYQVLHLKGITEEQRNQYIKTLREHPERAQEVFSESLKDSKN
PDRRVAQQNAFYNVLKNDNLTEQEKNNYIAQIKENPDRSQQVWVESVQSSKA
SEQ ID NO 4 Sbi part of fusion protein 
SENTQQTSTKHQTTQNNYVTDQQKAFYQVLHLKGITEEQRNQYIKTLREHPERAQEVFSE
SLKDSKNPDRRVAQQNAFYNVLKNDNLTEQEKNNYIAQIKENPDRSQQVWVESVQSSKAK
ERQNIENADKAIKDFQDNKAPHDKSAAYEANSKLPKDLRDKNNRFVEKVSIEKA
SEQ ID NO 5 Sbi Col
SEQ ID NO 6 Sbi Mu50 
MHMKNKYISKLLVGAATITLATMISNGEAKASENTQQTSTKHQTTQNNYVTDQQKAFYQV
LHLKGITEEQRNQYIKTLREHPERAQEVFSESLKDSKNPDRRVAQQNAFYNVLKNDNLTE
QEKNNYIAQIKENPDRSQQVWVESVQSSKAKERQNIENADKAIKDFQDNKAPHDKSAAYE
ANSKLPKDLRDKNNRFVEKVSIEKAIVRHDERVKSANDAISKLNEKDSIENRRLAQREVN
KAPMDVKEHLQKQLDALVAQKDAEKKVAPKVEAPQIQSPQIEKPKAESPKVEVPQSKLLG
YYQSLKDSFNYGYKYLTDTYKSYKEKYDTAKYYYNTYYKYKGAIDQTVLTVLGSGSKSYI
QPLKVDDKNGYLAKSYAQVRNYVTESINTGKVLYTFYQNPTLVKTAIKAQETASSIKNTL
SNLLSFWK
SEQ ID NO 7 sbi MRSA252 
MKNKYISKLLVGAATITLATMISNGEAKASENTQQTSTKHQTTQNNYVTDQQKAFYQVLH
LKGITEEQRNQYIKTLREHPERAQEVFSESLKDSKNPDRRVAQQNAFYNVLKNDNLTEQE
KNNYIAQIKENPDRSQQVWVESVQSSKAKERQNIENADKAIKDFQDNKAPHDKSAAYEAN
SKLPKDLRDKNNRFVEKVSIEKAIVRHDERVKSANDAISKLNEKDSIENRRLAQREVNKA
PMDVQKHLQKQLDALVAQKDAEKKVAPKVEAPQIQSPQIEKPKAESPKVEVPQIQSPKVE
VPQSKLLGYYQSLKDSFNYGYKYLTDTYKSYKEKYDTAKYYYNKYYQYKGLIDKTVLTTI
GSGYGSYIKPLEVSKESGNLAKSYAQVRNYVTESINTGKVLYAFYQKPELVKTAIKAQET
ATTFKNALTGIFKSFWK
SEQ ID NO 8 sbi MSSA476 
MKNKYISKLLVGAATITLATMISNGEAKASENTQQTSTKHQTTQNNYVTDQQKAFYQVLH
LKGITEEQRNQYIKTLREHPERAQEVFSESLKDSKNPDRRVAQQNAFYNVLKNDNLTEQE
KNNYIAQIKENPDRSQQVWVESVQSSKAKERQNIENADKAIKDFQDNKAPHDKSAAYEAN
SKLPKDLRDKNNRFVEKVSIEKAIVRHDERVKSANDAISKLNVKDSIENRRLAQREVNKA
PMDVKEHLQKQLDALVAQKDAEKKVAPKVEAPQIQSPQIEKPKAESPKVEVPQIQSPKVE
VPQSKLLGYYQSLKDSFNYGYKYLTDTYKSYKEKYDTAKYYYNTYYKYKGAIDKAVLTLL
GDGSKSYIQPLKVDDKNGYLAKSYAQVRNYVTESINTGKVLYTFYQNPTLVKTAIKAQET
ASSIKNTITGLFNSFWK
SEQ ID NO 9 sbi MW2 
MKNKYISKLLVGAATITLATMISNGEAKASENTQQTSTKHQTTQNNYVTDQQKAFYQVLH
LKGITEEQRNQYIKTLREHPERAQEVFSESLKDSKNPDRRVAQQNAFYNVLKNDNLTEQE
KNNYIAQIKENPDRSQQVWVESVQSSKAKERQNIENADKAIKDFQDNKAPHDKSAAYEAN
SKLPKDLRDKNNRFVEKVSIEKAIVRHDERVKSANDAISKLNVKDSIENRRLAQREVNKA
PMDVKEHLQKQLDALVAQKDAEKKVAPKVEAPQIQSPQIEKPKAESPKVEVPQIQSPKVE
VPQSKLLGYYQSLKDSFNYGYKYLTDTYKSYKEKYDTAKYYYNTYYKYKGAIDKAVLTLL
GDGSKSYIQPLKVDDKNGYLAKSYAQVRNYVTESINTGKVLYTFYQNPTLVKTAIKAQET
ASSIKNTITGLFNSFWK
SEQ ID NO 10 sbi N315 
MKNKYISKLLVGAATITLATMISNGEAKASENTQQTSTKHQTTQNNYVTDQQKAFYQVLH
LKGITEEQRNQYIKTLREHPERAQEVFSESLKDSKNPDRRVAQQNAFYNVLKNDNLTEQE
KNNYIAQIKENPDRSQQVWVESVQSSKAKERQNIENADKAIKDFQDNKAPHDKSAAYEAN
SKLPKDLRDKNNRFVEKVSIEKAIVRHDERVKSANDAISKLNEKDSIENRRLAQREVNKA
PMDVKEHLQKQLDALVAQKDAEKKVAPKVEAPQIQSPQIEKPKAESPKVEVPQIQSPKVE
VPQSKLLGYYQSLKDSFNYGYKYLTDTYKSYKEKYDTAKYYYNTYYKYKGAIDQTVLTVL
GSGSKSYIQPLKVDDKNGYLAKSYAQVRNYVTESINTGKVLYTFYQNPTLVKTAIKAQET
ASSIKNTLSNLLSFWK
SEQ ID NO 11 sbi NCTC8325
SEQ ID NO 12 SPA domain 1
NAAQHDEAQQNAFYQVLNMPNLNADQRNGFIQSLKDDPSQSANVLGEAQKLNDS
SEQ ID NO 13 SPA domain 2 (V8)
QQNKFNKDQQSAFYEILNMPNLNEEQRNGFIQSLKDDPSQSTNVLGEAKKLNES
SEQ ID NO 14 domain 2 (Mu50)
QQNNFNKDQQSAFYEILNMPNLNEAQRNGFIQSLKDDPSQSTNVLGEAKKLNES
SEQ ID NO 15 domain 3 (MRSA)
ADNNFNKEQQNAFYEILNMPNLNEEQRNGFIQSLKDDPSQSANLLAEAKKLNES
SEQ ID NO 16 domain 3 (V8)
ADNNFNKEQQNAFYEILNMPNLNEEQRNGFIQSLKDDPSQSANLLAEAKKLNDA
SEQ ID NO 17 domain 3 (MU50)
ADNNFNKEQQNAFYEILNMPNLNEEQRNGFIQSLKDDPSQSANLLSEAKKLNES
SEQ ID NO 18 domain 4 (MRSA)
ADNKFNKEQQNAFYEILHLPNLNEEQRNGFIQSLKDDPSQSANLLAEAKKLNDA
SEQ ID NO 19 domain 4 (V8)
ADNKFNKEQQNAFYEILHLPNLTEEQRNGFIQSLKDDPSVSKEILAEAKKLNDA
SEQ ID NO 20 domain 4 (MU50)
ADNKFNKEQQNAFYEILHLPNLNEEQRNGFIQSLKDDPSVSKEILAEAKKLNDA
SEQ ID NO 21 domain 5
ADNKFNKEQQNAFYEILHLPNLTEEQRNGFIQSLKDDPSVSKEILAEAKKLNDA
SEQ ID NO 22 domains 1+2 
MAQHDEAQQNAFYQVLNMPNLNADQRNGFIQSLKDDPSQSANVLGEAQKLNDSQ
APKADAQQNNFNKDQQSAFYEILNMPNLNEAQRNGFIQSLKDDPSQSTNVLGEA
KKLNES
SEQ ID NO 23 domains 2 -3 
QQNNFNKDQQSAFYEILNMPNLNEAQRNGFIQSLKDDPSQSTNVLGEA
KKLNESQAPKADNNFNKEQQNAFYEILNMPNLNEEQRNGFIQSLKDDPSQSANL
LSEAKKLNES
SEQ ID NO 24 domains 3-4 
ADNNFNKEQQNAFYEILNMPNLNEEQRNGFIQSLKDDPSQSANL
LSEAKKLNESQAPKADNKFNKEQQNAFYEILHLPNLNEEQRNGFIQSLKDDPSQ
 SANLLAEAKKLNDA
SEQ ID NO 25 domains 4-5 
ADNKFNKEQQNAFYEILHLPNLNEEQRNGFIQSLKDDPSQ
SANLLAEAKKLNDAQAPKADNKFNKEQQNAFYEILHLPNLTEEQRNGFIQSLKD
DPSVSKEILAEAKKLNDAQA
SEQ ID NO 26 domains 1-3 
MAQHDEAQQNAFYQVLNMPNLNADQRNGFIQSLKDDPSQSANVLGEAQKLNDSQ
APKADAQQNNFNKDQQSAFYEILNMPNLNEAQRNGFIQSLKDDPSQSTNVLGEA
KKLNESQAPKADNNFNKEQQNAFYEILNMPNLNEEQRNGFIQSLKDDPSQSANL
LSEAKKLNES
SEQ ID NO 27 domains 2-4 
QQNNFNKDQQSAFYEILNMPNLNEAQRNGFIQSLKDDPSQSTNVLGEA
KKLNESQAPKADNNFNKEQQNAFYEILNMPNLNEEQRNGFIQSLKDDPSQSANL
LSEAKKLNESQAPKADNKFNKEQQNAFYEILHLPNLNEEQRNGFIQSLKDDPSQ
SANLLAEAKKLNDA
SEQ ID NO 28 domains 3-5 
QAPKADNNFNKEQQNAFYEILNMPNLNEEQRNGFIQSLKDDPSQSANL
LSEAKKLNESQAPKADNKFNKEQQNAFYEILHLPNLNEEQRNGFIQSLKDDPSQ
SANLLAEAKKLNDAQAPKADNKFNKEQQNAFYEILHLPNLTEEQRNGFIQSLKD
DPSVSKEILAEAKKLNDAQA
SEQ ID NO 29 domains 1-4 
AQHDEAQQNAFYQVLNMPNLNADQRNGFIQSLKDDPSQSANVLGEAQKLNDSQ
APKADAQQNNFNKDQQSAFYEILNMPNLNEAQRNGFIQSLKDDPSQSTNVLGEA
KKLNESQAPKADNNFNKEQQNAFYEILNMPNLNEEQRNGFIQSLKDDPSQSANL
LSEAKKLNESQAPKADNKFNKEQQNAFYEILHLPNLNEEQRNGFIQSLKDDPSQ
SANLLAEAKKLNDA
SEQ ID NO 30 domains 2-5 
QQNNFNKDQQSAFYEILNMPNLNEAQRNGFIQSLKDDPSQSTNVLGEA
KKLNESQAPKADNNFNKEQQNAFYEILNMPNLNEEQRNGFIQSLKDDPSQSANL
LSEAKKLNESQAPKADNKFNKEQQNAFYEILHLPNLNEEQRNGFIQSLKDDPSQ
SANLLAEAKKLNDAQAPKADNKFNKEQQNAFYEILHLPNLTEEQRNGFIQSLKD
DPSVSKEILAEAKKLNDAQA
SEQ ID NO 31 domains 1-5 AQHDEAQQNAFYQVLNMPNLNADQRNGFIQSLKDDPSQSANVLGEAQKLNDSQ 
APKADAQQNNFNKDQQSAFYEILNMPNLNEAQRNGFIQSLKDDPSQSTNVLGEA
KKLNESQAPKADNNFNKEQQNAFYEILNMPNLNEEQRNGFIQSLKDDPSQSANL
LSEAKKLNESQAPKADNKFNKEQQNAFYEILHLPNLNEEQRNGFIQSLKDDPSQ
SANLLAEAKKLNDAQAPKADNKFNKEQQNAFYEILHLPNLTEEQRNGFIQSLKD
DPSVSKEILAEAKKLNDAQA
SEQ ID NO 32 protein A part of fusion protein 
MAQHDEAQQNAFYQVLNMPNLNADQRNGFIQSLKDDPSQSANVLGEAQKLNDSQ
APKADAQQNNFNKDQQSAFYEILNMPNLNEAQRNGFIQSLKDDPSQSTNVLGEA
KKLNESQAPKADNNFNKEQQNAFYEILNMPNLNEEQRNGFIQSLKDDPSQSANL
LSEAKKLNESQAPKADNKFNKEQQNAFYEILHLPNLNEEQRNGFIQSLKDDPSQ
SANLLAEAKKLNDAQAPKADNKFNKEQQNAFYEILHLPNLTEEQRNGFIQSLKD
DPSVSKEILAEAKKLNDAQAPKEEDNNKPGKEDNNKPGKEDNNKPGKEDNNKPG
KEDNNKPGKEDGNKPGKEDNKKPGKEDGNKPGKEDNKKPGKEDGNKPGKEDGNK
PGKEDGNGVHVG
SEQ ID NO 33 SPA NCTC8425 
MKKKNIYSIRKLGVGIASVTLGTLLISGGVTPAANAAQHDEAQQNAFYQVLNMPNLNADQ
RNGFIQSLKDDPSQSANVLGEAQKLNDSQAPKADAQQNNFNKDQQSAFYEILNMPNLNEA
QRNGFIQSLKDDPSQSTNVLGEAKKLNESQAPKADNNFNKEQQNAFYEILNMPNLNEEQR
NGFIQSLKDDPSQSANLLSEAKKLNESQAPKADNKFNKEQQNAFYEILHLPNLNEEQRNG
FIQSLKDDPSQSANLLAEAKKLNDAQAPKADNKFNKEQQNAFYEILHLPNLTEEQRNGFI
QSLKDDPSVSKEILAEAKKLNDAQAPKEEDNNKPGKEDNNKPGKEDNNKPGKEDNNKPGK
EDNNKPGKEDGNKPGKEDNKKPGKEDGNKPGKEDNKKPGKEDGNKPGKEDGNKPGKEDGN
GVHVVKPGDTVNDIAKANGTTADKIAADNKLADKNMIKPGQELVVDKKQPANHADANKAQ
ALPETGEENPFIGTTVFGGLSLALGAALLAGRRREL
SEQ ID NO 34 SPA Mu50 
MKKKNIYSIRKLGVGIASVTLGTLLISGGVTPAANAAQHDEAQQNAFYQVLNMPNLNADQ
RNGFIQSLKDDPSQSANVLGEAQKLNDSQAPKADAQQNNFNKDQQSAFYEILNMPNLNEA
QRNGFIQSLKDDPSQSTNVLGEAKKLNESQAPKADNNFNKEQQNAFYEILNMPNLNEEQR
NGFIQSLKDDPSQSANLLSEAKKLNESQAPKADNKFNKEQQNAFYEILHLPNLNEEQRNG
FIQSLKDDPSVSKEILAEAKKLNDAQAPKEEDNKKPGKEDGNKPGKEDGNKPGKEDNKKP
GKEDGNKPGKEDNNKPGKEDGNKPGKEDNNKPGKEDGNKPGKEDGNKPGKEDGNGVHVVK
PGDTVNDIAKANGTTADKIAADNKLADKNMIKPGQELVVDKKQPANHADANKAQALPETG
EENPFIGTTVFGGLSLALGAALLAGRRREL
SEQ ID NO 35 SPA N315 
MKKKNIYSIRKLGVGIASVTLGTLLISGGVTPAANAAQHDEAQQNAFYQVLNMPNLNADQ
RNGFIQSLKDDPSQSANVLGEAQKLNDSQAPKADAQQNNFNKDQQSAFYEILNMPNLNEA
QRNGFIQSLKDDPSQSTNVLGEAKKLNESQAPKADNNFNKEQQNAFYEILNMPNLNEEQR
NGFIQSLKDDPSQSANLLSEAKKLNESQAPKADNKFNKEQQNAFYEILHLPNLNEEQRNG
FIQSLKDDPSVSKEILAEAKKLNDAQAPKEEDNKKPGKEDGNKPGKEDGNKPGKEDNKKP
GKEDGNKPGKEDNNKPGKEDGNKPGKEDNNKPGKEDGNKPGKEDGNKPGKEDGNGVHVVK
PGDTVNDIAKANGTTADKIAADNKLADKNMIKPGQELVVDKKQPANHADANKAQALPETG
EENPFIGTTVFGGLSLALGAALLAGRRREL
SEQ ID NO 36 SPA MW2 
MKKKNIYSIRKLGVGIASVTLGTLLISGGVTPAANAAQHDEAQQNAFYQVLNMPNLNADQ
RNGFIQSLKDDPSQSANVLGEAQKLNDSQAPKADAQQNNFNKDQQSAFYEILNMPNLNEA
QRNGFIQSLKDDPSQSTNVLGEAKKLNESQAPKADNNFNKEQQNAFYEILNMPNLNEEQR
NGFIQSLKDDPSQSANLLSEAKKLNESQAPKADNKFNKEQQNAFYEILHLPNLNEEQRNG
FIQSLKDDPSQSANLLAEAKKLNDAQAPKADNKFNKEQQNAFYEILHLPNLTEEQRNGFI
QSLKDDPSVSKEILAEAKKLNDAQAPKEEDNNKPGKEDGNKPGKEDGNKPGKEDNNKPGK
EDGNKPGKEDNKKPGKEDGNKPGKEDNNKPGKEDGNGVHVVKPGDTVNDIAKANGTTADK
IAADNKLADKNMIKPGQELVVDKKQPANHADANKAQALPETGEENPFIGTTVFGGLSLAL
GAALLAGRRREL
SEQ ID NO 37 SPA Col 
LKKKNIYSIRKLGVGIASVTLGTLLISGGVTPAANAAQHDEAQQNAFYQVLNMPNLNADQ
RNGFIQSLKDDPSQSANVLGEAQKLNDSQAPKADAQQNNFNKDQQSAFYEILNMPNLNEA
QRNGFIQSLKDDPSQSTNVLGEAKKLNESQAPKADNNFNKEQQNAFYEILNMPNLNEEQR
NGFIQSLKDDPSQSANLLSEAKKLNESQAPKADNKFNKEQQNAFYEILHLPNLNEEQRNG
FIQSLKDDPSQSANLLAEAKKLNDAQAPKADNKFNKEQQNAFYEILHLPNLTEEQRNGFI
QSLKDDPSVSKEILAEAKKLNDAQAPKEEDNNKPGKEDNNKPGKEDNNKPGKEDNNKPGK
EDGNKPGKEDNKKPGKEDGNKPGKEDNKKPGKEDGNKPGKEDGNKPGKEDGNGVHVVKPG
DTVNDIAKANGTTADKIAADNKLADKNMIKPGQELVVDKKQPANHADANKAQALPETGEE
NPFIGTTVFGGLSLALGAALLAGRRREL
SEQ ID NO 38 MRSA252 
LKKKNIYSIRKLGVGIASVTLGTLLISGGVTPAANAAQHDEAQQNAFYQVLNMPNLNADQ
RNGFIQSLKDDPSQSANVLGEAQKLNDSQAPKADAQQNKFNKDQQSAFYEILNMPNLNEE
QRNGFIQSLKDDPSQSTNVLGEAKKLNESQAPKADNNFNKEQQNAFYEILNMPNLNEEQR
NGFIQSLKDDPSQSANLLAEAKKLNESQAPKADNKFNKEQQNAFYEILHLPNLNEEQRNG
FIQSLKDDPSQSANLLAEAKKLNDAQAPKADNKFNKEQQNAFYEILHLPNLTEEQRNGFI
QSLKDDPSVSKEILAEAKKLNDAQAPKEEDNNKPGKEDNNKPGKEDGNKPGKEDNKKPGK
EDGNKPGKEDNKKPGKEDGNKPGKEDGNKPGKEDGNKPGKEDGNKPGKEDGNKPGKEDGN
GVHVVKPGDTVNDIAKANGTTADKIAADNKLADKNMIKPGQELVVDKKQPANHADANKAQ
ALPETGEENPFIGTTVFGGLSLALGAALLAGRRREL
SEQ ID NO 39 SPA MSSA476 
LKKKNIYSIRKLGVGIASVTLGTLLISGGVTPAANAAQHDEAQQNAFYQVLNMPNLNADQ
RNGFIQSLKDDPSQSANVLGEAQKLNDSQAPKADAQQNNFNKDQQSAFYEILNMPNLNEA
QRNGFIQSLKDDPSQSTNVLGEAKKLNESQAPKADNNFNKEQQNAFYEILNMPNLNEEQR
NGFIQSLKDDPSQSANLLSEAKKLNESQAPKADNKFNKEQQNAFYEILHLPNLNEEQRNG
FIQSLKDDPSQSANLLAEAKKLNDAQAPKADNKFNKEQQNAFYEILHLPNLTEEQRNGFI
QSLKDDPSVSKEILAEAKKLNDAQAPKEEDNNKPGKEDGNKPGKEDGNKPGKEDNNKPGK
EDGNKPGKEDNKKPGKEDGNKPGKEDNNKPGKEDGNGVHVVKPGDTVNDIAKANGTTADK
IAADNKLADKNMIKPGQELVVDKKQPANHADANKAQALPETGEENPFIGTTVFGGLSLAL
GAALLAGRRREL
SEQ ID NO 40 SPA V8 
MMTLQIHTGGINLKKKNIYSIRKLGVGIASVTLGTLLISGGVTPAANAAQHDEAQQNAFY
QVLNMPNLNADQRNGFIQSLKDDPSQSANVLGEAQKLNDSQAPKADAQQNKFNKDQQSAF
YEILNMPNLNEEQRNGFIQSLKDDPSQSTNVLGEAKKLNESQAPKADNNFNKEQQNAFYE
ILNMPNLNEEQRNGFIQSLKDDPSQSANLLAEAKKLNDAQAPKADNKFNKEQQNAFYEIL
HLPNLTEEQRNGFIQSLKDDPSVSKEILAEAKKLNDAQAPKEEDNNKPGKEDNNKPGKED
 
GNKPGKEDNKKPGKEDGNKPGKEDNKKPGKEDGNKPGKEDGNKPGKEDGNKPGKEDGNGV
HVVKPGDTVNDIAKANGTTADKIAVDNKLADKNMIKPGQELVVDKKQPANHADANKAQAL
PETGEENPFIGTTVFGGLSLALGAALLAGRRREL
SEQ ID NO 41 fusion protein 
MAQHDEAQQNAFYQVLNMPNLNADQRNGFIQSLKDDPSQSANVLGEAQKLNDSQAPKADA
QQNNFNKDQQSAFYEILNMPNLNEAQRNGFIQSLKDDPSQSTNVLGEAKKLNESQAPKAD
NNFNKEQQNAFYEILNMPNLNEEQRNGFIQSLKDDPSQSANLLSEAKKLNESQAPKADNK
FNKEQQNAFYEILHLPNLNEEQRNGFIQSLKDDPSQSANLLAEAKKLNDAQAPKADNKFN
KEQQNAFYEILHLPNLTEEQRNGFIQSLKDDPSVSKEILAEAKKLNDAQAPKEEDNNKPG
KEDNNKPGKEDNNKPGKEDNNKPGKEDNNKPGKEDGNKPGKEDNKKPGKEDGNKPGKEDN
KKPGKEDGNKPGKEDGNKPGKEDGNGVHVGGSSENTQQTSTKHQTTQNNYVTDQQKAFYQ
VLHLKGITEEQRNQYIKTLREHPERAQEVFSESLKDSKNPDRRVAQQNAFYNVLKNDNLT
EQEKNNYIAQIKENPDRSQQVWVESVQSSKAKERQNIENADKAIKDFQDNKAPHDKSAAY
EANSKLPKDLRDKNNRFVEKVSIEKA
SEQ ID NO 42 fusion protein full length 
MAQHDEAQQNAFYQVLNMPNLNADQRNGFIQSLKDDPSQSANVLGEAQKLNDSQAPKADA
QQNNFNKDQQSAFYEILNMPNLNEAQRNGFIQSLKDDPSQSTNVLGEAKKLNESQAPKAD
NNFNKEQQNAFYEILNMPNLNEEQRNGFIQSLKDDPSQSANLLSEAKKLNESQAPKADNK
FNKEQQNAFYEILHLPNLNEEQRNGFIQSLKDDPSQSANLLAEAKKLNDAQAPKADNKFN
KEQQNAFYEILHLPNLTEEQRNGFIQSLKDDPSVSKEILAEAKKLNDAQAPKEEDNNKPG
KEDNNKPGKEDNNKPGKEDNNKPGKEDNNKPGKEDGNKPGKEDNKKPGKEDGNKPGKEDN
KKPGKEDGNKPGKEDGNKPGKEDGNGVHVGGSSENTQQTSTKHQTTQNNYVTDQQKAFYQ
VLHLKGITEEQRNQYIKTLREHPERAQEVFSESLKDSKNPDRRVAQQNAFYNVLKNDNLT
EQEKNNYIAQIKENPDRSQQVWVESVQSSKAKERQNIENADKAIKDFQDNKAPHDKSAAY
EANSKLPKDLRDKNNRFVEKVSIEKATSGHHHHHH
SEQ ID NO 43 Sbi domain 1 
ACTCAAAACAACTACGTAACAGATCAACAAAAAGCTTTTATCAAGTATTACATCTAAAAG
GTATCACAGAAGAACAACGTAACCAATACATCAAAACATTACGCGAACACCCAGAACGTGC
ACAAGAAGTATTCTCTGAATCACTTAAAGACAGC
SEQ ID NO 44 Sbi domain 2 
AAGAACCCAGACCGACGTGTTGCACAACAAAACGCTTTTTACAATGTTCTTAAAAATGATA
ACTTAACTGAACAAGAAAAAAATAATTACATTGCACAAATTAAAGAAAACCCTGATAGAAG
CCAACAAGTTTGGGTAGAATCAGTACAATCTTCTAAAGCT
SEQ ID NO 45 Sbi domains 1 and 2 
ACTCAAAACAACTACGTAACAGATCAACAAAAAGCTTTTATCAAGTATTACATCTAAAAG
GTATCACAGAAGAACAACGTAACCAATACATCAAAACATTACGCGAACACCCAGAACGTGC
ACAAGAAGTATTCTCTGAATCACTTAAAGACAGCAAGAACCCAGACCGACGTGTTGCACAA
CAAAACGCTTTTTACAATGTTCTTAAAAATGATAACTTAACTGAACAAGAAAAAAATAATT
ACATTGCACAAATTAAAGAAAACCCTGATAGAAGCCAACAAGTTTGGGTAGAATCAGTACA
 ATCTTCTAAAGCT
SEQ ID NO 46 sbi part of fusion protein 
AGTGAAAACACGCAACAAACTTCAACTAAGCACCAAACAACTCAAAACAACTACGTAACAG
ATCAACAAAAAGCTTTTTATCAAGTATTACATCTAAAAGGTATCACAGAAGAACAACGTAA
CCAATACATCAAAACATTACGCGAACACCCAGAACGTGCACAAGAAGTATTCTCTGAATCA
CTTAAAGACAGCAAGAACCCAGACCGACGTGTTGCACAACAAAACGCTTTTTACAATGTTC
TTAAAAATGATAACTTAACTGAACAAGAAAAAAATAATTACATTGCACAAATTAAAGAAAA
CCCTGATAGAAGCCAACAAGTTTGGGTAGAATCAGTACAATCTTCTAAAGCTAAAGAACGT
CAAAATATTGAAAATGCGGATAAAGCAATTAAAGATTTCCAAGATAACAAAGCACCACACG
ATAAATCAGCAGCATATGAAGCTAACTCAAAATTACCTAAAGATTTACGTGATAAAAACAA
CCGCTTTGTAGAAAAAGTTTCAATTGAAAAAGCA
SEQ ID NO 47 Sbi Col 
ATGAAAAATAAATATATCTCGAAGTTGCTAGTTGGGGCAGCAACAATTACGTTAGCTACA
ATGATTTCAAATGGGGAAGCAAAAGCGAGTGAAAACACGCAACAAACTTCAACTAAGCAC
CAAACAACTCAAAACAACTACGTAACAGATCAACAAAAAGCTTTTTATCAAGTATTACAT
CTAAAAGGTATCACAGAAGAACAACGTAACCAATACATCAAAACATTACGCGAACACCCA
GAACGTGCACAAGAAGTATTCTCTGAATCACTTAAAGACAGCAAGAACCCAGACCGACGT
GTTGCACAACAAAACGCTTTTTACAATGTTCTTAAAAATGATAACTTAACTGAACAAGAA
AAAAATAATTACATTGCACAAATTAAAGAAAACCCTGATAGAAGCCAACAAGTTTGGGTA
GAATCAGTACAATCTTCTAAAGCTAAAGAACGTCAAAATATTGAAAATGCGGATAAAGCA
ATTAAAGATTTCCAAGATAACAAAGCACCACACGATAAATCAGCAGCATATGAAGCTAAC
TCAAAATTACCTAAAGATTTACGTGATAAAAACAACCGCTTTGTAGAAAAAGTTTCAATT
GAAAAAGCAATCGTTCGTCATGATGAGCGTGTGAAATCAGCAAATGATGCAATCTCAAAA
TTAAATGAAAAAGATTCAATTGAAAACAGACGTTTAGCACAACGTGAAGTTAACAAAGCA
CCTATGGATGTAAAAGAGCATTTACAGAAACAATTAGACGCATTAGTTGCTCAAAAAGAT
GCTGAAAAGAAAGTGGCGCCAAAAGTTGAGGCTCCTCAAATTCAATCACCACAAATTGAA
AAACCTAAAGTAGAATCACCAAAAGTTGAAGTCCCTCAAATTCAATCACCAAAAGTTGAG
GTTCCTCAATCTAAATTATTAGGTTACTACCAATCATTAAAAGATTCATTTAACTATGGT
TACAAGTATTTAACAGATACTTATAAAAGCTATAAAGAAAAATATGATACAGCAAAGTAC
TACTATAATACGTACTATAAATACAAAGGTGCGATTGATCAAACAGTATTAACAGTACTA
GGTAGTGGTTCTAAATCTTACATCCAACCATTGAAAGTTGATGATAAAAACGGCTACTTA
GCTAAATCATATGCACAAGTAAGAAACTATGTAACTGAGTCAATCAATACTGGTAAAGTA
TTATATACTTTCTACCAAAACCCAACATTAGTAAAAACAGCTATTAAAGCTCAAGAAACT
GCATCATCAATCAAAAATACATTAAGTAATTTATTATCATTCTGGAAATAA
SEQ ID NO 48 Sbi Mu50 
ATACACATGAAAAATAAATATATCTCGAAGTTGCTAGTTGGGGCAGCAACAATTACTTTA
GCTACAATGATTTCAAATGGGGAAGCAAAAGCGAGTGAAAACACGCAACAAACTTCAACT
AAGCACCAAACAACTCAAAACAACTACGTAACAGATCAACAAAAAGCTTTTTATCAAGTA
TTACATCTAAAAGGTATCACAGAAGAACAACGTAACCAATACATCAAAACATTACGCGAA
CACCCAGAACGTGCACAAGAAGTATTCTCTGAATCACTTAAAGACAGCAAGAACCCAGAC
CGACGTGTTGCACAACAAAACGCTTTTTACAATGTTCTTAAAAATGATAACTTAACTGAA
CAAGAAAAAAATAATTACATTGCACAAATTAAAGAAAACCCTGATAGAAGCCAACAAGTT
TGGGTAGAATCAGTACAATCTTCTAAAGCTAAAGAACGTCAAAATATTGAAAATGCGGAT
AAAGCAATTAAAGATTTCCAAGATAACAAAGCACCACACGATAAATCAGCAGCATATGAA
GCTAACTCAAAATTACCTAAAGATTTACGCGATAAAAATAACCGCTTTGTAGAAAAAGTT
TCAATTGAAAAAGCAATCGTTCGTCATGATGAGCGTGTGAAATCAGCAAATGATGCAATC
TCAAAATTAAATGAAAAAGATTCAATTGAAAACAGACGTTTAGCACAACGTGAAGTTAAC
AAAGCACCTATGGATGTAAAAGAGCATTTACAGAAACAATTAGACGCATTAGTAGCTCAA
AAAGATGCTGAAAAGAAAGTGGCGCCAAAAGTTGAGGCTCCTCAAATTCAATCACCACAA
 
ATTGAAAAACCTAAAGCAGAATCACCAAAAGTTGAAGTCCCTCAATCTAAATTATTAGGT
TACTACCAATCATTAAAAGATTCATTTAACTATGGTTACAAGTATTTAACAGATACTTAT
AAAAGCTATAAAGAAAAATATGATACAGCAAAGTACTACTATAATACGTACTATAAATAC
AAAGGTGCGATTGATCAAACAGTATTAACAGTACTAGGTAGTGGTTCTAAATCTTACATC
CAACCATTGAAAGTTGATGATAAAAACGGCTACTTAGCTAAATCATATGCACAAGTAAGA
AACTATGTAACTGAGTCAATCAATACTGGTAAAGTATTATATACTTTCTACCAAAACCCA
ACATTAGTAAAAACAGCTATTAAAGCTCAAGAAACTGCATCATCAATCAAAAATACATTA
AGTAATTTATTATCATTCTGGAAATAA
SEQ ID NO 49 Sbi MRSA252 
ATGAAAAATAAATATATCTCGAAGTTGCTAGTTGGGGCAGCAACAATTACTTTAGCTACA
ATGATTTCAAATGGGGAAGCAAAAGCGAGTGAAAACACGCAACAAACTTCAACTAAGCAC
CAAACAACTCAAAACAACTACGTAACAGATCAACAAAAAGCTTTTTATCAAGTATTACAT
CTAAAAGGTATCACAGAAGAACAACGTAACCAATACATCAAAACATTACGCGAACACCCA
GAACGTGCACAAGAAGTATTCTCTGAATCACTTAAAGACAGCAAGAACCCAGACCGACGT
GTTGCACAACAAAACGCTTTTTACAATGTTCTTAAAAATGATAACTTAACTGAACAAGAA
AAAAATAATTACATTGCACAAATTAAAGAAAACCCTGATAGAAGCCAACAAGTTTGGGTA
GAATCAGTACAATCTTCTAAAGCTAAAGAACGTCAAAATATTGAAAATGCGGATAAAGCA
ATTAAAGATTTCCAAGATAACAAAGCACCACACGATAAATCAGCAGCATATGAAGCTAAC
TCAAAATTACCTAAAGATTTACGTGATAAAAATAACCGCTTTGTAGAAAAAGTTTCAATT
GAAAAAGCAATCGTTCGTCATGATGAGCGTGTGAAATCAGCAAATGATGCAATCTCAAAA
TTAAATGAAAAAGATTCAATTGAAAACAGACGTTTAGCACAACGTGAAGTTAATAAAGCA
CCTATGGATGTACAAAAGCATTTACAGAAACAATTAGACGCATTAGTAGCTCAAAAAGAT
GCTGAAAAGAAAGTGGCGCCAAAAGTTGAGGCTCCTCAAATTCAATCACCACAAATTGAA
AAACCTAAAGCAGAATCACCAAAAGTTGAAGTCCCTCAAATTCAATCACCAAAAGTTGAG
GTTCCTCAATCTAAATTATTAGGTTACTACCAATCATTAAAAGATTCATTTAACTATGGT
TACAAGTATTTAACAGATACTTATAAAAGCTATAAAGAAAAATATGATACAGCAAAGTAC
TACTATAATAAATATTACCAATATAAAGGTTTGATTGATAAAACAGTTTTAACAACTATC
GGTAGTGGCTATGGTTCATACATTAAACCTCTTGAAGTAAGCAAAGAAAGCGGGAACTTA
GCTAAATCATATGCACAAGTAAGAAACTATGTAACTGAGTCAATCAACACTGGTAAAGTG
TTATACGCATTCTACCAAAAACCAGAATTAGTAAAAACAGCTATTAAAGCTCAAGAAACA
GCAACAACTTTCAAAAACGCTTTAACAGGCATATTCAAATCATTCTGGAAATAA
SEQ ID NO 50 Sbi MSSA476 
ATGAAAAATAAATATATCTCGAAGTTGCTAGTTGGGGCAGCAACAATTACTTTAGCTACA
ATGATTTCAAATGGGGAAGCAAAAGCGAGTGAAAACACGCAACAAACTTCAACTAAGCAC
CAAACAACTCAAAACAACTACGTAACAGATCAACAAAAAGCTTTTTATCAAGTATTACAT
CTAAAAGGTATCACAGAAGAACAACGTAACCAATACATCAAAACATTACGCGAACACCCA
GAACGTGCACAAGAAGTATTCTCTGAATCACTTAAAGACAGCAAGAACCCAGACCGACGT
GTTGCACAACAAAACGCTTTTTACAATGTTCTTAAAAATGATAACTTAACTGAACAAGAA
AAAAATAATTACATTGCACAAATTAAAGAAAACCCTGATAGAAGCCAACAAGTTTGGGTA
GAATCAGTACAATCTTCTAAAGCTAAAGAACGTCAAAATATTGAAAATGCGGATAAAGCA
ATTAAAGATTTCCAAGATAACAAAGCACCACACGATAAATCAGCAGCATATGAAGCTAAC
TCAAAATTACCTAAAGATTTACGTGATAAAAATAACCGCTTTGTAGAAAAAGTTTCAATT
GAAAAAGCAATCGTTCGTCATGATGAGCGTGTGAAATCAGCAAATGATGCAATCTCAAAA
TTAAATGTAAAAGATTCAATTGAAAACAGACGTTTAGCACAACGTGAAGTTAACAAAGCA
CCTATGGATGTAAAAGAGCATTTACAGAAACAATTAGACGCATTAGTAGCTCAAAAAGAT
GCTGAAAAGAAAGTGGCGCCAAAAGTTGAGGCTCCTCAAATTCAATCACCACAAATTGAA
AAACCTAAAGCAGAATCACCAAAAGTTGAAGTCCCTCAAATCCAATCACCAAAAGTTGAG
GTTCCTCAATCTAAATTATTAGGTTACTACCAATCATTAAAAGATTCATTTAACTATGGT
TACAAGTATTTAACAGATACTTATAAAAGCTATAAAGAAAAATATGATACAGCAAAGTAC
TACTATAATACGTACTATAAATACAAAGGTGCGATTGACAAAGCTGTATTAACTTTACTT
GGCGATGGTTCTAAATCTTATATCCAACCATTGAAAGTTGATGATAAAAATGGCTATTTA
GCTAAATCATATGCACAAGTAAGAAACTATGTAACTGAGTCAATCAATACTGGTAAAGTA
TTATATACTTTCTACCAAAACCCAACATTAGTAAAAACAGCTATTAAAGCTCAAGAAACT
GCATCATCAATCAAAAATACAATAACTGGATTATTTAACTCATTCTGGAAATAA
SEQ ID NO 51 Sbi MW2 
ATGAAAAATAAATATATCTCGAAGTTGCTAGTTGGGGCAGCAACAATTACTTTAGCTACA
ATGATTTCAAATGGGGAAGCAAAAGCGAGTGAAAACACGCAACAAACTTCAACTAAGCAC
CAAACAACTCAAAACAACTACGTAACAGATCAACAAAAAGCTTTTTATCAAGTATTACAT
CTAAAAGGTATCACAGAAGAACAACGTAACCAATACATCAAAACATTACGCGAACACCCA
GAACGTGCACAAGAAGTATTCTCTGAATCACTTAAAGACAGCAAGAACCCAGACCGACGT
GTTGCACAACAAAACGCTTTTTACAATGTTCTTAAAAATGATAACTTAACTGAACAAGAA
AAAAATAATTACATTGCACAAATTAAAGAAAACCCTGATAGAAGCCAACAAGTTTGGGTA
GAATCAGTACAATCTTCTAAAGCTAAAGAACGTCAAAATATTGAAAATGCGGATAAAGCA
ATTAAAGATTTCCAAGATAACAAAGCACCACACGATAAATCAGCAGCATATGAAGCTAAC
TCAAAATTACCTAAAGATTTACGTGATAAAAATAACCGCTTTGTAGAAAAAGTTTCAATT
GAAAAAGCAATCGTTCGTCATGATGAGCGTGTGAAATCAGCAAATGATGCAATCTCAAAA
TTAAATGTAAAAGATTCAATTGAAAACAGACGTTTAGCACAACGTGAAGTTAACAAAGCA
CCTATGGATGTAAAAGAGCATTTACAGAAACAATTAGACGCATTAGTAGCTCAAAAAGAT
GCTGAAAAGAAAGTGGCGCCAAAAGTTGAGGCTCCTCAAATTCAATCACCACAAATTGAA
AAACCTAAAGCAGAATCACCAAAAGTTGAAGTCCCTCAAATCCAATCACCAAAAGTTGAG
GTTCCTCAATCTAAATTATTAGGTTACTACCAATCATTAAAAGATTCATTTAACTATGGT
TACAAGTATTTAACAGATACTTATAAAAGCTATAAAGAAAAATATGATACAGCAAAGTAC
TACTATAATACGTACTATAAATACAAAGGTGCGATTGACAAAGCTGTATTAACTTTACTT
GGCGATGGTTCTAAATCTTATATCCAACCATTGAAAGTTGATGATAAAAATGGCTATTTA
GCTAAATCATATGCACAAGTAAGAAACTATGTAACTGAGTCAATCAATACTGGTAAAGTA
TTATATACTTTCTACCAAAACCCAACATTAGTAAAAACAGCTATTAAAGCTCAAGAAACT
GCATCATCAATCAAAAATACAATAACTGGATTATTTAACTCATTCTGGAAATAA
SEQ ID NO 52 Sbi N315 
ATGAAAAATAAATATATCTCGAAGTTGCTAGTTGGGGCAGCAACAATTACTTTAGCTACA
ATGATTTCAAATGGGGAAGCAAAAGCGAGTGAAAACACGCAACAAACTTCAACTAAGCAC
CAAACAACTCAAAACAACTACGTAACAGATCAACAAAAAGCTTTTTATCAAGTATTACAT
CTAAAAGGTATCACAGAAGAACAACGTAACCAATACATCAAAACATTACGCGAACACCCA
GAACGTGCACAAGAAGTATTCTCTGAATCACTTAAAGACAGCAAGAACCCAGACCGACGT
GTTGCACAACAAAACGCTTTTTACAATGTTCTTAAAAATGATAACTTAACTGAACAAGAA
AAAAATAATTACATTGCACAAATTAAAGAAAACCCTGATAGAAGCCAACAAGTTTGGGTA
GAATCAGTACAATCTTCTAAAGCTAAAGAACGTCAAAATATTGAAAATGCGGATAAAGCA
ATTAAAGATTTCCAAGATAACAAAGCACCACACGATAAATCAGCAGCATATGAAGCTAAC
TCAAAATTACCTAAAGATTTACGCGATAAAAATAACCGCTTTGTAGAAAAAGTTTCAATT
GAAAAAGCAATCGTTCGTCATGATGAGCGTGTGAAATCAGCAAATGATGCAATCTCAAAA
TTAAATGAAAAAGATTCAATTGAAAACAGACGTTTAGCACAACGTGAAGTTAACAAAGCA
CCTATGGATGTAAAAGAGCATTTACAGAAACAATTAGACGCATTAGTAGCTCAAAAAGAT
GCTGAAAAGAAAGTGGCGCCAAAAGTTGAGGCTCCTCAAATTCAATCACCACAAATTGAA
AAACCTAAAGCAGAATCACCAAAAGTTGAAGTCCCTCAAATCCAATCACCAAAAGTTGAG
GTTCCTCAATCTAAATTATTAGGTTACTACCAATCATTAAAAGATTCATTTAACTATGGT
TACAAGTATTTAACAGATACTTATAAAAGCTATAAAGAAAAATATGATACAGCAAAGTAC
TACTATAATACGTACTATAAATACAAAGGTGCGATTGATCAAACAGTATTAACAGTACTA
GGTAGTGGTTCTAAATCTTACATCCAACCATTGAAAGTTGATGATAAAAACGGCTACTTA
GCTAAATCATATGCACAAGTAAGAAACTATGTAACTGAGTCAATCAATACTGGTAAAGTA
TTATATACTTTCTACCAAAACCCAACATTAGTAAAAACAGCTATTAAAGCTCAAGAAACT
GCATCATCAATCAAAAATACATTAAGTAATTTATTATCATTCTGGAAATAA
SEQ ID NO 53 Sbi NCTC8325 
ATGAAAAATAAATATATCTCGAAGTTGCTAGTTGGGGCAGCAACAATTACGTTAGCTACA
ATGATTTCAAATGGGGAAGCAAAAGCGAGTGAAAACACGCAACAAACTTCAACTAAGCAC
CAAACAACTCAAAACAACTACGTAACAGATCAACAAAAAGCTTTTTATCAAGTATTACAT
CTAAAAGGTATCACAGAAGAACAACGTAACCAATACATCAAAACATTACGCGAACACCCA
GAACGTGCACAAGAAGTATTCTCTGAATCACTTAAAGACAGCAAGAACCCAGACCGACGT
GTTGCACAACAAAACGCTTTTTACAATGTTCTTAAAAATGATAACTTAACTGAACAAGAA
AAAAATAATTACATTGCACAAATTAAAGAAAACCCTGATAGAAGCCAACAAGTTTGGGTA
 
GAATCAGTACAATCTTCTAAAGCTAAAGAACGTCAAAATATTGAAAATGCGGATAAAGCA
ATTAAAGATTTCCAAGATAACAAAGCACCACACGATAAATCAGCAGCATATGAAGCTAAC
TCAAAATTACCTAAAGATTTACGTGATAAAAACAACCGCTTTGTAGAAAAAGTTTCAATT
GAAAAAGCAATCGTTCGTCATGATGAGCGTGTGAAATCAGCAAATGATGCAATCTCAAAA
TTAAATGAAAAAGATTCAATTGAAAACAGACGTTTAGCACAACGTGAAGTTAACAAAGCA
CCTATGGATGTAAAAGAGCATTTACAGAAACAATTAGACGCATTAGTTGCTCAAAAAGAT
GCTGAAAAGAAAGTGGCGCCAAAAGTTGAGGCTCCTCAAATTCAATCACCACAAATTGAA
AAACCTAAAGTAGAATCACCAAAAGTTGAAGTCCCTCAAATTCAATCACCAAAAGTTGAG
GTTCCTCAATCTAAATTATTAGGTTACTACCAATCATTAAAAGATTCATTTAACTATGGT
TACAAGTATTTAACAGATACTTATAAAAGCTATAAAGAAAAATATGATACAGCAAAGTAC
TACTATAATACGTACTATAAATACAAAGGTGCGATTGATCAAACAGTATTAACAGTACTA
GGTAGTGGTTCTAAATCTTACATCCAACCATTGAAAGTTGATGATAAAAACGGCTACTTA
GCTAAATCATATGCACAAGTAAGAAACTATGTAACTGAGTCAATCAATACTGGTAAAGTA
TTATATACTTTCTACCAAAACCCAACATTAGTAAAAACAGCTATTAAAGCTCAAGAAACT
GCATCATCAATCAAAAATACATTAAGTAATTTATTATCATTCTGGAAATAA
SEQ ID NO 54 SPA domain 1 
AATGCTGCGCAACACGATGAAGCTCAACAAAATGCTTTTTATCAAGTCTTAAATATGCCT
AACTTAAATGCTGATCAACGCAATGGTTTTATCCAAAGCCTTAAAGATGATCCAAGCCAA
AGTGCTAACGTTTTAGGTGAAGCTCAAAAACTTAATGACTCT
SEQ ID NO 55 SPA domain 2 (V8) 
CAACAAAGATCAACAAAGCGCCTTCTATGAAATCTTGAACATGCCTAACTTAAACGAAGA
GCAACGCAATGGTTTCATTCAAAGTCTTAAAGACGATCCAAGCCAAAGCACTAACGTTTT
AGGTGAAGCTAAAAAATTAAACGAATCT
SEQ ID NO 56 domain 2 (Mu50) 
CAACAAAATAACTTCAACAAAGATCAACAAAGCGCCTTCTATGAAATCTTGAACATGCCT
AACTTAAACGAAGCGCAACGTAACGGCTTCATTCAAAGTCTTAAAGACGACCCAAGCCAA
AGCACTAATGTTTTAGGTGAAGCTAAAAAATTAAACGAATCT
SEQ ID NO 57 domain 3 (MRSA) 
GCTGACAACAATTTCAACAAAGAACAACAAAATGCTTTCTATGAAATCTTGAACATGCCT
AACTTGAACGAAGAACAACGCAATGGTTTCATCCAAAGCTTAAAAGATGACCCAAGTCAA
AGTGCTAACCTTTTAGCAGAAGCTAAAAAGTTAAATGAATCT
SEQ ID NO 58 domain 3 (V8) 
GCTGACAACAATTTCAACAAAGAACAACAAAATGCTTTCTATGAAATCTTGAACATGCCT
AACTTGAACGAAGAACAACGCAATGGTTTCATCCAAAGCTTAAAAGATGACCCAAGTCAA
AGTGCTAACCTTTTAGCAGAAGCTAAAAAGCTAAATGATGCA
SEQ ID NO 59 domain 3 (MU50) 
GCTGATAACAATTTCAACAAAGAACAACAAAATGCTTTCTATGAAATCTTGAATATGCCT
AACTTAAACGAAGAACAACGCAATGGTTTCATCCAAAGCTTAAAAGATGACCCAAGCCAA
AGTGCTAACCTATTGTCAGAAGCTAAAAAGTTAAATGAATCT
SEQ ID NO 60 domain 4 (MRSA) 
GCTGATAACAAATTCAACAAAGAACAACAAAATGCTTTCTATGAAATCTTACATTTACCT
AACTTAAATGAAGAACAACGCAATGGTTTCATCCAAAGCTTAAAAGATGACCCAAGCCAA
AGCGCTAACCTTTTAGCAGAAGCTAAAAAGCTAAATGATGCA
SEQ ID NO 61 domain 4 (V8) 
GCTGACAACAAATTCAACAAAGAACAACAAAATGCTTTCTATGAAATTTTACATTTACCT
AACTTAACTGAAGAACAACGTAACGGCTTCATCCAAAGCCTTAAAGACGATCCTTCAGTG
AGCAAAGAAATTTTAGCAGAAGCTAAAAAGCTAAACGATGCT
SEQ ID NO 62 domain 4 (MU50) 
GCGGATAACAAATTCAACAAAGAACAACAAAATGCTTTCTATGAAATCTTACATTTACCT
AACTTAAACGAAGAACAACGTAACGGCTTCATCCAAAGCCTTAAAGACGATCCTTCAGTG
AGCAAAGAAATTTTAGCAGAAGCTAAAAAGCTAAACGATGCT
SEQ ID NO 63 domain 5 
GCTGACAACAAATTCAACAAAGAACAACAAAATGCTTTCTATGAAATTTTACATTTACCT
AACTTAACTGAAGAACAACGTAACGGCTTCATCCAAAGCCTTAAAGACGATCCTTCAGTG
AGCAAAGAAATTTTAGCAGAAGCTAAAAAGCTAAACGATGCT
SEQ ID NO 64 protein A part of fusion protein 
ATGGCGCAACACGATGAAGCTCAACAAAATGCTTTTTATCAAGTCTTAAATATGCCTAAC
TTAAATGCTGATCAACGCAATGGTTTTATCCAAAGCCTTAAAGATGATCCAAGCCAAAGT
GCTAACGTTTTAGGTGAAGCTCAAAAACTTAATGACTCTCAAGCTCCAAAAGCTGATGCG
CAACAAAATAACTTCAACAAAGATCAACAAAGCGCCTTCTATGAAATCTTGAACATGCCT
AACTTAAACGAAGCGCAACGTAACGGCTTCATTCAAAGTCTTAAAGACGACCCAAGCCAA
AGCACTAACGTTTTAGGTGAAGCTAAAAAATTAAACGAATCTCAAGCACCGAAAGCTGAT
AACAATTTCAACAAAGAACAACAAAATGCTTTCTATGAAATCTTGAATATGCCTAACTTA
AACGAAGAACAACGCAATGGTTTCATCCAAAGCTTAAAAGATGACCCAAGCCAAAGTGCT
AACCTATTGTCAGAAGCTAAAAAGTTAAATGAATCTCAAGCACCGAAAGCGGATAACAAA
TTCAACAAAGAACAACAAAATGCTTTCTATGAAATCTTACATTTACCTAACTTAAACGAA
GAACAACGCAATGGTTTCATCCAAAGCCTAAAAGATGACCCAAGCCAAAGCGCTAACCTT
TTAGCAGAAGCTAAAAAGCTAAATGATGCTCAAGCACCAAAAGCTGACAACAAATTCAAC
AAAGAACAACAAAATGCTTTCTATGAAATTTTACATTTACCTAACTTAACTGAAGAACAA
CGTAACGGCTTCATCCAAAGCCTTAAAGACGATCCTTCAGTGAGCAAAGAAATTTTAGCA
GAAGCTAAAAAGCTAAACGATGCTCAAGCACCAAAAGAGGAAGACAATAACAAGCCTGGC
AAAGAAGACAATAACAAGCCTGGCAAAGAAGACAACAACAAGCCTGGTAAAGAAGACAAC
AACAAGCCTGGTAAAGAAGACAACAACAAGCCTGGCAAAGAAGACGGCAACAAGCCTGGT
AAAGAAGACAACAAAAAACCTGGTAAAGAAGATGGCAACAAGCCTGGTAAAGAAGACAAC
AAAAAACCTGGTAAAGAAGACGGCAACAAGCCTGGCAAAGAAGATGGCAACAAACCTGGT
AAAGAAGATGGTAACGGAGTACATGTCGGC
SEQ ID NO 65 SPA NCTC8325 
TTGAAAAAGAAAAACATTTATTCAATTCGTAAACTAGGTGTAGGTATTGCATCTGTAACT
TTAGGTACATTACTTATATCTGGTGGCGTAACACCTGCTGCAAATGCTGCGCAACACGAT
GAAGCTCAACAAAATGCTTTTTATCAAGTCTTAAATATGCCTAACTTAAATGCTGATCAA
CGCAATGGTTTTATCCAAAGCCTTAAAGATGATCCAAGCCAAAGTGCTAACGTTTTAGGT
 
GAAGCTCAAAAACTTAATGACTCTCAAGCTCCAAAAGCTGATGCGCAACAAAATAACTTC
AACAAAGATCAACAAAGCGCCTTCTATGAAATCTTGAACATGCCTAACTTAAACGAAGCG
CAACGTAACGGCTTCATTCAAAGTCTTAAAGACGACCCAAGCCAAAGCACTAACGTTTTA
GGTGAAGCTAAAAAATTAAACGAATCTCAAGCACCGAAAGCTGATAACAATTTCAACAAA
GAACAACAAAATGCTTTCTATGAAATCTTGAATATGCCTAACTTAAACGAAGAACAACGC
AATGGTTTCATCCAAAGCTTAAAAGATGACCCAAGCCAAAGTGCTAACCTATTGTCAGAA
GCTAAAAAGTTAAATGAATCTCAAGCACCGAAAGCGGATAACAAATTCAACAAAGAACAA
CAAAATGCTTTCTATGAAATCTTACATTTACCTAACTTAAACGAAGAACAACGCAATGGT
TTCATCCAAAGCCTAAAAGATGACCCAAGCCAAAGCGCTAACCTTTTAGCAGAAGCTAAA
AAGCTAAATGATGCTCAAGCACCAAAAGCTGACAACAAATTCAACAAAGAACAACAAAAT
GCTTTCTATGAAATTTTACATTTACCTAACTTAACTGAAGAACAACGTAACGGCTTCATC
CAAAGCCTTAAAGACGATCCTTCAGTGAGCAAAGAAATTTTAGCAGAAGCTAAAAAGCTA
AACGATGCTCAAGCACCAAAAGAGGAAGACAATAACAAGCCTGGCAAAGAAGACAATAAC
AAGCCTGGCAAAGAAGACAACAACAAGCCTGGTAAAGAAGACAACAACAAGCCTGGTAAA
GAAGACAACAACAAGCCTGGCAAAGAAGACGGCAACAAGCCTGGTAAAGAAGACAACAAA
AAACCTGGTAAAGAAGATGGCAACAAGCCTGGTAAAGAAGACAACAAAAAACCTGGTAAA
GAAGACGGCAACAAGCCTGGCAAAGAAGATGGCAACAAACCTGGTAAAGAAGATGGTAAC
GGAGTACATGTCGTTAAACCTGGTGATACAGTAAATGACATTGCAAAAGCAAACGGCACT
ACTGCTGACAAAATTGCTGCAGATAACAAATTAGCTGATAAAAACATGATCAAACCTGGT
CAAGAACTTGTTGTTGATAAGAAGCAACCAGCAAACCATGCAGATGCTAACAAAGCTCAA
GCATTACCAGAAACTGGTGAAGAAAATCCATTCATCGGTACAACTGTATTTGGTGGATTA
TCATTAGCCTTAGGTGCAGCGTTATTAGCTGGACGTCGTCGCGAACTATAA
SEQ ID NO 66 SPA Mu50 
TTGAAAAAGAAAAACATTTATTCAATTCGTAAACTAGGTGTAGGTATTGCATCTGTAACT
TTAGGTACATTACTTATATCTGGTGGCGTAACACCTGCTGCAAATGCTGCGCAACACGAT
GAAGCTCAACAAAATGCTTTTTATCAAGTGTTAAATATGCCTAACTTAAACGCTGATCAA
CGTAATGGTTTTATCCAAAGCCTTAAAGATGATCCAAGCCAAAGTGCTAACGTTTTAGGT
GAAGCTCAAAAACTTAATGACTCTCAAGCTCCAAAAGCTGATGCGCAACAAAATAACTTC
AACAAAGATCAACAAAGCGCCTTCTATGAAATCTTGAACATGCCTAACTTAAACGAAGCG
CAACGTAACGGCTTCATTCAAAGTCTTAAAGACGACCCAAGCCAAAGCACTAATGTTTTA
GGTGAAGCTAAAAAATTAAACGAATCTCAAGCACCGAAAGCTGATAACAATTTCAACAAA
GAACAACAAAATGCTTTCTATGAAATCTTGAATATGCCTAACTTAAACGAAGAACAACGC
AATGGTTTCATCCAAAGCTTAAAAGATGACCCAAGCCAAAGTGCTAACCTATTGTCAGAA
GCTAAAAAGTTAAATGAATCTCAAGCACCGAAAGCGGATAACAAATTCAACAAAGAACAA
CAAAATGCTTTCTATGAAATCTTACATTTACCTAACTTAAACGAAGAACAACGTAACGGC
TTCATCCAAAGCCTTAAAGACGATCCTTCAGTGAGCAAAGAAATTTTAGCAGAAGCTAAA
AAGCTAAACGATGCTCAAGCACCAAAAGAGGAAGACAACAAAAAACCTGGTAAAGAAGAC
GGCAACAAACCTGGCAAAGAAGACGGCAACAAGCCTGGTAAAGAAGACAACAAAAAACCT
GGTAAAGAAGACGGCAACAAGCCTGGTAAAGAAGACAACAACAAACCTGGCAAAGAAGAC
GGCAACAAGCCTGGTAAAGAAGACAACAACAAGCCTGGTAAAGAAGACGGCAACAAGCCT
GGTAAAGAAGACGGCAACAAACCTGGTAAAGAAGACGGCAACGGAGTACATGTCGTTAAA
CCTGGTGATACAGTAAATGACATTGCAAAAGCAAACGGCACTACTGCTGACAAAATTGCT
GCAGATAACAAATTAGCTGATAAAAACATGATCAAACCTGGTCAAGAACTTGTTGTTGAT
AAGAAGCAACCAGCAAACCATGCAGATGCTAACAAAGCTCAAGCATTACCAGAAACTGGT
GAAGAAAATCCATTCATCGGTACAACTGTATTTGGTGGATTATCATTAGCCTTAGGTGCA
GCGTTATTAGCTGGACGTCGTCGCGAACTATAA
SEQ ID NO 67 SPA N315 
TTGAAAAAGAAAAACATTTATTCAATTCGTAAACTAGGTGTAGGTATTGCATCTGTAACT
TTAGGTACATTACTTATATCTGGTGGCGTAACACCTGCTGCAAATGCTGCGCAACACGAT
GAAGCTCAACAAAATGCTTTTTATCAAGTGTTAAATATGCCTAACTTAAACGCTGATCAA
CGTAATGGTTTTATCCAAAGCCTTAAAGATGATCCAAGCCAAAGTGCTAACGTTTTAGGT
GAAGCTCAAAAACTTAATGACTCTCAAGCTCCAAAAGCTGATGCGCAACAAAATAACTTC
AACAAAGATCAACAAAGCGCCTTCTATGAAATCTTGAACATGCCTAACTTAAACGAAGCG
 
CAACGTAACGGCTTCATTCAAAGTCTTAAAGACGACCCAAGCCAAAGCACTAATGTTTTA
GGTGAAGCTAAAAAATTAAACGAATCTCAAGCACCGAAAGCTGATAACAATTTCAACAAA
GAACAACAAAATGCTTTCTATGAAATCTTGAATATGCCTAACTTAAACGAAGAACAACGC
AATGGTTTCATCCAAAGCTTAAAAGATGACCCAAGCCAAAGTGCTAACCTATTGTCAGAA
GCTAAAAAGTTAAATGAATCTCAAGCACCGAAAGCGGATAACAAATTCAACAAAGAACAA
CAAAATGCTTTCTATGAAATCTTACATTTACCTAACTTAAACGAAGAACAACGTAACGGC
TTCATCCAAAGCCTTAAAGACGATCCTTCAGTGAGCAAAGAAATTTTAGCAGAAGCTAAA
AAGCTAAACGATGCTCAAGCACCAAAAGAGGAAGACAACAAAAAACCTGGTAAAGAAGAC
GGCAACAAACCTGGCAAAGAAGACGGCAACAAGCCTGGTAAAGAAGACAACAAAAAACCT
GGTAAAGAAGACGGCAACAAGCCTGGTAAAGAAGACAACAACAAACCTGGCAAAGAAGAC
GGCAACAAGCCTGGTAAAGAAGACAACAACAAGCCTGGTAAAGAAGACGGCAACAAGCCT
GGTAAAGAAGACGGCAACAAACCTGGTAAAGAAGACGGCAACGGAGTACATGTCGTTAAA
CCTGGTGATACAGTAAATGACATTGCAAAAGCAAACGGCACTACTGCTGACAAAATTGCT
GCAGATAACAAATTAGCTGATAAAAACATGATCAAACCTGGTCAAGAACTTGTTGTTGAT
AAGAAGCAACCAGCAAACCATGCAGATGCTAACAAAGCTCAAGCATTACCAGAAACTGGT
GAAGAAAATCCATTCATCGGTACAACTGTATTTGGTGGATTATCATTAGCCTTAGGTGCA
GCGTTATTAGCTGGACGTCGTCGCGAACTATAA
SEQ ID NO 68 SPA MW2 
TTGAAAAAGAAAAACATTTATTCAATTCGTAAACTAGGTGTAGGTATTGCATCTGTAACT
TTAGGTACATTACTTATATCTGGTGGCGTAACACCTGCTGCAAATGCTGCGCAACACGAT
GAAGCTCAACAAAATGCTTTTTATCAAGTGTTAAATATGCCTAACTTAAACGCTGATCAA
CGTAATGGTTTTATCCAAAGCCTTAAAGATGATCCAAGCCAAAGTGCTAACGTTTTAGGT
GAAGCTCAAAAACTTAATGACTCTCAAGCTCCAAAAGCTGATGCGCAACAAAATAACTTC
AACAAAGATCAACAAAGCGCCTTCTATGAAATCTTGAACATGCCTAACTTAAACGAAGCG
CAACGCAATGGTTTCATTCAAAGTCTTAAAGACGATCCAAGCCAAAGCACTAACGTTTTA
GGTGAAGCTAAAAAATTAAACGAATCTCAAGCACCGAAAGCTGACAACAATTTCAACAAA
GAACAACAAAATGCTTTCTATGAAATCTTGAACATGCCTAACTTGAACGAAGAACAACGC
AATGGTTTCATCCAAAGCTTAAAAGATGACCCAAGTCAAAGTGCTAACCTATTGTCAGAA
GCTAAAAAGTTAAATGAATCTCAAGCACCGAAAGCGGATAACAAATTCAACAAAGAACAA
CAAAATGCTTTCTATGAAATCTTACATTTACCTAACTTAAACGAAGAACAACGCAATGGT
TTCATCCAAAGCTTAAAAGATGACCCAAGCCAAAGCGCTAACCTTTTAGCAGAAGCTAAA
AAGCTAAATGATGCACAAGCACCAAAAGCTGACAACAAATTCAACAAAGAACAACAAAAT
GCTTTCTATGAAATTTTACATTTACCTAACTTAACTGAAGAACAACGTAACGGCTTCATC
CAAAGCCTTAAAGACGATCCTTCAGTGAGCAAAGAAATTTTAGCAGAAGCTAAAAAGCTA
AACGATGCTCAAGCACCAAAAGAGGAAGACAACAACAAACCTGGTAAAGAAGACGGCAAC
AAACCTGGCAAAGAAGACGGCAACAAACCTGGCAAAGAAGACAACAACAAGCCTGGCAAA
GAAGACGGCAACAAACCTGGTAAAGAAGACAACAAAAAACCTGGTAAAGAAGATGGCAAC
AAGCCTGGCAAAGAAGACAACAACAAACCTGGTAAAGAAGACGGCAACGGAGTACATGTC
GTTAAACCTGGTGATACAGTAAATGACATTGCAAAAGCAAACGGCACTACTGCTGACAAA
ATTGCTGCAGATAACAAATTAGCTGATAAAAACATGATCAAACCTGGTCAAGAACTTGTT
GTTGATAAGAAGCAACCAGCAAACCATGCAGATGCTAACAAAGCTCAAGCATTACCAGAA
ACTGGTGAAGAAAATCCATTCATTGGTACAACTGTATTTGGTGGATTATCATTAGCGTTA
GGTGCAGCGTTATTAGCTGGACGTCGTCGCGAACTATAA
SEQ ID NO 69 PSA COL 
TTGAAAAAGAAAAACATTTATTCAATTCGTAAACTAGGTGTAGGTATTGCATCTGTAACT
TTAGGTACATTACTTATATCTGGTGGCGTAACACCTGCTGCAAATGCTGCGCAACACGAT
GAAGCTCAACAAAATGCTTTTTATCAAGTCTTAAATATGCCTAACTTAAATGCTGATCAA
CGCAATGGTTTTATCCAAAGCCTTAAAGATGATCCAAGCCAAAGTGCTAACGTTTTAGGT
GAAGCTCAAAAACTTAATGACTCTCAAGCTCCAAAAGCTGATGCGCAACAAAATAACTTC
AACAAAGATCAACAAAGCGCCTTCTATGAAATCTTGAACATGCCTAACTTAAACGAAGCG
CAACGTAACGGCTTCATTCAAAGTCTTAAAGACGACCCAAGCCAAAGCACTAACGTTTTA
GGTGAAGCTAAAAAATTAAACGAATCTCAAGCACCGAAAGCTGATAACAATTTCAACAAA
GAACAACAAAATGCTTTCTATGAAATCTTGAATATGCCTAACTTAAACGAAGAACAACGC
 
AATGGTTTCATCCAAAGCTTAAAAGATGACCCAAGCCAAAGTGCTAACCTATTGTCAGAA
GCTAAAAAGTTAAATGAATCTCAAGCACCGAAAGCGGATAACAAATTCAACAAAGAACAA
CAAAATGCTTTCTATGAAATCTTACATTTACCTAACTTAAACGAAGAACAACGCAATGGT
TTCATCCAAAGCCTAAAAGATGACCCAAGCCAAAGCGCTAACCTTTTAGCAGAAGCTAAA
AAGCTAAATGATGCTCAAGCACCAAAAGCTGACAACAAATTCAACAAAGAACAACAAAAT
GCTTTCTATGAAATTTTACATTTACCTAACTTAACTGAAGAACAACGTAACGGCTTCATC
CAAAGCCTTAAAGACGATCCTTCAGTGAGCAAAGAAATTTTAGCAGAAGCTAAAAAGCTA
AACGATGCTCAAGCACCAAAAGAGGAAGACAATAACAAGCCTGGCAAAGAAGACAATAAC
AAGCCTGGCAAAGAAGACAACAACAAGCCTGGTAAAGAAGACAACAACAAGCCTGGCAAA
GAAGACGGCAACAAGCCTGGTAAAGAAGACAACAAAAAACCTGGTAAAGAAGATGGCAAC
AAGCCTGGTAAAGAAGACAACAAAAAACCTGGTAAAGAAGACGGCAACAAGCCTGGCAAA
GAAGATGGCAACAAACCTGGTAAAGAAGATGGTAACGGAGTACATGTCGTTAAACCTGGT
GATACAGTAAATGACATTGCAAAAGCAAACGGCACTACTGCTGACAAAATTGCTGCAGAT
AACAAATTAGCTGATAAAAACATGATCAAACCTGGTCAAGAACTTGTTGTTGATAAGAAG
CAACCAGCAAACCATGCAGATGCTAACAAAGCTCAAGCATTACCAGAAACTGGTGAAGAA
AATCCATTCATCGGTACAACTGTATTTGGTGGATTATCATTAGCCTTAGGTGCAGCGTTA
TTAGCTGGACGTCGTCGCGAACTATAA
SEQ ID NO 70 SPA MRSA252 
TTGAAAAAGAAAAACATTTATTCAATTCGTAAACTAGGTGTAGGTATTGCATCTGTAACT
TTAGGTACATTACTTATATCTGGTGGCGTAACACCTGCTGCAAATGCTGCGCAACACGAT
GAAGCTCAACAAAATGCTTTTTATCAAGTGTTAAATATGCCTAACTTAAACGCTGATCAA
CGTAATGGTTTTATCCAAAGCCTTAAAGATGATCCAAGCCAAAGTGCTAACGTTTTAGGT
GAAGCTCAAAAACTTAATGACTCTCAAGCTCCAAAAGCTGATGCGCAACAAAATAAGTTC
AACAAAGATCAACAAAGCGCCTTCTATGAAATCTTGAACATGCCTAACTTAAACGAAGAG
CAACGCAATGGTTTCATTCAAAGTCTTAAAGACGATCCAAGCCAAAGCACTAACGTTTTA
GGTGAAGCTAAAAAATTAAACGAATCTCAAGCACCGAAAGCTGACAACAATTTCAACAAA
GAACAACAAAATGCTTTCTATGAAATCTTGAACATGCCTAACTTGAACGAAGAACAACGC
AATGGTTTCATCCAAAGCTTAAAAGATGACCCAAGTCAAAGTGCTAACCTTTTAGCAGAA
GCTAAAAAGTTAAATGAATCTCAAGCACCGAAAGCTGATAACAAATTCAACAAAGAACAA
CAAAATGCTTTCTATGAAATCTTACATTTACCTAACTTAAATGAAGAACAACGCAATGGT
TTCATCCAAAGCTTAAAAGATGACCCAAGCCAAAGCGCTAACCTTTTAGCAGAAGCTAAA
AAGCTAAATGATGCACAAGCACCAAAAGCTGACAACAAATTCAACAAAGAACAACAAAAT
GCTTTCTATGAAATTTTACATTTACCTAACTTAACTGAAGAACAACGTAACGGCTTCATC
CAAAGCCTTAAAGACGATCCTTCAGTGAGCAAAGAAATTTTAGCAGAAGCTAAAAAGCTA
AACGATGCTCAAGCACCAAAAGAGGAAGACAACAACAAGCCTGGCAAAGAAGACAACAAC
AAGCCTGGTAAAGAAGACGGCAACAAACCTGGTAAAGAAGACAACAAAAAACCTGGCAAA
GAAGACGGCAACAAACCTGGTAAAGAAGACAACAAAAAACCTGGCAAAGAAGATGGCAAC
AAACCTGGTAAAGAAGACGGCAACAAGCCTGGTAAAGAAGATGGCAACAAGCCTGGTAAA
GAAGATGGCAACAAGCCTGGTAAAGAAGATGGCAACAAGCCTGGTAAAGAAGACGGCAAC
GGAGTACATGTCGTTAAACCTGGTGATACAGTAAATGACATTGCAAAAGCAAACGGCACT
ACTGCTGACAAAATTGCTGCAGATAACAAATTAGCTGATAAAAACATGATCAAACCTGGT
CAAGAACTTGTTGTTGATAAGAAGCAACCAGCAAACCATGCAGATGCTAACAAAGCTCAA
GCATTACCAGAAACTGGTGAAGAAAATCCATTCATCGGTACAACTGTATTTGGTGGATTA
TCATTAGCGTTAGGTGCAGCGTTATTAGCTGGACGTCGTCGCGAACTATAA
SEQ ID NO 71 SPA MSSA476 
TTGAAAAAGAAAAACATTTATTCAATTCGTAAACTAGGTGTAGGTATTGCATCTGTAACT
TTAGGTACATTACTTATATCTGGTGGCGTAACACCTGCTGCAAATGCTGCGCAACACGAT
GAAGCTCAACAAAATGCTTTTTATCAAGTGTTAAATATGCCTAACTTAAACGCTGATCAA
CGTAATGGTTTTATCCAAAGCCTTAAAGATGATCCAAGCCAAAGTGCTAACGTTTTAGGT
GAAGCTCAAAAACTTAATGACTCTCAAGCTCCAAAAGCTGATGCGCAACAAAATAACTTC
AACAAAGATCAACAAAGCGCCTTCTATGAAATCTTGAACATGCCTAACTTAAACGAAGCG
CAACGCAATGGTTTCATTCAAAGTCTTAAAGACGATCCAAGCCAAAGCACTAACGTTTTA
GGTGAAGCTAAAAAATTAAACGAATCTCAAGCACCGAAAGCTGACAACAATTTCAACAAA
 
GAACAACAAAATGCTTTCTATGAAATCTTGAACATGCCTAACTTGAACGAAGAACAACGC
AATGGTTTCATCCAAAGCTTAAAAGATGACCCAAGTCAAAGTGCTAACCTATTGTCAGAA
GCTAAAAAGTTAAATGAATCTCAAGCACCGAAAGCGGATAACAAATTCAACAAAGAACAA
CAAAATGCTTTCTATGAAATCTTACATTTACCTAACTTAAACGAAGAACAACGCAATGGT
TTCATCCAAAGCTTAAAAGATGACCCAAGCCAAAGCGCTAACCTTTTAGCAGAAGCTAAA
AAGCTAAATGATGCACAAGCACCAAAAGCTGACAACAAATTCAACAAAGAACAACAAAAT
GCTTTCTATGAAATTTTACATTTACCTAACTTAACTGAAGAACAACGTAACGGCTTCATC
CAAAGCCTTAAAGACGATCCTTCAGTGAGCAAAGAAATTTTAGCAGAAGCTAAAAAGCTA
AACGATGCTCAAGCACCAAAAGAGGAAGACAACAACAAACCTGGTAAAGAAGACGGCAAC
AAACCTGGTAAAGAAGACGGCAACAAACCTGGCAAAGAAGACAACAACAAGCCTGGCAAA
GAAGACGGCAACAAACCTGGTAAAGAAGACAACAAAAAACCTGGTAAAGAAGATGGCAAC
AAGCCTGGCAAAGAAGACAACAACAAACCTGGTAAAGAAGACGGCAACGGAGTACATGTC
GTTAAACCTGGTGATACAGTAAATGACATTGCAAAAGCAAACGGCACTACTGCTGACAAA
ATTGCTGCAGATAACAAATTAGCTGATAAAAACATGATCAAACCTGGTCAAGAACTTGTT
GTTGATAAGAAGCAACCAGCAAACCATGCAGATGCTAACAAAGCTCAAGCATTACCAGAA
ACTGGTGAAGAAAATCCATTCATTGGTACAACTGTATTTGGTGGATTATCATTAGCGTTA
GGTGCAGCGTTATTAGCTGGACGTCGTCGCGAACTATAA
SEQ ID NO 72 SPA V8 
ATGATGACTTTACAAATACATACAGGGGGTATTAATTTGAAAAAGAAAAACATTTATTCA
ATTCGTAAACTAGGTGTAGGTATTGCATCTGTAACTTTAGGTACATTACTTATATCTGGT
GGCGTAACACCTGCTGCAAATGCTGCGCAACACGATGAAGCTCAACAAAATGCTTTTTAT
CAAGTGTTAAATATGCCTAACTTAAACGCTGATCAACGTAATGGTTTTATCCAAAGCCTT
AAAGATGATCCAAGCCAAAGTGCTAACGTTTTAGGTGAAGCTCAAAAACTTAATGACTCT
CAAGCTCCAAAAGCTGATGCGCAACAAAATAAGTTCAACAAAGATCAACAAAGCGCCTTC
TATGAAATCTTGAACATGCCTAACTTAAACGAAGAGCAACGCAATGGTTTCATTCAAAGT
CTTAAAGACGATCCAAGCCAAAGCACTAACGTTTTAGGTGAAGCTAAAAAATTAAACGAA
TCTCAAGCACCGAAAGCTGACAACAATTTCAACAAAGAACAACAAAATGCTTTCTATGAA
ATCTTGAACATGCCTAACTTGAACGAAGAACAACGCAATGGTTTCATCCAAAGCTTAAAA
GATGACCCAAGTCAAAGTGCTAACCTTTTAGCAGAAGCTAAAAAGCTAAATGATGCACAA
GCACCAAAAGCTGACAACAAATTCAACAAAGAACAACAAAATGCTTTCTATGAAATTTTA
CATTTACCTAACTTAACTGAAGAACAACGTAACGGCTTCATCCAAAGCCTTAAAGACGAT
CCTTCAGTGAGCAAAGAAATTTTAGCAGAAGCTAAAAAGCTAAACGATGCTCAAGCACCA
AAAGAGGAAGACAACAACAAGCCTGGCAAAGAAGACAACAACAAGCCTGGTAAAGAAGAC
GGCAACAAACCTGGTAAAGAAGACAACAAAAAACCTGGCAAAGAAGACGGCAACAAACCT
GGTAAAGAAGACAACAAAAAACCTGGTAAAGAAGATGGCAACAAACCTGGTAAAGAAGAC
GGCAACAAGCCTGGTAAAGAAGATGGCAACAAGCCTGGTAAAGAAGACGGCAACGGAGTA
CATGTCGTTAAACCTGGTGATACAGTAAATGACATTGCAAAAGCAAACGGCACTACTGCT
GACAAAATTGCTGTAGATAACAAATTAGCTGATAAAAACATGATCAAACCTGGTCAAGAA
CTTGTTGTTGATAAGAAGCAACCAGCAAACCATGCAGATGCTAACAAAGCTCAAGCATTA
CCAGAAACTGGTGAAGAAAATCCATTCATCGGTACAACTGTATTTGGTGGATTATCATTA
GCGTTAGGTGCAGCGTTATTAGCTGGACGTCGTCGCGAACTATAA
SEQ ID NO 73 fusion protein 
ATGGCGCAACACGATGAAGCTCAACAAAATGCTTTTTATCAAGTCTTAAATATGCCTAAC
TTAAATGCTGATCAACGCAATGGTTTTATCCAAAGCCTTAAAGATGATCCAAGCCAAAGT
GCTAACGTTTTAGGTGAAGCTCAAAAACTTAATGACTCTCAAGCTCCAAAAGCTGATGCG
CAACAAAATAACTTCAACAAAGATCAACAAAGCGCCTTCTATGAAATCTTGAACATGCCT
AACTTAAACGAAGCGCAACGTAACGGCTTCATTCAAAGTCTTAAAGACGACCCAAGCCAA
AGCACTAACGTTTTAGGTGAAGCTAAAAAATTAAACGAATCTCAAGCACCGAAAGCTGAT
AACAATTTCAACAAAGAACAACAAAATGCTTTCTATGAAATCTTGAATATGCCTAACTTA
AACGAAGAACAACGCAATGGTTTCATCCAAAGCTTAAAAGATGACCCAAGCCAAAGTGCT
 
AACCTATTGTCAGAAGCTAAAAAGTTAAATGAATCTCAAGCACCGAAAGCGGATAACAAA
TTCAACAAAGAACAACAAAATGCTTTCTATGAAATCTTACATTTACCTAACTTAAACGAA
GAACAACGCAATGGTTTCATCCAAAGCCTAAAAGATGACCCAAGCCAAAGCGCTAACCTT
TTAGCAGAAGCTAAAAAGCTAAATGATGCTCAAGCACCAAAAGCTGACAACAAATTCAAC
AAAGAACAACAAAATGCTTTCTATGAAATTTTACATTTACCTAACTTAACTGAAGAACAA
CGTAACGGCTTCATCCAAAGCCTTAAAGACGATCCTTCAGTGAGCAAAGAAATTTTAGCA
GAAGCTAAAAAGCTAAACGATGCTCAAGCACCAAAAGAGGAAGACAATAACAAGCCTGGC
AAAGAAGACAATAACAAGCCTGGCAAAGAAGACAACAACAAGCCTGGTAAAGAAGACAAC
AACAAGCCTGGTAAAGAAGACAACAACAAGCCTGGCAAAGAAGACGGCAACAAGCCTGGT
AAAGAAGACAACAAAAAACCTGGTAAAGAAGATGGCAACAAGCCTGGTAAAGAAGACAAC
AAAAAACCTGGTAAAGAAGACGGCAACAAGCCTGGCAAAGAAGATGGCAACAAACCTGGT
AAAGAAGATGGTAACGGAGTACATGTCGGCGGATCCAGTGAAAACACGCAACAAACTTCA
ACTAAGCACCAAACAACTCAAAACAACTACGTAACAGATCAACAAAAAGCTTTTTATCAA
GTATTACATCTAAAAGGTATCACAGAAGAACAACGTAACCAATACATCAAAACATTACGC
GAACACCCAGAACGTGCACAAGAAGTATTCTCTGAATCACTTAAAGACAGCAAGAACCCA
GACCGACGTGTTGCACAACAAAACGCTTTTTACAATGTTCTTAAAAATGATAACTTAACT
GAACAAGAAAAAAATAATTACATTGCACAAATTAAAGAAAACCCTGATAGAAGCCAACAA
GTTTGGGTAGAATCAGTACAATCTTCTAAAGCTAAAGAACGTCAAAATATTGAAAATGCG
GATAAAGCAATTAAAGATTTCCAAGATAACAAAGCACCACACGATAAATCAGCAGCATAT
GAAGCTAACTCAAAATTACCTAAAGATTTACGTGATAAAAACAACCGCTTTGTAGAAAAA
GTTTCAATTGAAAAAGCA
SEQ ID NO 74 fusion protein with His tag 
ATGGCGCAACACGATGAAGCTCAACAAAATGCTTTTTATCAAGTCTTAAATATGCCTAAC
TTAAATGCTGATCAACGCAATGGTTTTATCCAAAGCCTTAAAGATGATCCAAGCCAAAGT
GCTAACGTTTTAGGTGAAGCTCAAAAACTTAATGACTCTCAAGCTCCAAAAGCTGATGCG
CAACAAAATAACTTCAACAAAGATCAACAAAGCGCCTTCTATGAAATCTTGAACATGCCT
AACTTAAACGAAGCGCAACGTAACGGCTTCATTCAAAGTCTTAAAGACGACCCAAGCCAA
AGCACTAACGTTTTAGGTGAAGCTAAAAAATTAAACGAATCTCAAGCACCGAAAGCTGAT
AACAATTTCAACAAAGAACAACAAAATGCTTTCTATGAAATCTTGAATATGCCTAACTTA
AACGAAGAACAACGCAATGGTTTCATCCAAAGCTTAAAAGATGACCCAAGCCAAAGTGCT
AACCTATTGTCAGAAGCTAAAAAGTTAAATGAATCTCAAGCACCGAAAGCGGATAACAAA
TTCAACAAAGAACAACAAAATGCTTTCTATGAAATCTTACATTTACCTAACTTAAACGAA
GAACAACGCAATGGTTTCATCCAAAGCCTAAAAGATGACCCAAGCCAAAGCGCTAACCTT
TTAGCAGAAGCTAAAAAGCTAAATGATGCTCAAGCACCAAAAGCTGACAACAAATTCAAC
AAAGAACAACAAAATGCTTTCTATGAAATTTTACATTTACCTAACTTAACTGAAGAACAA
CGTAACGGCTTCATCCAAAGCCTTAAAGACGATCCTTCAGTGAGCAAAGAAATTTTAGCA
GAAGCTAAAAAGCTAAACGATGCTCAAGCACCAAAAGAGGAAGACAATAACAAGCCTGGC
AAAGAAGACAATAACAAGCCTGGCAAAGAAGACAACAACAAGCCTGGTAAAGAAGACAAC
AACAAGCCTGGTAAAGAAGACAACAACAAGCCTGGCAAAGAAGACGGCAACAAGCCTGGT
AAAGAAGACAACAAAAAACCTGGTAAAGAAGATGGCAACAAGCCTGGTAAAGAAGACAAC
AAAAAACCTGGTAAAGAAGACGGCAACAAGCCTGGCAAAGAAGATGGCAACAAACCTGGT
 
AAAGAAGATGGTAACGGAGTACATGTCGGCGGATCCAGTGAAAACACGCAACAAACTTCA
ACTAAGCACCAAACAACTCAAAACAACTACGTAACAGATCAACAAAAAGCTTTTTATCAA
GTATTACATCTAAAAGGTATCACAGAAGAACAACGTAACCAATACATCAAAACATTACGC
GAACACCCAGAACGTGCACAAGAAGTATTCTCTGAATCACTTAAAGACAGCAAGAACCCA
GACCGACGTGTTGCACAACAAAACGCTTTTTACAATGTTCTTAAAAATGATAACTTAACT
GAACAAGAAAAAAATAATTACATTGCACAAATTAAAGAAAACCCTGATAGAAGCCAACAA
GTTTGGGTAGAATCAGTACAATCTTCTAAAGCTAAAGAACGTCAAAATATTGAAAATGCG
GATAAAGCAATTAAAGATTTCCAAGATAACAAAGCACCACACGATAAATCAGCAGCATAT
GAAGCTAACTCAAAATTACCTAAAGATTTACGTGATAAAAACAACCGCTTTGTAGAAAAA
GTTTCAATTGAAAAAGCAACTAGTGGCCACCATCACCATCACCATTAA
Sequence ID NO 75 - protein A-SdrG-Sbi fusion protein 
atgGCGCAACACGATGAAGCTCAACAAAATGCTTTTTATCAAGTCTTAAATATGCCTAACTTAAATGCTGATCAACGCAA
TGGTTTTATCCAAAGCCTTAAAGATGATCCAAGCCAAAGTGCTAACGTTTTAGGTGAAGCTCAAAAACTTAATGACTCTC
AAGCTCCAAAAGCTGATGCGCAACAAAATAACTTCAACAAAGATCAACAAAGCGCCTTCTATGAAATCTTGAACATGCCT
AACTTAAACGAAGCGCAACGTAACGGCTTCATTCAAAGTCTTAAAGACGACCCAAGCCAAAGCACTAACGTTTTAGGTGA
AGCTAAAAAATTAAACGAATCTCAAGCACCGAAAGCTGATAACAATTTCAACAAAGAACAACAAAATGCTTTCTATGAAA
TCTTGAATATGCCTAACTTAAACGAAGAACAACGCAATGGTTTCATCCAAAGCTTAAAAGATGACCCAAGCCAAAGTGCT
AACCTATTGTCAGAAGCTAAAAAGTTAAATGAATCTCAAGCACCGAAAGCGGATAACAAATTCAACAAAGAACAACAAAA
TGCTTTCTATGAAATCTTACATTTACCTAACTTAAACGAAGAACAACGCAATGGTTTCATCCAAAGCCTAAAAGATGACC
CAAGCCAAAGCGCTAACCTTTTAGCAGAAGCTAAAAAGCTAAATGATGCTCAAGCACCAAAAGCTGACAACAAATTCAAC
AAAGAACAACAAAATGCTTTCTATGAAATTTTACATTTACCTAACTTAACTGAAGAACAACGTAACGGCTTCATCCAAAG
CCTTAAAGACGATCCTTCAGTGAGCAAAGAAATTTTAGCAGAAGCTAAAAAGCTAAACGATGCTCAAGCACCAAAAGAGG
AAGACAATAACAAGCCTGGCAAAGAAGACAATAACAAGCCTGGCAAAGAAGACAACAACAAGCCTGGTAAAGAAGACAAC
AACAAGCCTGGTAAAGAAGACAACAACAAGCCTGGCAAAGAAGACGGCAACAAGCCTGGTAAAGAAGACAACAAAAAACC
TGGTAAAGAAGATGGCAACAAGCCTGGTAAAGAAGACAACAAAAAACCTGGTAAAGAAGACGGCAACAAGCCTGGCAAAG
AAGATGGCAACAAACCTGGTAAAGAAGATGGTAACGGAGTACATGTCGGCggatccGAGGAGAATTCAGTACAAGACGTT
AAAGATTCGAATACGGATGATGAATTATCAGACAGCAATGATCAGTCTAGTGATGAAGAAAAGAATGATGTGATCAATAA
TAATCAGTCAATAAACACCGACGATAATAACCAAATAATTAAAAAAGAAGAAACGAATAACTACGATGGCATAGAAAAAC
GCTCAGAAGATAGAACAGAGTCAACAACAAATGTAGATGAAAACGAAGCAACATTTTTACAAAAGACCCCTCAAGATAAT
ACTCATCTTACAGAAGAAGAGGTAAAAGAATCCTCATCAGTCGAATCCTCAAATTCATCAATTGATACTGCCCAACAACC
ATCTCACACAACAATAAATAGAGAAGAATCTGTTCAAACAAGTGATAATGTAGAAGATTCACACGTATCAGATTTTGCTA
ACTCTAAAATAAAAGAGAGTAACACTGAATCTGGTAAAGAAGAGAATACTATAGAGCAACCTAATAAAGTAAAAGAAGAT
TCAACAACAAGTCAGCCGTCTGGCTATACAAATATAGATGAAAAAATTTCAAATCAAGATGAGTTATTAAATTTACCAAT
AAATGAATATGAAAATAAGGCTAGACCATTATCTACAACATCTGCCCAACCATCGATTAAACGTGTAACCGTAAATCAAT
TAGCGGCGGAACAAGGTTCGAATGTTAATCATTTAATTAAAGTTACTGATCAAAGTATTACTGAAGGATATGATGATAGT
GAAGGTGTTATTAAAGCACATGATGCTGAAAACTTAATCTATGATGTAACTTTTGAAGTAGATGATAAGGTGAAATCTGG
TGATACGATGACAGTGGATATAGATAAGAATACAGTTCCATCAGATTTAACCGATAGCTTTACAATACCAAAAATAAAAG
ATAATTCTGGAGAAATCATCGCTACAGGTACTTATGATAACAAAAATAAACAAATCACCTATACTTTTACAGATTATGTA
GATAAGTATGAAAATATTAAAGCACACCTTAAATTAACGTCATACATTGATAAATCAAAGGTTCCAAATAATAATACCAA
GTTAGATGTAGAATATAAAACGGCCCTTTCATCAGTAAATAAAACAATTACGGTTGAATATCAAAGACCTAACGAAAATC
GGACTGCTAACCTTCAAAGTATGTTTACAAACATAGATACGAAAAATCATACAGTTGAGCAAACGATTTATATTAACCCT
 
CTTCGTTATTCAGCCAAGGAAACAAATGTAAATATTTCAGGGAATGGTGATGAAGGTTCAACAATTATAGACGATAGCAC
AATAATTAAAGTTTATAAGGTTGGAGATAATCAAAATTTACCAGATAGTAACAGAATTTATGATTACAGTGAATATGAAG
ATGTCACAAATGATGATTATGCCCAATTAGGAAATAATAATGATGTGAATATTAATTTTGGTAATATAGATTCACCATAT
ATTATTAAAGTTATTAGTAAATATGACCCTAATAAGGATGATTACACGACTATACAGCAAACTGTGACAATGCAGACGAC
TATAAATGAGTATACTGGTGAGTTTAGAACAGCATCCTATGATAATACAATTGCTTTCTCTACAAGTTCAGGTCAAGGAC
AAGGTGACTTGCCTCCTGAAAAAACTTATAAAATCGGAGATTACGTATGGGAAGATGTAGATAAAGATGGTATTCAAAAT
ACAAATGATAATGAAAAACCGCTTAGTAATGTATTGGTAACTTTGACGTATCCTGATGGAACTTCAAAATCAGTCAGAAC
AGATGAAGATGGGAAATATCAATTTGATGGATTGAAAAACGGATTGACTTATAAAATTACATTCGAAACACCTGAAGGAT
ATACGCCGACGCTTAAACATTCAGGAACAAATCCTGCACTAGACTCAGAAGGTAATTCTGTATGGGTAACTATTAATGGA
CAAGACGATATGACGATTGATAGTGGATTTTATCAAACACCTAAATACAGCTTAGGGAACTATGTATGGTATGACACTAA
TAAAGATGGTATTCAAGGTGATGATGAAAAAGGAATCTCTGGAGTTAAAGTGACGTTAAAAGATGAAAACGGAAATATCA
TTAGTACAACTACAACCGATGAAAATGGAAAGTATCAATTTGATAATTTAAATAGTGGTAATTATATTGTTCATTTTGAT
AAACCTTCAGGTATGACTCAAACAACAACAGATTCTGGTGATGATGACGAACAGGATGCTGATGGGGAAGAAGTTCATGT
AACAATTACTGATCATGATGACTTTAGTATAGATAACGGATACTATGATGACGAAggatccAGTGAAAACACGCAACAAA
CTTCAACTAAGCACCAAACAACTCAAAACAACTACGTAACAGATCAACAAAAAGCTTTTTATCAAGTATTACATCTAAAA
GGTATCACAGAAGAACAACGTAACCAATACATCAAAACATTACGCGAACACCCAGAACGTGCACAAGAAGTATTCTCTGA
ATCACTTAAAGACAGCAAGAACCCAGACCGACGTGTTGCACAACAAAACGCTTTTTACAATGTTCTTAAAAATGATAACT
TAACTGAACAAGAAAAAAATAATTACATTGCACAAATTAAAGAAAACCCTGATAGAAGCCAACAAGTTTGGGTAGAATCA
GTACAATCTTCTAAAGCTAAAGAACGTCAAAATATTGAAAATGCGGATAAAGCAATTAAAGATTTCCAAGATAACAAAGC
ACCACACGATAAATCAGCAGCATATGAAGCTAACTCAAAATTACCTAAAGATTTACGTGATAAAAACAACCGCTTTGTAG
AAAAAGTTTCAATTGAAAAAGCAactagtGGCCACCATCACCATCACCATTAA
Sequence ID No 76 Protein A-SdrG-Sbi fusion protein

MAQHDEAQQNAFYQVLNMPNLNADQRNGFIQSLKDDPSQSANVLGEAQKLNDSQAPKADAQQNNFNKDQQSAFYEILNMP
NLNEAQRNGFIQSLKDDPSQSTNVLGEAKKLNESQAPKADNNFNKEQQNAFYEILNMPNLNEEQRNGFIQSLKDDPSQSA
NLLSEAKKLNESQAPKADNKFNKEQQNAFYEILHLPNLNEEQRNGFIQSLKDDPSQSANLLAEAKKLNDAQAPKADNKFN
KEQQNAFYEILHLPNLTEEQRNGFIQSLKDDPSVSKEILAEAKKLNDAQAPKEEDNNKPGKEDNNKPGKEDNNKPGKEDN
NKPGKEDNNKPGKEDGNKPGKEDNKKPGKEDGNKPGKEDNKKPGKEDGNKPGKEDGNKPGKEDGNGVHVGGSEENSVQDV
KDSNTDDELSDSNDQSSDEEKNDVINNNQSINTDDNNQIIKKEETNNYDGIEKRSEDRTESTTNVDENEATFLQKTPQDN
THLTEEEVKESSSVESSNSSIDTAQQPSHTTINREESVQTSDNVEDSHVSDFANSKIKESNTESGKEENTIEQPNKVKED
STTSQPSGYTNIDEKISNQDELLNLPINEYENKARPLSTTSAQPSIKRVTVNQLAAEQGSNVNHLIKVTDQSITEGYDDS
EGVIKAHDAENLIYDVTFEVDDKVKSGDTMTVDIDKNTVPSDLTDSFTIPKIKDNSGEIIATGTYDNKNKQITYTFTDYV
DKYENIKAHLKLTSYIDKSKVPNNNTKLDVEYKTALSSVNKTITVEYQRPNENRTANLQSMFTNIDTKNHTVEQTIYINP
LRYSAKETNVNISGNGDEGSTIIDDSTIIKVYKVGDNQNLPDSNRIYDYSEYEDVTNDDYAQLGNNNDVNINFGNIDSPY
IIKVISKYDPNKDDYTTIQQTVTMQTTINEYTGEFRTASYDNTIAFSTSSGQGQGDLPPEKTYKIGDYVWEDVDKDGIQN
TNDNEKPLSNVLVTLTYPDGTSKSVRTDEDGKYQFDGLKNGLTYKITFETPEGYTPTLKHSGTNPALDSEGNSVWVTING
QDDMTIDSGFYQTPKYSLGNYVWYDTNKDGIQGDDEKGISGVKVTLKDENGNIISTTTTDENGKYQFDNLNSGNYIVHFD
KPSGMTQTTTDSGDDDEQDADGEEVHVTITDHDDFSIDNGYYDDEGSSENTQQTSTKHQTTQNNYVTDQQKAFYQVLHLK
GITEEQRNQYIKTLREHPERAQEVFSESLKDSKNPDRRVAQQNAFYNVLKNDNLTEQEKNNYIAQIKENPDRSQQVWVES
VQSSKAKERQNIENADKAIKDFQDNKAPHDKSAAYEANSKLPKDLRDKNNRFVEKVSIEKATSGHHHHHH

## Claims

1. An immunogenic composition comprising two different isolated staphylococcal polypeptides, each comprising an IgG binding domain, wherein the isolated staphylococcal polypeptides comprise an isolated protein A polypeptide from *S*. *aureus* having a sequence which is at least 90% identical to SEQ ID NO:12-40, and an isolated Sbi polypeptide from *S*. *aureus* having a sequence which is at least 90% identical to SEQ ID NO:1-11.

2. The immunogenic composition of claim 1 wherein the isolated protein A polypeptide is covalently bonded to the isolated Sbi polypeptide to form a fusion protein.

3. The immunogenic composition of claim 2 wherein the fusion protein has a polypeptide sequence which is at least 90% identical to the sequence of SEQ ID NO:41-42 or 76.

4. The immunogenic composition of any one of claims 1-3 comprising a further staphylococcal antigen.

5. The immunogenic composition of claim 4 wherein the further staphylococcal antigen comprises *S*. *aureus* type 8 capsular polysaccharide.

6. The immunogenic composition of claim 5 wherein the *S*. *aureus* type 8 capsular polysaccharide is conjugated to a carrier protein.

7. The immunogenic composition of any one of clams 6 wherein the carrier protein is independently selected from the group consisting of tetanus toxoid, diphtheria toxoid, CRM197, protein D, alpha toxin, SdrG, ClfA, IsdA, IsdB, IsdH, protein A, Sbi and a proteinA-Sbi fusion protein.

8. The immunogenic composition of claim 6 or 7 wherein the further staphylococcal antigen comprises a protein selected from the group consisting of Ebh, Elastin binding protein, EFB, ClfA, ClfB, SdrC, SdrG, FnbA, FbpA, IsaA/PisA, Penicillin binding protein 4, AhpC, SsaA, Aap, SasA, IsdA, IsdB, IsdC, HarA, alpha toxin, alpha toxin H35R mutant and RAP.

9. A polypeptide comprising: a) a protein A part having an amino acid sequence having at least 90% identity to an amino acid sequence selected from the group consisting of SEQ ID NOs 12-40 and b) an Sbi part which has an amino acid sequence having at least 90% identity to an amino acid sequence selected from the group consisting of SEQ ID NOs 1-11.

10. The polypeptide of claim 9 having an amino acid sequence which is at least 90% identical to SEQ ID NO 41 or 42 or 76.

11. A polynucleotide comprising: a) a Protein A encoding region having at least 90% identity to a polynucleotide sequence selected from the group consisting of SEQ ID NOs 54-72 and b) an Sbi-encoding region which has at least 90% identity to a polynucleotide sequence selected from the group consisting of SEQ ID NOs 43-53.

12. A polynucleotide encoding a fusion protein comprising: a) a protein A part having at least 90% identity to an amino acid sequence selected from the group consisting of SEQ ID NOs 12-40 and b) an Sbi part which has at least 90% identity to an amino acid sequence selected from the group consisting of SEQ ID NOs 1-11.

13. A vaccine comprising the immunogenic composition of any one of claims 1-8 or the polypeptide of claim 9 or 10 and a pharmaceutically acceptable carrier.

14. A process for making the vaccine of claim 13 comprising the step of adding a pharmaceutically acceptable excipient to the immunogenic composition of any one of claims 1-8 or the polypeptide of any one of claims 9 or 10.

15. An immunogenic composition according to any one of claims 1-8 for use in the treatment of prevention of staphylococcal disease.

## Patentansprüche

1. Immunogene Zusammensetzung, umfassend zwei verschiedene isolierte Staphylokokken-Polypeptide, wovon jedes eine IgG-bindende Domäne umfasst, wobei die isolierten Staphylokokken-Polypeptide ein isoliertes Protein-A-Polypeptid von *S. aureus,* das eine Sequenz aufweist, welche mindestens 90 % identisch ist zu SEQ ID NO: 12-40, und ein isoliertes Sbi-Polypeptid von *S. aureus,* das eine Sequenz aufweist, welche mindestens 90 % identisch ist zu SEQ ID NO: 1-11, umfassen.

2. Immunogene Zusammensetzung gemäß Anspruch 1, wobei das isolierte Protein-A-Polypeptid kovalent an das isolierten Sbi-Polypeptid gebunden ist, sodass ein Fusionsprotein gebildet wird.

3. Immunogene Zusammensetzung gemäß Anspruch 2, wobei das Fusionsprotein eine Polypeptid-Sequenz aufweist, welche mindestens 90 % identisch ist zu der Sequenz von SEQ ID NO: 41-42 oder 76.

4. Immunogene Zusammensetzung gemäß einem der Ansprüche 1 bis 3, umfassend ein weiteres Staphylokokken-Antigen.

5. Immunogene Zusammensetzung gemäß Anspruch 4, wobei das weitere Staphylokokken-Antigen *S. aureus* Typ 8-Kapsel-Polysaccharid umfasst.

6. Immunogene Zusammensetzung gemäß Anspruch 5, wobei das *S. aureus* Typ 8-Kapsel-Polysaccharid mit einem Trägerprotein konjugiert ist.

7. Immunogene Zusammensetzung gemäß einem der Ansprüche 6, wobei das Trägerprotein unabhängig ausgewählt ist aus der Gruppe, bestehend aus Tetanus-Toxoid, Diphtherie-Toxoid, CRM197, Protein D, Alpha-Toxin, SdrG, ClfA, IsdA, IsdB, IsdH, Protein A, Sbi und einem Protein-A-Sbi-Fusionsprotein.

8. Immunogene Zusammensetzung gemäß Anspruch 6 oder 7, wobei das weitere Staphylokokken-Antigen ein Protein umfasst, das ausgewählt ist aus der Gruppe, bestehend aus Ebh, Elastin-bindendem Protein, EFB, ClfA, ClfB, SdrC, SdrG, FnbA, FbpA, IsaA/PisA, Penicillin-bindendem Protein 4, Ahpc, SsaA, Aap, SasA, IsdA, IsdB, IsdC, HarA, Alpha-Toxin, Alpha-Toxin H35R-Mutanten und RAP.

9. Polypeptid, umfassend: a) einen Protein-A-Teil mit einer Aminosäuresequenz mit mindestens 90 % Identität zu einer Aminosäuresequenz, ausgewählt aus der Gruppe, bestehend aus SEQ ID NOs: 12-40, und b) einen Sbi-Teil mit einer Aminosäuresequenz mit mindestens 90 % Identität zu einer Aminosäuresequenz, ausgewählt aus der Gruppe, bestehend aus SEQ ID NOs: 1-11.

10. Polypeptid gemäß Anspruch 9 mit einer Aminosäuresequenz, welche mindestens 90 % identisch ist zu SEQ ID NO: 41 oder 42 oder 76.

11. Polynukleotid, umfassend: a) eine Protein-A-kodierende Region mit mindestens 90 % Identität zu einer Polynukleotidsequenz, ausgewählt aus der Gruppe, bestehend aus SEQ ID NOs: 54-72 und b) eine Sbikodierende Region mit mindestens 90 % Identität zu einer Polynukleotidsequenz, ausgewählt aus der Gruppe, bestehend aus SEQ ID NOs: 43-53.

12. Polynukleotid, das ein Fusionsprotein kodiert, umfassend: a) einen Protein-A-Teil mit mindestens 90 % Identität zu einer Aminosäuresequenz, ausgewählt aus der Gruppe, bestehend aus SEQ ID NO: 12-40, und b) einen Sbi-Teil mit mindestens 90 % Identität zu einer Aminosäuresequenz, ausgewählt aus der Gruppe, bestehend aus SEQ ID NO: 1-11.

13. Impfstoff, der die immunogene Zusammensetzung gemäß einem der Ansprüche 1 bis 8 oder das Polypeptid gemäß Anspruch 9 oder 10 und einen pharmazeutisch annehmbaren Träger umfasst.

14. Verfahren zur Herstellung des Impfstoffs gemäß Anspruch 13, das den Schritt der Zugabe eines pharmazeutisch annehmbaren Hilfsstoffs zu der immunogenen Zusammensetzung gemäß einem der Ansprüche 1 bis 8 oder dem Polypeptid gemäß einem der Ansprüche 9 oder 10 umfasst.

15. Immunogene Zusammensetzung gemäß einem der Ansprüche 1 bis 8 zur Verwendung bei der Behandlung oder Prävention von Staphylokokken-Krankheit.

## Revendications

1. Composition immunogène comprenant deux polypeptides staphylococciques isolés différents, chacun comprenant un domaine de liaison d'IgG, les polypeptides staphylococciques isolés comprenant un polypeptide de la protéine A isolé provenant de *S. aureus* ayant une séquence qui est au moins identique à 90 % avec SEQ ID NO : 12 à 40, et un polypeptide Sbi isolé provenant de *S. aureus* ayant une séquence qui est au moins identique à 90 % avec SEQ ID NO : 1 à 11.

2. Composition immunogène selon la revendication 1, dans laquelle le polypeptide de la protéine A isolé s'est lié de façon covalente au polypeptide Sbi isolé pour former une protéine de fusion.

3. Composition immunogène selon la revendication 2, dans laquelle la protéine de fusion a une séquence polypeptidique qui est au moins identique à 90 % avec la séquence de SEQ ID NO : 41-42 ou 76.

4. Composition immunogène selon l'une quelconque des revendications 1 à 3 comprenant un autre antigène staphylococcique.

5. Composition immunogène selon la revendication 4, dans laquelle l'autre antigène staphylococcique comprend un polysaccharide capsulaire de type 8 de *S. aureus.*

6. Composition immunogène selon la revendication 5, dans laquelle le polysaccharide capsulaire de type 8 de *S. aureus* est conjugué à une protéine de support.

7. Composition immunogène selon la revendication 6, dans laquelle la protéine de support est choisie indépendamment dans le groupe constitué de l'anatoxine tétanique, l'anatoxine diphtérique, CRM197, la protéine D, la toxine alpha, SdrG, ClfA, IsdA, IsdB, IsdH, la protéine A, Sbi et une protéine de fusion protéine A-Sbi.

8. Composition immunogène selon la revendication 6 ou 7, dans laquelle l'autre antigène staphylococcique comprend une protéine choisie dans le groupe constitué de Ebh, la protéine de liaison de l'élastine, EFB, ClfA, ClfB, SdrC, SdrG, FnbA, FbpA, IsaA/PisA, la protéine de liaison de la pénicilline 4, AhpC, SsaA, Aap, SasA, IsdA, IsdB, IsdC, HarA, la toxine alpha, le mutant H35R de la toxine alpha et RAP.

9. Polypeptide comprenant : a) une partie protéine A ayant une séquence d'acides aminés présentant au moins 90 % d'identité avec une séquence d'acides aminés choisie dans le groupe constitué de SEQ ID NO : 12 à 40 et b) une partie Sbi qui a une séquence d'acides aminés présentant au moins 90 % d'identité avec une séquence d'acides aminés choisie dans le groupe constitué de SEQ ID NO : 1 à 11.

10. Polypeptide selon la revendication 9 ayant une séquence d'acides aminés qui est au moins identique à 90 % avec SEQ ID NO : 41 ou 42 ou 76.

11. Polynucléotide comprenant : a) une région codant pour la protéine A présentant au moins 90 % d'identité avec une séquence polynucléotidique choisie dans le groupe constitué de SEQ ID NO : 54 à 72 et b) une région codant pour Sbi qui présente au moins 90 % d'identité avec une séquence polynucléotidique choisie dans le groupe constitué de SEQ ID NO : 43 à 53.

12. Polynucléotide codant pour une protéine de fusion comprenant : a) une partie protéine A présentant au moins 90 % d'identité avec une séquence d'acides aminés choisie dans le groupe constitué de SEQ ID NO : 12 à 40 et b) une partie Sbi qui présente au moins 90 % d'identité avec une séquence d'acides aminés choisie dans le groupe constitué de SEQ ID NO : 1 à 11.

13. Vaccin comprenant la composition immunogène selon l'une quelconque des revendications 1 à 8 ou le polypeptide selon la revendication 9 ou 10 et un support pharmaceutiquement acceptable.

14. Procédé de fabrication du vaccin selon la revendication 13 comprenant l'étape d'addition d'un excipient pharmaceutiquement acceptable à la composition immunogène selon l'une quelconque des revendications 1 à 8 ou au polypeptide selon l'une quelconque des revendications 9 ou 10.

15. Composition immunogène selon l'une quelconque des revendications 1 à 8 pour une utilisation dans le traitement ou la prévention d'une maladie staphylococcique.
